# EUROPEAN PATENT SPECIFICATION

(11) **EP 4 305 181 B1**
(45) Date of publication and mention of the grant of the patent: **05.11.2025**
(21) Application number: 22710714.1
(22) Date of filing: 14.03.2022
(51) Int. Cl.: C12N 15/86, C12N 15/63

(54) **MINIMAL NEPHRIN PROMOTER**
MINIMALER NEPHRIN-PROMOTER
PROMOTEUR DE NÉPHRINE MINIMAL

(30) Priority: 12.03.2021 GB 202103470; 12.03.2021 WO PCT/GB2021/050633; 30.06.2021 WO PCT/GB2021/051668
(43) Date of publication of application: 17.01.2024
(60) Divisional application: 25181596.5
(73) Proprietor: The University of Bristol, Bristol BS8 1QU (GB); Syncona IP Holdco (3) Limited, London WC1B 3SR (GB)
(72) Inventor: SALEEM-UDDIN, Moin, Bristol BS8 1QU (GB); WELSH, Gavin, Bristol BS8 1QU (GB); KUZMUK, Valeryia, London WC1B 3SR (GB); GRIFFITH, Alan William, London WC1B 3SR (GB)
(74) Representative: Stratagem IPM Limited
(86) International application number: PCT/GB2022/050649
(87) International publication number: WO 2022/189811

(56) References cited:
- WO-A1-2020/148548
- RISTOLA M ET AL: "Transcription of nephrin-Neph3 gene pair is synergistically activated by WT1 and NF- B and silenced by DNA methylation", NEPHROLOGY DIALYSIS TRANSPLANTATION, vol. 27, no. 5, 6 October 2011 (2011-10-06), GB, pages 1737 - 1745, XP055929853, ISSN: 0931-0509, DOI: 10.1093/ndt/gfr576
- RISTOLA M ET AL: "Regulation of nephrin gene by the Ets transcription factor, GA-binding protein", NEPHROLOGY DIALYSIS TRANSPLANTATION, vol. 28, no. 4, 1 April 2013 (2013-04-01), GB, pages 846 - 855, XP055929781, ISSN: 0931-0509, Retrieved from the Internet <URL:https://academic.oup.com/ndt/article-pdf/28/4/846/12928529/gfs482.pdf> DOI: 10.1093/ndt/gfs482
- GUO G ET AL: "WT1 Activates a Glomerular-Specific Enhancer Identified from the Human Nephrin Gene", JOURNAL OF THE AMERICAN SOCIETY OF NEPHROLOGY, vol. 15, no. 11, 1 November 2004 (2004-11-01), US, pages 2851 - 2856, XP055929896, ISSN: 1046-6673, Retrieved from the Internet <URL:https://jasn.asnjournals.org/content/jnephrol/15/11/2851.full.pdf> DOI: 10.1097/01.ASN.0000143474.91362.C4

## Description

### FIELD OF THE INVENTION

The present invention relates to promoters which are capable of driving transgene expression in podocytes. The present invention also relates to polynucleotides and vectors comprising said promoters.

### BACKGROUND TO THE INVENTION

There are many diseases which affect kidney function by attacking the glomerulus. The glomerulus filters approximately 180 litres of plasma each day, and the healthy glomerular filtration barrier has an astonishing ability to retain about 99.9% of large proteins including albumin over our lifetimes without clogging. The glomerular filtration barrier (GFB) comprises 3 main layers: the glomerular endothelial cell, the glomerular basement membrane (GBM) and the podocyte.

The GBM is made of a highly crosslinked macromolecular meshwork of type IV collagen, proteoglycans, and laminin. Genetic forms of glomerular disease can be caused by genetic defects in these molecular structures. For example, Alport syndrome is caused by pathogenic variants in the COL4A3, COL4A4 and COL4A5 genes, which result in abnormalities of the collagen IV α345 network of basement membranes. Alport syndrome affects approximately 1 in 5,000-10,000 of all individuals in continental Europe and the USA. The condition usually presents during childhood and is associated with a spectrum of phenotypes that include a progressive loss of kidney function, and can also include hearing loss and eye abnormalities. Other GBM-associated diseases include Pierson syndrome and Nail-patella syndrome (Chiang, C.K. and Inagi, R., 2010. Nature Reviews Nephrology, 6(9), p.539).

The podocyte has also been implicated as a key cell in the progression of glomerular disease. Podocytes are mesodermally derived cells that are highly specialized and found only in the renal glomerulus. They exhibit unique characteristics such as foot processes and slit diaphragms, which are critical for glomerular filtration. Podocyte-associated genetic glomerular diseases include Nephrotic Syndrome, Frasier syndrome and Denys-Drash syndrome, Schimke immuno-osseous dysplasia, and Epstein and Fechtner syndrome. (Chiang, C.K. and Inagi, R., 2010. Nature Reviews Nephrology, 6(9), p.539).

Accordingly, glomerular cells, such as podocytes, represent a potential target for gene therapy approaches.

In order to maximise gene therapy potential, promoter sequences which can drive transgene expression in glomerular cells, such as podocytes, are required. Wong et al. (American Journal of Physiology Renal Physiology; 2000; 279(6); F1027-32) described a 1.25-kb DNA fragment from the human nephrin promoter and 5'-flanking region that is capable of directing podocyte-specific expression. This promoter has been used to achieve kidney-specific expression of GFP using an AAV9 vector (Picconi et al.; 2014; Molecular Therapy - Methods & Clinical Development; 1, 14014).

At present a significant amount of vector cargo capacity (e.g. AAV cargo capacity) is taken up with non-coding elements such as the nephrin or podocin promotors, WPRE elements and polyadenylation sequence. Thus, there is a need in the art for minimal promoters which can drive transgene expression in glomerular cells, such as podocytes, in order to provide maximal flexibility for gene therapy approaches for glomerular diseases.

WO 2020/148548 A1 provides an AAV vector gene therapy for use in treating a monogenic form of nephrotic syndrome, wherein the AAV vector comprises a NS-associated transgene and minimal nephrin promoter NPHS1 or podocin promoter NPHS2.

Ristola, M., et al., 2012. Nephrology Dialysis Transplantation, 27(5), pp.1737-1745 discloses that transcription of the nephrin-Neph3 gene pair is synergistically activated by WT1 and NF-κB and silenced by DNA methylation.

Ristola, M., et al., 2013. Nephrology Dialysis Transplantation, 28(4), pp.846-855 discloses that transcription factor GA-binding protein (GABP) is expressed in podocytes and is involved in the regulation of nephrin gene expression.

Guo, G., et al., 2004. Journal of the American Society of Nephrology, 15(11), pp.2851-2856 discloses that WT1 activates a glomerular-specific enhancer identified from the human nephrin gene.

### SUMMARY OF THE INVENTION

The present invention is based on the inventors surprising provision of a minimal promoter which is capable of driving transgene expression in kidney cells, in particular glomerular cells, such as podocytes.

In one aspect, the present invention provides a promoter which is capable of driving transgene expression in kidney cells and comprises or consists of a nucleotide sequence having at least 90% identity to SEQ ID NO: 47.

In some embodiments, the promoter comprises or consists of a nucleotide sequence having at least 95%, at least 96%, at least 97%, at least 98%, or at least 99% identity to SEQ ID NO: 47.

The promoter may comprise (i) a nucleotide sequence having at least, or at least 99% identity to SEQ ID NO: 17 and the promoter may have a length of 1.1 kb or less.

The promoter may be a kidney-specific promoter. The promoter may be operable in a podocyte cell. The promoter may be capable of driving transgene expression in podocytes. The promoter may be a podocyte-specific promoter.

Suitably, the promoter further comprises (ii) a nucleotide sequence having at least 95%, or at least 99% identity to SEQ ID NO: 18, and/or a nucleotide sequence having at least 95%, or at least 99% identity to SEQ ID NO: 19. In some embodiments, the promoter further comprises (ii) a nucleotide sequence having at least 95%, or at least 99% identity to SEQ ID NO: 19. The promoter may have a length of about 1.0 kb or less, about 0.9 kb or less, about 0.8 kb or less, about 0.7 kb or less, about 0.6 kb or less, about 0.5 kb or less, about 0.4 kb or less, or about 0.3 kb or less. The promoter may have a length of from 0.265 kb to 1.0 kb, from 0.265 kb to 0.9 kb, from 0.265 kb to 0.8 kb, from 0.265 kb to 0.7 kb, from 0.265 kb to 0.6 kb, from 0.265 kb to 0.5 kb, from 0.265 kb to 0.4 kb, or from 0.265 kb to 0.3 kb.

The promoter may comprise: (a) a retinoic acid receptor binding site; (b) a WT1 binding site; (c) an enhancer box; (d) a transcription factor binding region; and/or (e) a transcription initiation site. Suitably, the retinoic acid receptor binding site comprises or consists of a nucleotide sequence shown as SEQ ID NO: 10 or a nucleotide sequence having one or two substitutions, deletions, or insertions compared to SEQ ID NO: 10. Suitably, the WT1 binding site comprises or consists of a nucleotide sequence shown as SEQ ID NO: 11 or a nucleotide sequence having one, two or three substitutions, deletions, or insertions compared to SEQ ID NO: 11. Suitably, the enhancer box comprises or consists of a nucleotide sequence shown as SEQ ID NO: 12 or a nucleotide sequence having one or two substitutions, deletions, or insertions compared to SEQ ID NO: 12. Suitably, the transcription factor binding region comprises or consists of a nucleotide sequence shown as SEQ ID NO: 13 or 43 or a nucleotide sequence having one, two, three, four or five substitutions, deletions, or insertions compared to SEQ ID NO: 13 or 43. Suitably, the transcription initiation site comprises or consists of an "AG" dinucleotide. Suitably, the transcription factor binding site is operably linked to the transcription initiation site, optionally wherein the transcription factor binding site is directly upstream of the transcription initiation site.

The nucleotide sequence having at least 95%, or at least 99% identity to SEQ ID NO: 17 may comprise one or more of: (a) a retinoic acid receptor binding site; (b) a WT1 binding site; and (c) an enhancer box. One or more of the following nucleotide sequences may be present in the nucleotide sequence having at least 95%, or at least 99% identity to SEQ ID NO: 17: (a) GGGGTCA at position 7 to position 13 of the proximal promoter region (b) CGGAGGCTGGGGAGGCA at position 14 to position 30 of the proximal promoter region and (c) ATGTG at position 49 to position 53 of the proximal promoter region.

In some embodiments, the promoter comprises or consists of the nucleotide sequence of SEQ ID NO: 47.

In one aspect, the present invention provides a polynucleotide comprising the promoter according to the present invention.

The promoter may be operably linked to a protein coding sequence. In some embodiments, the protein coding sequence encodes NPHS2 or a fragment and/or variant thereof; a COL4A3, COL4A4 or COL4A5 polypeptide, or a fragment or derivative thereof; or CFI, CFH or FHL-1, or a fragment and/or variant thereof.

In some embodiments, the protein coding sequence encodes NPHS2, or a fragment and/or variant thereof. In some embodiments, the protein coding sequence encodes an amino acid sequence which is at least 70%, at least 80%, at least 90%, at least 95%, at least 98% or at least 99% identical to SEQ ID NO: 55 or a fragment thereof. In some embodiments, the protein coding sequence comprises or consists of a nucleotide sequence which is at least 70%, at least 80%, at least 90%, at least 95%, at least 98% or at least 99% identical to SEQ ID NO: 56 or a fragment thereof. In some embodiments, the protein coding sequence comprises or consists of a nucleotide sequence which is at least 70%, at least 80%, at least 90%, at least 95%, at least 98% or at least 99% identical to SEQ ID NO: 57 or a fragment thereof.

In some embodiments, the protein coding sequence encodes a COL4A3, COL4A4 or COL4A5 polypeptide, or a fragment or derivative thereof. In some embodiments, the protein coding sequence encodes an amino acid sequence which is at least 70%, at least 80%, at least 90%, at least 95%, at least 98% or at least 99% identical to one of SEQ ID NOs: 21-23 or a fragment thereof. In some embodiments, the protein coding sequence comprises or consists of a nucleotide sequence which is at least 70%, at least 80%, at least 90%, at least 95%, at least 98% or at least 99% identical to one of SEQ ID NOs: 24-26 or a fragment thereof.

In some embodiments, the protein coding sequence encodes CFI, or a fragment and/or variant thereof. In some embodiments, the protein coding sequence encodes an amino acid sequence which is at least 70%, at least 80%, at least 90%, at least 95%, at least 98% or at least 99% identical to SEQ ID NO: 49 or a fragment thereof. In some embodiments, the protein coding sequence comprises or consists of a nucleotide sequence which is at least 70%, at least 80%, at least 90%, at least 95%, at least 98% or at least 99% identical to SEQ ID NO: 50 or a fragment thereof.

In some embodiments, the protein coding sequence encodes CFH, or a fragment and/or variant thereof. In some embodiments, the protein coding sequence encodes an amino acid sequence which is at least 70%, at least 80%, at least 90%, at least 95%, at least 98% or at least 99% identical to SEQ ID NO: 51 or a fragment thereof. In some embodiments, the protein coding sequence comprises or consists of a nucleotide sequence which is at least 70%, at least 80%, at least 90%, at least 95%, at least 98% or at least 99% identical to SEQ ID NO: 52 or a fragment thereof.

In some embodiments, the protein coding sequence encodes FHL-1, or a fragment and/or variant thereof. In some embodiments, the protein coding sequence encodes an amino acid sequence which is at least 70%, at least 80%, at least 90%, at least 95%, at least 98% or at least 99% identical to SEQ ID NO: 53 or a fragment thereof. In some embodiments, the protein coding sequence comprises or consists of a nucleotide sequence which is at least 70%, at least 80%, at least 90%, at least 95%, at least 98% or at least 99% identical to SEQ ID NO: 54 or a fragment thereof.

In some embodiments, the protein coding sequence is operably linked to one or more further regulatory elements, such as a post-transcriptional regulatory element and/or a polyadenylation sequence.

In one aspect, the present invention provides a vector comprising the polynucleotide according to the present invention. Suitably, the vector is capable of transducing podocytes, optionally wherein the vector is capable of specifically transducing podocytes.

Suitably, the vector is a viral vector, such as an adeno-associated virus (AAV) vector, a lentiviral vector, a retroviral vector, an adenoviral vector, an adeno-associated viral vector, a herpes simplex viral vector, an alphaviral vector, a flaviviral vector, a rhabdoviral vector, a measles viral vector, a Newcastle disease viral vector, a poxviral vector, and a picornaviral vector, preferably wherein the vector is an AAV vector. Suitably, the viral vector is in the form of a viral vector particle, preferably wherein the viral vector is in the form of an AAV vector particle.

In some embodiments, the vector is the form of an AAV vector particle encapsidated by AAV3B, LK03, or AAV9 capsid proteins.

In one aspect, the present invention provides a cell comprising the polynucleotide according to the present invention, or the vector according to the present invention.

In one aspect, the present invention provides a pharmaceutical composition comprising the polynucleotide according to the present invention, the vector according to the present invention, or the cell according to the present invention.

In one aspect, the present invention provides a polynucleotide according to the present invention, vector according to the present invention, or cell according to the present invention for use in medicine.

In one aspect, the present invention provides for *in vitro* use of the promoter according to the present invention to drive expression of a coding sequence. The expression may be kidney-specific. Suitably, the expression is podocyte-specific.

### DESCRIPTION OF DRAWINGS

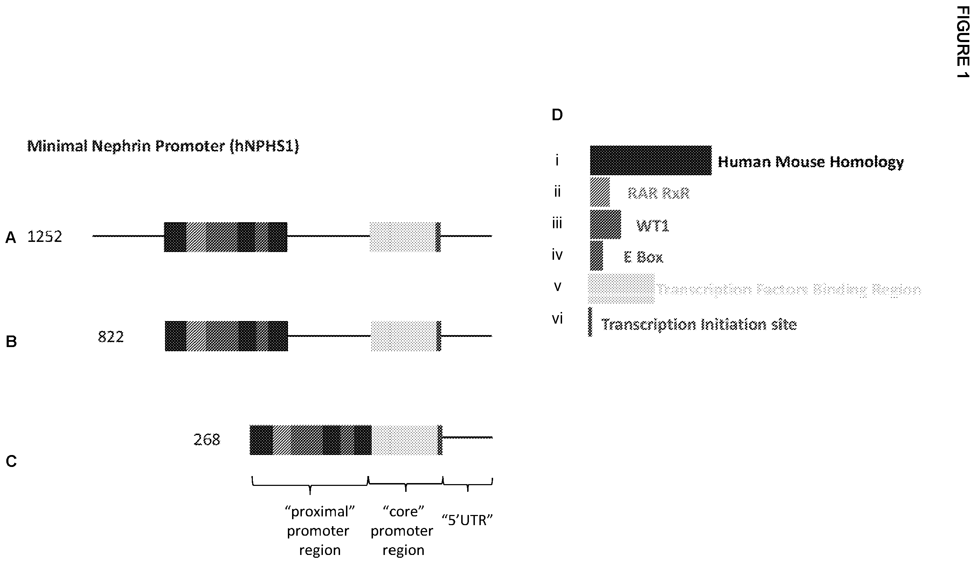

**Figure 1** **- Schematic illustration of minimal nephrin promoters**
   **(A)** An exemplary full-length nephrin promoter may be 1249 bp in length (excluding the start codon), hereafter referred to as the "FL" nephrin promoter. **(B)** An exemplary minimal nephrin promoter with the 5' region deleted may be 819 bp in length (excluding the start codon), hereafter referred to as the "midi" nephrin promoter. **(C)** An exemplary minimal nephrin promoter with the 5' region deleted and central region deleted may be 265 bp in length (excluding the start codon), hereafter referred to as the "mini" nephrin promoter. **(D)** The following regions of the nephrin promoter are indicated: (i) human mouse homology region, (ii) retinoic acid receptor (RAR) binding site, (iii) WT1 binding site, (iv) Enhancer box, (v) transcription factors binding region, and (vi) transcription initiation site.
**Figure 2** **- Schematic of a lentiviral vector comprising GFP operably coupled to midi nephrin promoter**
   **(A)** pACE_hNPHS1 promoter was used as a template, to introduce BamH1 and Cla1 restriction sites. **(B)** Final construct vector comprising GFP operably coupled to midi nephrin promoter.
**Figure 3** **- Schematic of a lentiviral vector comprising GFP operably coupled to mini nephrin promoter**
   **(A)** pACE_hNPHS1 promoter was used as a template to PCR and gel extract the two sections of the promoter. **(B)** Final construct vector comprising GFP operably coupled to mini nephrin promoter.
**Figure 4** **- Expression of GFP in CiPodocytes following transduction with lentiviral vectors**
   Human CiPodocytes stably expressing GFP-tagged nephrin promoters were generated using the lentiviral approach. GFP expression was observed by fluorescence microscopy. **(A)** Untransduced CiPodocytes. **(B)** CiPodocytes stably expressing the GFP-tagged mini nephrin promoter. **(C)** CiPodocytes stably expressing the GFP-tagged FL nephrin promoter.

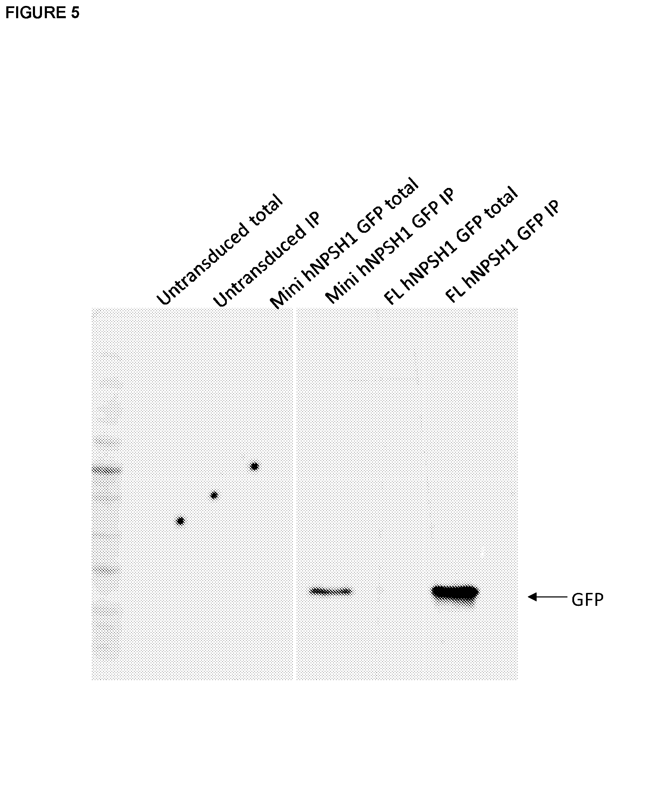

**Figure 5** **- Expression of GFP in differentiated ciPodocytes following transduction with lentiviral vectors**
   Lentiviral vectors comprising GFP operably linked to nephrin promoters were transduced into differentiated conditionally immortalised podocytes (ciPodocytes). Immunoprecipitation (IP) was used to detect GFP expression.

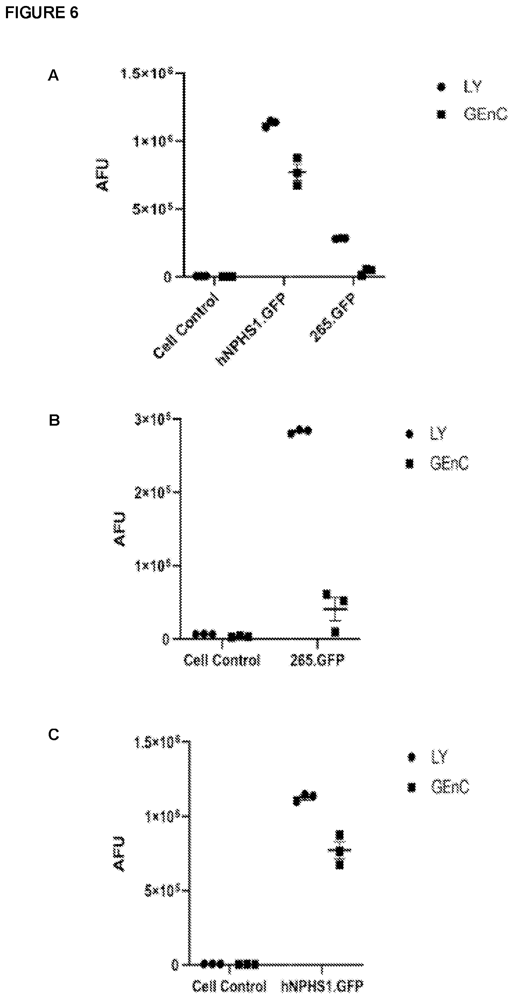

**Figure 6** **- Human glomerular cells transduced with Lentivirus - GFP.nephrin promoter (FL or 265)**
   FACS analysis displaying median GFP fluorescence (AFU) of all live singlets of conditionally immortalised human podocytes (LY) and glomerular endothelial cells (GEnC) using a Novocyte Analyser. Untransduced cells (Cell Control) were compared with those transduced with lentivirus constructs harbouring a GFP expression cassette controlled by a full length human nephrin promoter (hNPHS1.GFP) or a mini human nephrin promoter (265.GFP). All cells were differentiated for 10 days, trypsinised (100 uL) and diluted in PBS, 2%FBS, 1:1000 DRAQ7 (150uL). Data and error bars represent 3 technical repeats (100 uL, >2500 cells) ±SEM.

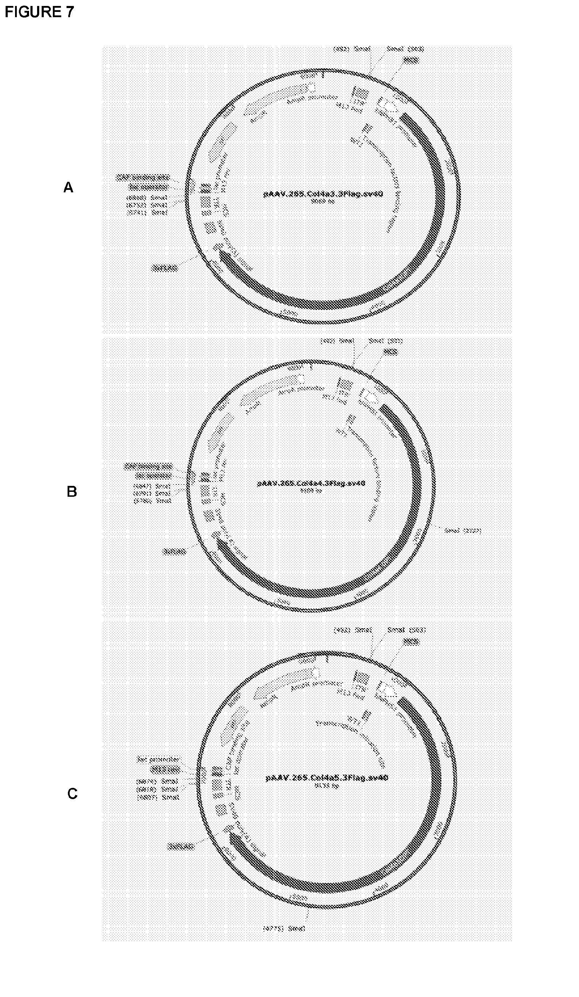

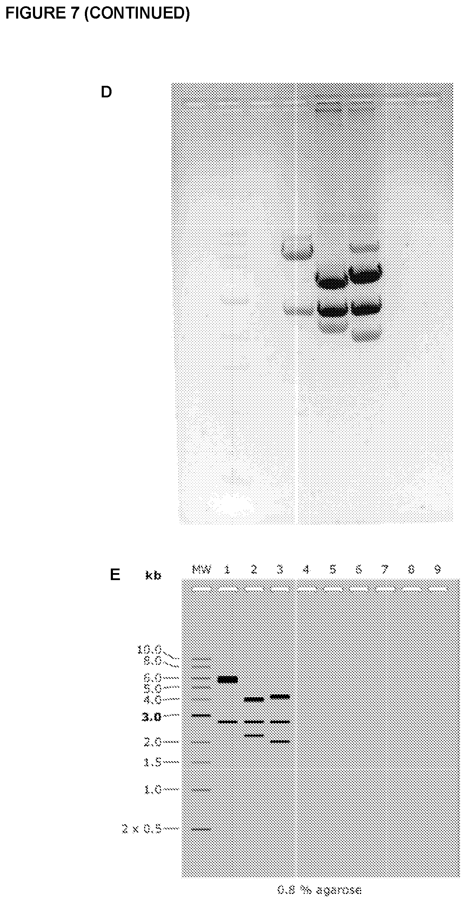

**Figure 7** **- AAV transfer plasmids comprising COL4A3, COL4A4, and COL4A5 coupled to mini nephrin promoter**
   **(A)** Schematic for pAAV.265.Col4a3.3flag.sv40, an AAV plasmid comprising COL4A3 coupled to mini nephrin promoter. Smal sites are shown and the following fragments are expected following restriction with Smal: 1. 6238 bp, 2. 2753 bp, 3. 56 bp, 4. 11 bp, 5. 11 bp. **(B)** Schematic for pAAV.265.Col4a4.3flag.sv40, an AAV plasmid comprising COL4A4 coupled to mini nephrin promoter. Smal sites are shown and the following fragments are expected following restriction with Smal: 1. 4052 bp, 2. 2753 bp, 3. 2224 bp, 4. 56 bp, 5. 11 bp, 6. 11 bp. **(C)** Schematic for pAAV.265.Col4a5.3flag.sv40, an AAV plasmid comprising COL4A5 coupled to mini nephrin promoter. Smal sites are shown and the following fragments are expected following restriction with Smal: 1. 4272 bp, 2. 2753 bp, 3. 2032 bp, 4. 56 bp, 5. 11 bp, 6. 11 bp. (D) Restriction digest with Smal. MW = 1 Kb DNA ladder, Lanes 1, 2 and 3 correspond to the digests for plasmids shown in (A), (B), and (C), respectively. **(E)** Schematic showing restriction digest.

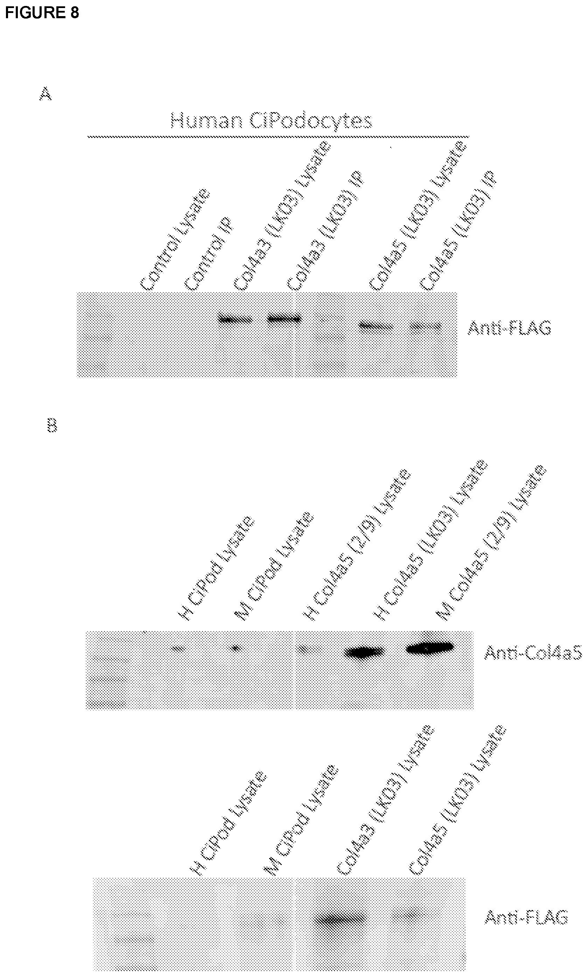

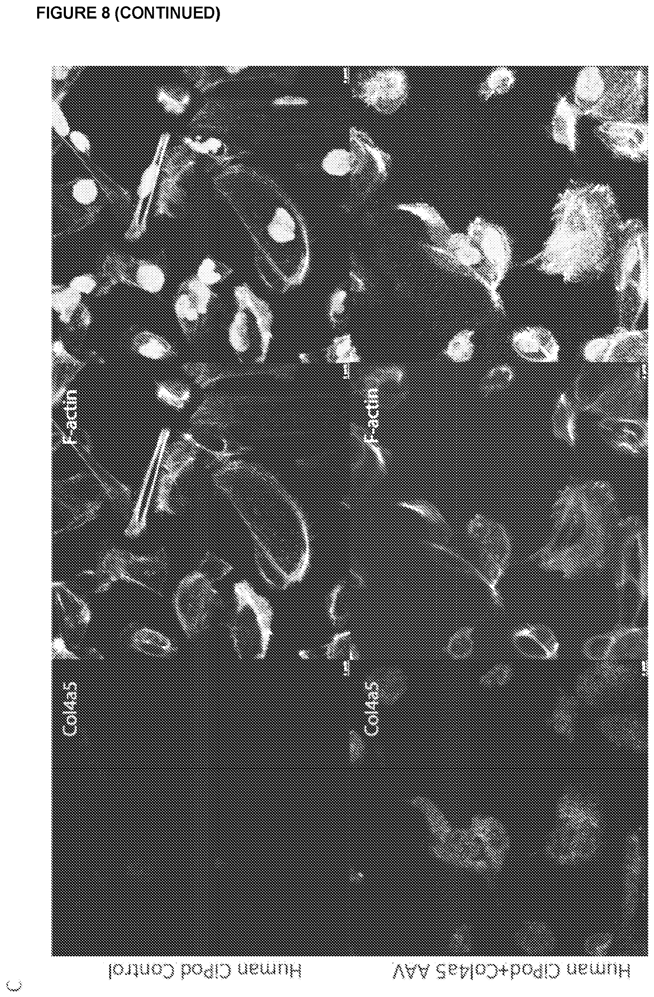

**Figure 8** **- Podocytes transduced with AAV.COL4.nephrin265.Sv40 virus**
   **(A)** Immunoprecipitation experiments of the full-length FLAG-tagged Col4a3 (LK03) or Col4a5 (LK03) in human differentiated CiPodocytes (conditionally immortalised) pulled down with the anti-FLAG antibody. Anti-FLAG antibody precipitated both Col4a3 and Col4a5. Human-FLAG IgG was used as a control. **(B)** Western blots of protein lysates showing the expression levels of Col4a3 (LK03 capsid serotype), Col4a5 (LK03) and Col4a5 (2/9 capsid serotype) in human or mouse differentiated CiPodocytes. Non-infected human and mouse Cipodocytes were used as controls. (C) Confocal images showing immunofluorescence staining of transduced Col4a5 in Human wild-type CiPodocytes/Col4a5 3xFlag AAV CiPodocytes with F-Actin. Col4a5 is present at the cytosolic level in the human differentiated podocytes infected with Col4a5 3xFlag AAV virus in comparison to the wild-type counterpart.

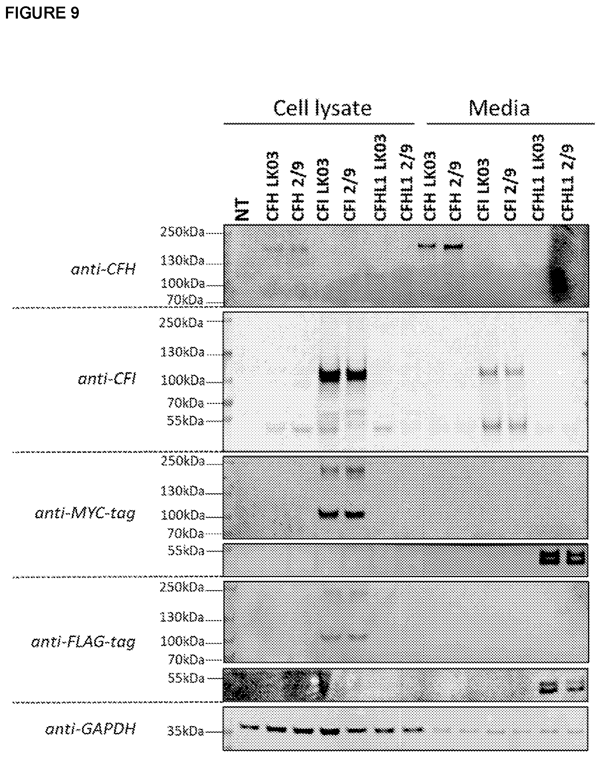

**Figure 9** **- Expression of CFH, CFI and CFHL1 in HEK cells**
   Protein expression analysis by western blot in 293T Human Embryonic Kidney cells. Cells were transfected with pAAV-265-CFH, pAAV-FL-CFI or pAAV-FL-CFHL1. NT (non-transfected 293T HEK cells). Expression of each of the transgenes is demonstrated in the cell lysate and/or media using protein-specific antibodies or anti-FLAG-tag or anti-MYC-tag antibodies. HEK293T cells transfected with plasmid expressing the CFH transgene under the control of the 265bp minimal nephrin promoter expressed and secreted human Factor H.

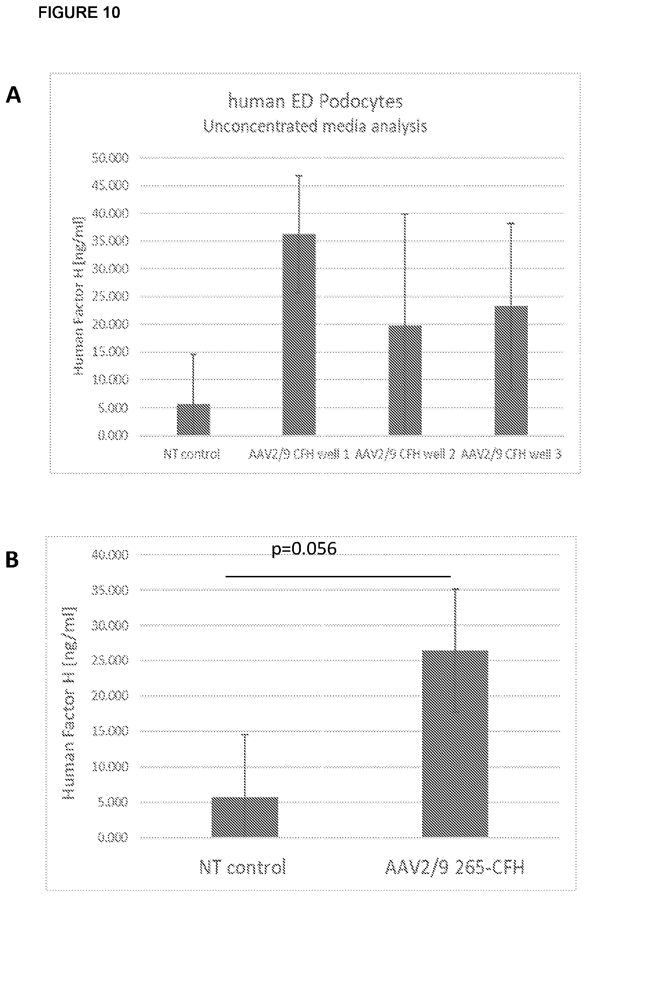

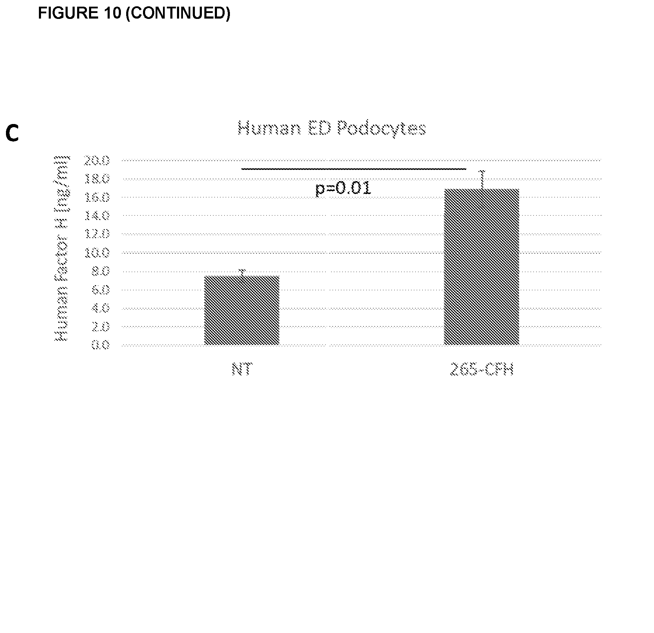

**Figure 10** **- Transduction of Factor H mutated podocytes ("Human early disease (ED) podocytes") with AAV2/9 265-CFH or transfection with plasmid encoding CFH**
   (A) Analysis of human Factor H concentration using an ELISA assay. Podocytes transduced with AAV2/9 virus containing the CFH transgene demonstrated higher concentrations of human Factor H in the culture media than the non-transduced control. **(B)** The average human Factor H concentration from (A). **(C)** Analysis of human Factor H concentration using an ELISA assay. Podocytes transfected with plasmid expressing the CFH transgene under the control of the 265bp minimal nephrin promoter demonstrated higher concentrations of human Factor H than the non-transfected control.

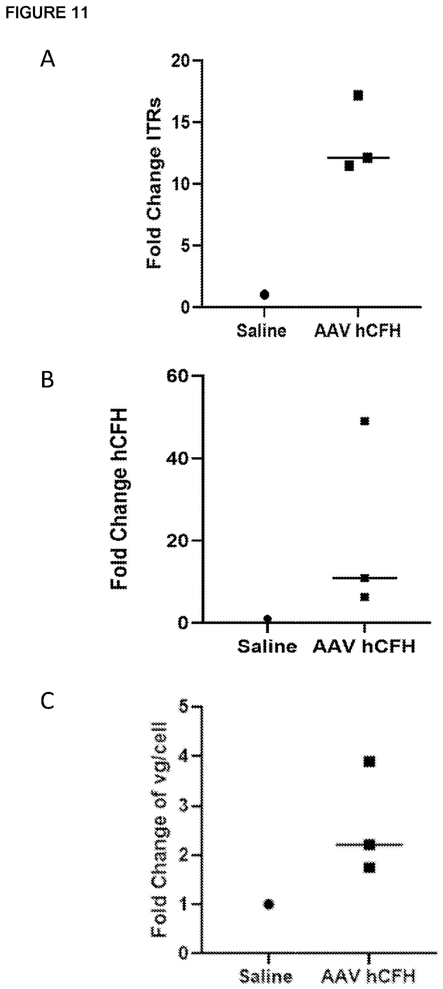

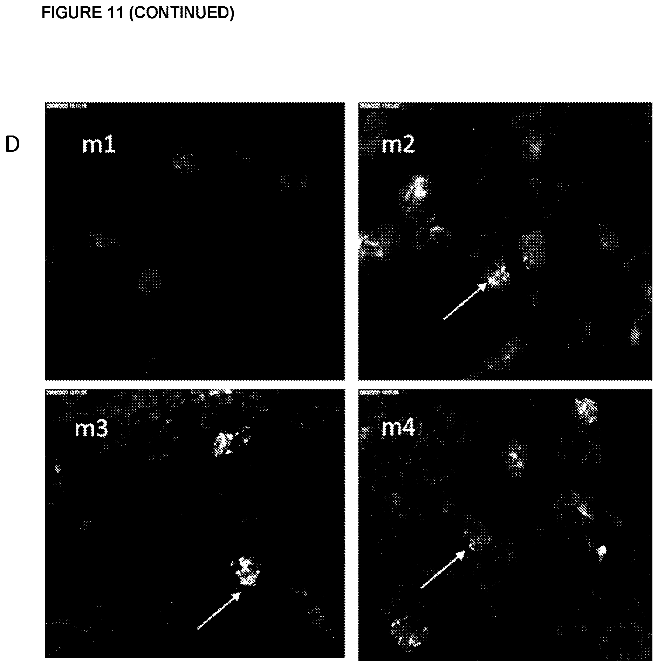

**Figure 11** **- Expression of CFH in the kidney in WT mice**
   Gene expression profile demonstrating an increase in **(A)** viral ITRs, **(B)** human CFH cDNA, and (C) viral particles in wild-type mice injected with pAAV.NPHS1(265).hCFH.WPRE.bGH in comparison to mice injected with the saline control. **(D)** Immunofluorescence imaging of kidney sections derived from wild-type C57BL6 mice injected with the saline control (m1) or the AAV gene therapy product (pAAV.NPHS1(265).hCFH.WPRE.bGH, n=3) (m2/m3/m4). Arrows indicate the co-localization of nephrin and CFH in the mouse glomerulus. Zoom =20x.

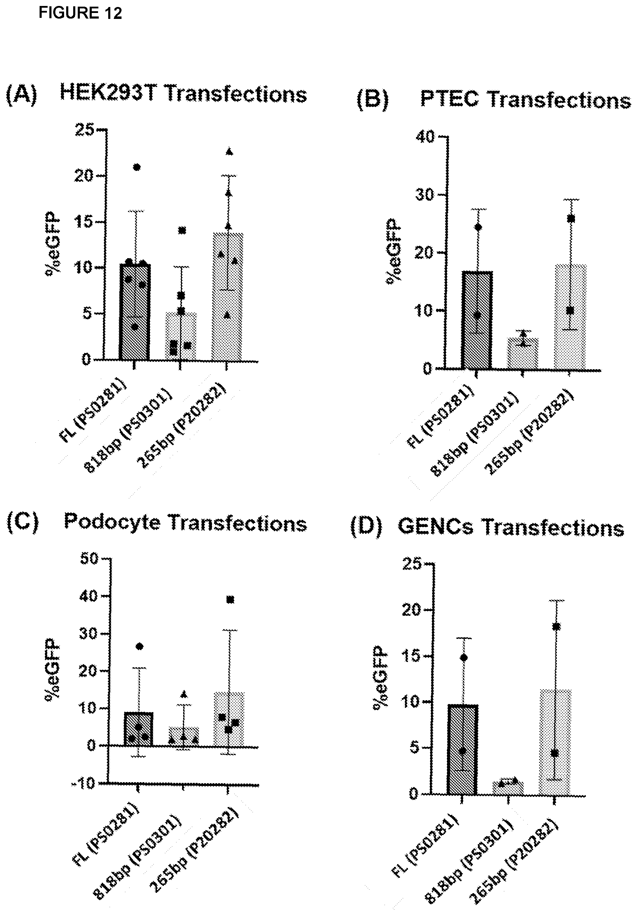

**Figure 12** **- Expression of GFP using 265bp v 818bp v FL minimal nephrin promoters (plasmid transfection) in kidney cells**
   Expression of eGFP in cells transfected with plasmids containing Full Length (PS0281), 818bp (PS0301) and 265bp (PS0282) minimal nephrin promoters driving eGFP. **(A)** Transfection results in HEK293T cells. **(B)** Transfection results in Proximal Tube Epithelial cells (PTECs). **(C)** Transfection results in human Podocytes. **(D)** Transfection results in Glomerular Endothelial cells (GENCs).

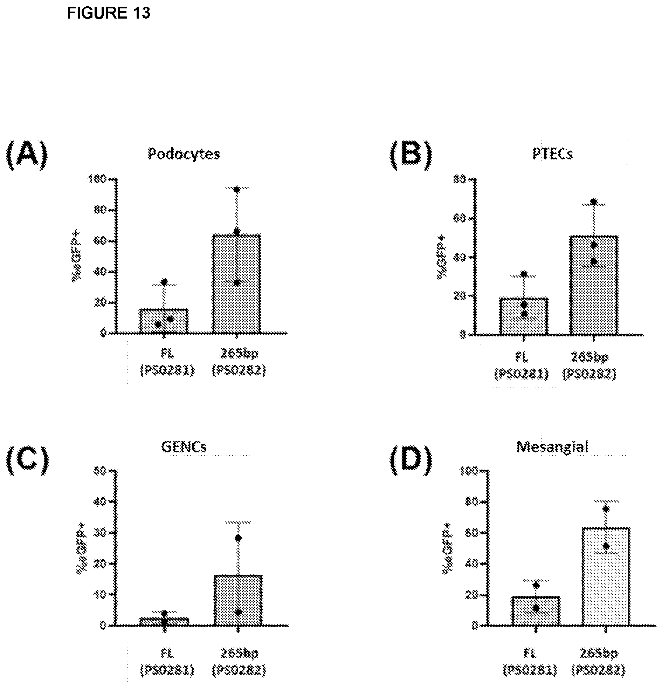

**Figure 13** **- Expression of GFP using 265bp v FL minimal promoters (AAV transduction) in kidney cells**
   Expression of eGFP in cells transduced with AAV particles incorporating transgene cassettes containing Full Length (PS0281) and 265bp (PS0282) minimal nephrin promoters driving eGFP. **(A)** Transduction results in human Podocytes. **(B)** Transduction results in Proximal Tube Epithelial cells (PTECs). **(C)** Transduction results in Glomerular Endothelial cells (GENCs). **(D)** Transduction results in Mesangial cells.

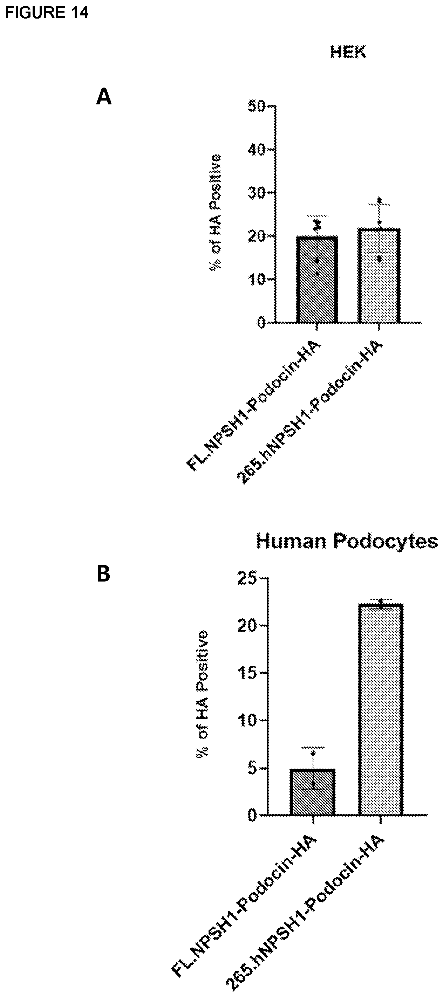

**Figure 14** **- Expression of human podocin using 265bp v FL minimal nephrin promoters**
   **(A)** Protein expression analysis by FACs in Lenti-X 293T Human Embryonic Kidney cells. Cells were transfected with 3ug of plasmid with PEI at ratio of 1:3. Each plasmid expresses human Podocin-HA driven by either full length (FL.NPSH1-Podocin-HA) or 265bp (265.hNPSH1-Podocin-HA) minimal nephrin promoter. Expression of human Podocin is demonstrated by anti-HA antibody on Day 2 post transfection. N=3 data expressed as mean values ± SD. **(B)** Protein expression analysis by FACs in Human Podocytes cells. Cells were transduced with AAV particles, incorporating human Podocin-HA transgene cassette driven by either full length (FL.NPSH1-Podocin-HA) or 265bp (265.hNPSH1-Podocin-HA) minimal nephrin promoter. Expression of human Podocin is demonstrated by anti-HA antibody on Day 9 post transduction and differentiation.

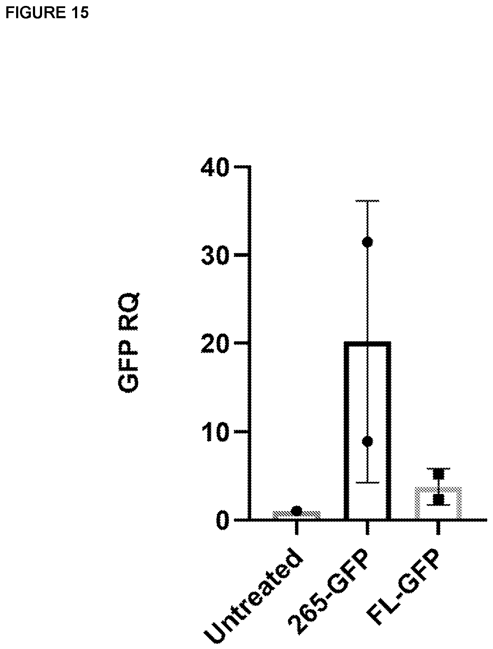

**Figure 15** ***- In vivo* expression of GFP using 265bp v FL minimal nephrin promoters via AAV transduction**
   Evaluation of 265- and FL- nephrin promoter in vivo in WT mice (C57/BI6). AAV2/9 particles were generated expressing GFP under the control of either full-length (FL-GFP) or 265bp (265-GFP) minimal nephrin promoters. Tail vein injection of ~1x10e13 AAV2/9 per mouse (2x mice per treatment group and 1x untreated mouse). Tissue harvest of kidney occurred 4 weeks post injection. qPCR analysis was performed and GFP copy number normalised to 18S (housekeeping gene).

### DETAILED DESCRIPTION

Various preferred features and embodiments of the present invention will now be described by way of non-limiting examples.

It must be noted that as used herein and in the appended claims, the singular forms "a", "an", and "the" include plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. The terms "comprising", "comprises" and "comprised of" also include the term "consisting of".

Numeric ranges are inclusive of the numbers defining the range. Unless otherwise indicated, any nucleic acid sequences are written left to right in 5' to 3' orientation; amino acid sequences are written left to right in amino to carboxy orientation, respectively.

The practice of the present invention will employ, unless otherwise indicated, conventional techniques of chemistry, biochemistry, molecular biology, microbiology and immunology, which are within the capabilities of a person of ordinary skill in the art. Such techniques are explained in the literature. See, for example, Sambrook, J., Fritsch, E.F. and Maniatis, T. (1989) Molecular Cloning: A Laboratory Manual, 2nd Edition, Cold Spring Harbor Laboratory Press; Ausubel, F.M. et al. (1995 and periodic supplements) Current Protocols in Molecular Biology, Ch. 9, 13 and 16, John Wiley & Sons; Roe, B., Crabtree, J. and Kahn, A. (1996) DNA Isolation and Sequencing: Essential Techniques, John Wiley & Sons; Polak, J.M. and McGee, J.O'D. (1990) In Situ Hybridization: Principles and Practice, Oxford University Press; Gait, M.J. (1984) Oligonucleotide Synthesis: A Practical Approach, IRL Press; and Lilley, D.M. and Dahlberg, J.E. (1992) Methods in Enzymology: DNA Structures Part A: Synthesis and Physical Analysis of DNA, Academic Press.

### Promoters

The present invention provides a promoter for glomerular gene therapy.

A "promoter" is used herein according to its typical meaning to refer to a sequence of DNA which regulates and/or initiates transcription of a RNA from a DNA sequence.

Suitably, the promoter is operable in mammalian cells, e.g. human cells. The promoter may be capable of driving transgene expression in mammalian cells, e.g. human cells. The promoter may be a mammalian promoter, e.g. a human promoter.

The promoter of the invention is capable of driving transgene expression in the kidney. Examples of kidney cells in which the promoter may be operable include, but are not limited to glomerular cells.

The promoter may be operable in glomerular cells. The promoter of the invention may be capable of driving transgene expression in the glomerulus. The mature glomerulus contains four cell types: Parietal epithelial cells that form Bowman's capsule, podocytes that cover the outermost layer of the glomerular filtration barrier, glycocalyx-coated fenestrated endothelial cells that are in direct contact with blood, and mesangial cells that sit between the capillary loops (Vaughan, M.R. and Quaggin, S.E., 2008. Journal of the American Society of Nephrology, 19(1), pp.24-33).

The promoter may be operable in a podocyte cell. The promoter of the invention may be capable of driving transgene expression in podocytes.

The promoter of the invention may be a tissue-specific promoter. As used herein, a "tissue-specific promoter" is a promoter which preferentially facilitates expression of a transgene in a specific type of cells or tissue. Suitably, a tissue-specific promoter may facilitate higher expression of a transgene in one cell-type as compared to other cell-types. For example, a tissue-specific promoter may be a promoter which facilitates transgene expression levels at least 10% higher, at least 20% higher, at least 30% higher, at least 40% higher, at least 50% higher, at least 100% higher, at least 200% higher, at least 300% higher, at least 400% higher, at least 500% higher, or at least 1000% higher in one cell-type as compared to expression levels in other cell-types.

Suitably, the promoter is a kidney-specific promoter. In some embodiments, the promoter is a glomerular-specific promoter. In some embodiments, the promoter is a podocyte-specific promoter.

Transgene expression may be measured by any suitable method known in the art. For example by measuring the expression of a reporter transgene, e.g. Green fluorescent protein (GFP), operably linked to the promoter, wherein expression of the reporter transgene correlates with the ability of the promoter to facilitate expression of a gene. Expression of the reporter transgene, e.g. GFP may be determined by any suitable method e.g. FACS. For example, a kidney-specific promoter may facilitate higher expression of a reporter transgene in kidney cells compared to other cell types (e.g. CNS, retina, lung, pancreas, heart, or muscle cells). For example, a podocyte-specific promoter may facilitate higher expression of a reporter transgene in conditionally immortalised podocytes compared to other cell-types. Suitable podocyte cell lines will be well known to those of skill in the art, for example CIHP-1. Methods to generate immortalized podocytes will be well known to those of skill in the art. Suitable methods are described in Ni, L., et al., 2012. Nephrology, 17(6), pp.525-531.

Suitably, the promoter is a minimal kidney-specific promoter. In some embodiments, the promoter is a minimal glomerular-specific promoter. In some embodiments, the promoter is a minimal podocyte-specific promoter.

The promoter may have a length of 1.2 kb or less. Suitably, the promoter has a length of 1.18 kb or less, 1.17 kb or less, 1.16 kb or less, 1.15 kb or less, 1.14 kb or less, 1.13 kb or less, 1.12 kb or less, 1.11 kb or less, or 1.10 kb or less. Suitably, the promoter has a length of 1.15 kb or less.

The promoter may have a length of 1.1 kb or less. In some embodiments, the promoter has a length of about 1.1 kb or less, 1.0 kb or less, about 0.9 kb or less, about 0.8 kb or less, about 0.7 kb or less, about 0.6 kb or less, about 0.5 kb or less, about 0.4 kb or less, or about 0.3 kb or less.

In some embodiments, the promoter has a length of about 0.8 kb or less, about 0.7 kb or less, about 0.6 kb or less, about 0.5 kb or less, about 0.4 kb or less, or about 0.3 kb or less. In some embodiments, the promoter has a length of 818 bp or less. In some embodiments, the promoter has a length of 800 bp or less.

In some embodiments, the promoter has a length of about 0.5 kb or less, about 0.4 kb or less, or about 0.3 kb or less. In some embodiments, the promoter has a length of about 0.3 kb or less.

The promoter may have a length of about 250 bp or more. In some embodiments, the promoter has a length of about 250-1100 bp, 250-1000 bp, 250-900 bp, 250-800 bp, 250-700 bp, 250-600 bp, 250-500 bp, 250-400 bp, 250-300 bp.

The promoter may have a length of about 265 bp or more. In some embodiments, the promoter has a length of about 265-1100 bp, 265-1000 bp, 265-900 bp, 265-800 bp, 265-700 bp, 265-600 bp, 265-500 bp, 265-400 bp, 265-300 bp.

In one embodiment, the promoter has a length of 250-300 bp, 250-280 bp, 255-275 bp, 260-270 bp, or about 265 bp. In one embodiment, the promoter has a length of 800-850 bp, 800-840 bp, 810-830 bp, 815-825 bp, about 819 bp, or about 818 bp.

### Nephrin promoters

The promoter may be a synthetic promoter. As used herein, a "synthetic promoter" is a promoter which does not occur naturally. Typically, a synthetic promoter will be produced by genetic modification of a pre-existing promoter. Thus, a synthetic promoter may be derived from a pre-existing promoter, i.e. identical to a pre-existing promoter, except comprising one or more genetic modifications (e.g. substitutions, deletions, or insertions).

Suitably, the promoter may be derived from the nephrin (NPHS1) promoter. Suitably, the promoter may be a minimal nephrin promoter. The NPHS1 gene encodes nephrin, which is selectively expressed in podocytes.

A human NPHS1 promoter has been described in Moeller et al. 2002 J Am Soc Nephrol, 13(6):1561-7 and Wong MA et al. 2000 Am J Physiol Renal Physiol, 279(6):F1027-32. This NPHS1 promoter is a 1.2kb fragment and appears to be podocyte-specific. The 1.2kb promoter region lacks a TATA box, but has recognition motifs for other transcription factors e.g. PAX-2 binding element, E-box and GATA consensus sequences.

Exemplary nephrin promoters are shown as SEQ ID NO: 1, SEQ ID NO: 14, SEQ ID NO: 44, and SEQ ID NO: 45.
*Exemplary nephrin promoter -* 1,249 *bp (SEQ ID NO: 1)* 
CCTGCAGGGCCCACTAGTCTGTAATCCCAGCATTTTGGGAGGCTGAGGCAGATGGATCACCTGAGGTC
AGGAGTTCGAGACCAGCCTGGCCAACATGATGAAACCCCGTCTCTAGTAAAAATACAAAAATTAGCCA
GGCATGGTGCTATATACCTGTAGTACCAGCTACTTGGGAGACAGAGGTGGGAGAATTACTTGAACCTG
GGAGGTTCAAGCCATGGGAGGTGGAAGTTGCAGTGAGCCGAGATGCCACTGCACTCCAGCCTGAGCAA
CAGAGCAAGACTATCTCAAGAAAAAAAAGAAAGAAAGAAAGGGACTTGCCAAGGTCATGTATCAGGGC
AAGGAAGAGCTGGGGGCCCAGCTGGCTGCTCCCCTGCTGAGCTGGGAGACCACCTTGATCTGACTTCT
CCCATCTTCCCAGCCTAAGCCAGGCCCTGGGGTCACGGAGGCTGGGAGGCACCGAGGAACGCGCCTG
GCATGTGCTGACAGGGAATTTTATGCTCCAGCTGGGCCAGCTGGGAGGAGCCTGCTGGGCAGAGGCCA
GAGCTGGGGGCTCTGGAAGGTACCTGGGGGAGGTTGCACTGTGAGAATGAGCTCAAGCTGGGTCAGAG
AGCAGGGCTGACTCTGCCAGTGCCTGCATCAGCCTCATCGCTCTCCTAGGCTCCTGGCCTGCTGGACT
CTGGGCTGCAGGTCCTTCTTGAAAGGCTGTGAGTAGTGAGACAAGGAGCAGGAGTGAGGGGTGGCAGG
AGAGAAGATAGAGATTGAGAGAGAGAGAGAGAGACAGAGAGAGAGGAAGAGACAGAGACAAAAGGA
GAGAGAACGGCTTAGACAAGGAGAGAAAGATGGAAAGATAAAGAGACTGGCGCAGTGGCTCACGCCT
GTAATCCCAACACTTGGGGAGGCCAAGGTGGGAGGATGGCTTGAAGGAAAGAGTCTGAGATCAACCTG
GCCAACATAGTGAGACCCCGTCTCTAAAAAAAAAAAAGAAAAAAAAAAGAAAAAAGAAAAAAAAAGTTT
TTTTAAAGAGACAGAGAAAGAGACTCAGAGATTGAGACTGAGAGCAAGACAGAGAGAGACACTCACAG
GGAAGAGGGGAAGAGGAAAACGAGAAAGGGAGGAGAGTAACGGAAAGAGATAAAAAAGAAAAGCAGGT
GGCAGAGACACAGAGAGAGGGACCCAGAGAAAGCCAGACAGACGCAGGTGGCTGGCAGCGGGCGCTGT
GGGGGTCACAGTAGGGGGACCTGTC
*Exemplary variant nephrin promoter - 1,192 bp (SEQ ID NO: 14)* 
CACCTGAGGTCAGGAGTTCGAGACCAGCGTGGCCAACATGATGAAACCCCGTCTCTAGTAAAAATACA
AAAATTAGCCAGGCATGGTGCTATATACCTGTAGCACCAGCTACTTGGAGACAGAGGTGGGAGAATT
ACTTGAACCTGGGAGGTTCAAGCCATGGGAGGTGGAAGTTGCAGTGAGCCGAGATGCCACTGCACTCC
AGCCTGAGCAACAGAGCAAGACTATCTCAAGAAAAGAAAGAAAGAAAGAAAGAGACTTGCCAAGGTCA
TGTATCAGGGCAAGGAAGAGCTGGGGGCCCAGCTGGCTGCTCCCCTGCTGAGCTGGGAGACCACCTTG
ATCTGACTTCTCCCATCTTCCCAGCCTAAGCCAGGCCCTGGGGTCACGGAGGCTGGGGAGGCACCGAG
GAACGCGCCTGGCATGTGCTGACAGGGGATTTATGCTCCAGCTGGGCCAGCTGGGAGGAGCCTGCTG
GGCAGAGGCCAGAGCTGGGGGCTCTGGAAGGTACCTGGGGGAGGTTGCACTGTGAGAATGAGCTCAAG
CTGGGTCAGAGAGCAGGGCTGACTCTGCCAGTGCCTGCATCAGCCTCATCGCTCTCCTAGGCTCCTGG
CCTGCTGGACTCTGGGCTGCAGGTCCTTCTTGAAAGGCTGTGAGTAGTGAGACAAGGAGCAGGAGTGA
GGGGTGGCAGGAGAGAAGATAGAGATTGAGAGAGAGAGAGAGAGAGACAGAGAGAGAGGAAGAGAC
AGAGACAAAAGGAGAGAGAACGGCTTAGACAAGGAGAGAAAGATGGAAAGATAAAGAGACTGGGCGCA
GTGGCTCACGCCTGTAATCCCAACACTTGGGGAGGCCAAGGTGGGAGGATGGCTTGAAGGAAAGAGTC
TGAGATCAACCTGGCCAACATAGTGAGACCCCGTCTCTAAAAAAAAAAGAAAAAAAAAAGAAAAAAGA
AAAAAAAGTTTTTTAAAGAGACAGAGAAAGAGACTCAGAGATTGAGACTGAGAGCAAGACAGAGAGA
GATACTCACAGGGAAGAGGGGAAGAGGAAAACGAGAAAGGGAGGAGAGTAACGGAAAGAGATAAAAAA
GAAAAGCAGGTGGCAGAGACACACAGAGAGGGACCCAGAGAAAGCCAGACAGACGCAGGTGGCTGGCA
GCGGGCGCTGTGGGGGTCACAGTAGGGGGACCTGTG
*Exemplary variant nephrin promoter - 1, 192 bp (SEQ ID NO: 44)* 
CACCTGAGGTCAGGAGTTCGAGACCAGCGTGGCCAACATGATGAAACCCCGTCTCTAGTAAAAATACA
AAAATTAGCCAGGCATGGTGCTATATACCTGTAGCACCAGCTACTTGGGAGACAGAGGTGGGAGAATT
ACTTGAACCTGGGAGGTTCAAGCCATGGGAGGTGGAAGTTGCAGTGAGCCGAGATGCCACTGCACTCC
AGCCTGAGCAACAGAGCAAGACTATCTCAAGAAAAGAAAGAAAGAAAGAGACTTGCCAAGGTCA
TGTATCAGGGCAAGGAAGAGCTGGGGGCCCAGCTGGCTGCTCCCCTGCTGAGCTGGGAGACCACCTTG
ATCTGACTTCTCCCATCTTCCCAGCCTAAGCCAGGCCCTGGGGTCACGGAGGCTGGGGAGGCACCGAG
 
GAACGCGCCTGGCATGTGCTGACAGGAATTTATGCTCCAGCTGGCCAGCTGGAGGAGCCTGCTG
GGCAGAGGCCAGAGCTGGGGCTCTGGAAGGTACCTGGGGAGGTTGCACTGTGAGAATGAGCTCAAG
CTGGGTCAGAGAGCAGGGCTGACTCTGCCAGTGCCTGCATCAGCCTCATCGCTCTCCTAGGCTCCTGG
CCTGCTGGACTCTGGGCTGCAGGTCCTTCTTGAAAGGCTGTGAGTAGTGAGACAAGGAGCAGGAGTGA
GGGGTGGCAGGAGAGAAGATAGAGATTGAGAGAGAGAGAGAGAGAGACAGAGAGAGAGGAAGAGAC
AGAGACAAAAGGAGAGAGAACGGCTTAGACAAGGAGAGAAAGATGGAAAGATAAAGAGACTGGGCGCA
GTGGCTCACGCCTGTAATCCCAACACTTGGGGAGGCCAAGGTGGAGGATGGCTTGAAGGAAAGAGTC
TGAGATCAACCTGGCCAACATAGTGAGACCCCGTCTCTAAAAAAAAAGAAAAAAAAAGAAAAAAGA
AAAAAAAGTTTTTTTAAAGAGACAGAGAAAGAGACTCAGAGATTGAGACTGAGAGCAAGACAGAGAGA
GACACTCACAGGGAAGAGGGGAAGAGGAAAACGAGAAAGGGAGGAGAGTAACGGAAAGAGATAAAAAA
GAAAAGCAGGTGGCAGAGACACAGAGAGAGGGACCCAGAGAAAGCCAGACAGACGCAGGTGGCTGGCA
GCGGGCGCTGTGGGGGTCACAGTAGGGGGACCTGTC
*Exemplary variant nephrin promoter - 1, 192 bp (SEQ ID NO: 45)*

CACCTGAGGTCAGGAGTTCGAGACCAGCGTGGCCAACATGATGAAACCCCGTCTCTAGTAAAAATACA
AAAATTAGCCAGGCATGGTGCTATATACCTGTAGCACCAGCTACTTGGAGACAGAGGTGGGAGAATT
ACTTGAACCTGGGAGGTTCAAGCCATGGGAGGTGGAAGTTGCAGTGAGCCGAGATGCCACTGCACTCC
AGCCTGAGCAACAGAGCAAGACTATCTCAAGAAAAGAAAGAAAGAAAGAAAGAGACTTGCCAAGGTCA
TGTATCAGGGCAAGGAAGAGCTGGGGGCCCAGCTGGCTGCTCCCCTGCTGAGCTGGGAGACCACCTTG
ATCTGACTTCTCCCATCTTCCCAGCCTAAGCCAGGCCCTGGGGTCACGGAGGCTGGGGAGGCACCGAG
GAACGCGCCTGGCATGTGCTGACAGGGGATTTTATGCTCCAGCTGGGCCAGCTGGGAGGAGCCTGCTG
GGCAGAGGCCAGAGCTGGGGGCTCTGGAAGGTACCTGGGGGAGGTTGCACTGTGAGAATGAGCTCAAG
CTGGGTCAGAGAGCAGGGCTGACTCTGCCAGTGCCTGCATCAGCCTCATCGCTCTCCTAGGCTCCTGG
CCTGCTGGACTCTGGGCTGCAGGTCCTTCTTGAAAGGCTGTGAGTAGTGAGACAAGGAGCAGGAGTGA
GGGGTGGCAGGAGAGAAGATAGAGATTGAGAGAGAGAGAGAGAGAGACAGAGAGAGAGGAAGAGAC
AGAGACAAAAGGAGAGAGAACGGCTTAGACAAGGAGAGAAAGATGGAAAGATAAAGAGACTGGGCGCA
GTGGCTCACGCCTGTAATCCCAACACTTGGGGAGGCCAAGGTGGGAGGATGGCTTGAAGGAAAGAGTC
TGAGATCAACCTGGCCAACATAGTGAGACCCCGTCTCTAAAAAAAAAAGAAAAAAAAAAGAAAAAAGA
AAAAAAAGTTTTTTAAAGAGACAGAGAAAGAGACTCAGAGATTGAGACTGAGAGCAAGACAGAGAGA
GATACTCACAGGGAAGAGGGGAAGAGGAAAACGAGAAAGGGAGGAGAGTAACGGAAAGAGATAAAAAA
GAAAAGCAGGTGGCAGAGACACAGAGAGAGGGACCCAGAGAAAGCCAGACAGACGCAGGTGGCTGGCA
GCGGGCGCTGTGGGGGTCACAGTAGGGGGACCTGTC

A promoter disclosed herein but not part of the present invention is derived from SEQ ID NO: 1 or a variant that has at least 70% identity to SEQ ID NO: 1. Suitably, the promoter has one or more deletions compared to SEQ ID NO: 1 or a variant that has at least 70% identity to SEQ ID NO: 1. Suitably, the variant may be at least 75%, at least 80%, at least 85%, at least 90%, at least 95%, at least 96%, at least 97%, at least 98%, at least 99% identical to SEQ ID NO: 1. An exemplary variant is SEQ ID NO: 14, SEQ ID NO: 44, or SEQ ID NO: 45. Suitably, the variant of SEQ ID NO: 1 comprises one or more of the following substitutions: A493G, C1080T, G1169C, and C1249G. Suitably, the variant of SEQ ID NO: 1 comprises each of the substitutions A493G, C1080T, G1169C, and C1249G (see SEQ ID NO: 14).

Suitably, the promoter is derived from SEQ ID NO: 14, SEQ ID NO: 44, or SEQ ID NO: 45, or a variant that has at least 70% identity to SEQ ID NO: 14, SEQ ID NO: 44, or SEQ ID NO: 45. Suitably, the promoter has one or more deletions compared to SEQ ID NO: 14, SEQ ID NO: 44, or SEQ ID NO: 45, or a variant that has at least 70% identity to SEQ ID NO: 14, SEQ ID NO: 44, or SEQ ID NO: 45. Suitably, the variant may be at least 75%, at least 80%, at least 85%, at least 90%, at least 95%, at least 96%, at least 97%, at least 98%, at least 99% identical to SEQ ID NO: 14, SEQ ID NO: 44, or SEQ ID NO: 45.

Suitably, the promoter is derived from SEQ ID NO: 14, or a variant that has at least 70% identity to SEQ ID NO: 14. Suitably, the promoter has one or more deletions compared to SEQ ID NO: 14, or a variant that has at least 70% identity to SEQ ID NO: 14. Suitably, the variant may be at least 75%, at least 80%, at least 85%, at least 90%, at least 95%, at least 96%, at least 97%, at least 98%, at least 99% identical to SEQ ID NO: 14.

Suitably, the promoter comprises or consists of a nucleotide sequence according to SEQ ID NO: 1 and having one or more deletions, e.g. one or two deletions, or nucleotide sequence with at least 70%, at least 75%, at least 80%, at least 85%, at least 90%, at least 95%, at least 96%, at least 97%, at least 98%, or at least 99% identity thereto. Suitably, the promoter comprises or consists of a nucleotide sequence according to SEQ ID NO: 1 and having two or more deletions, or nucleotide sequence with at least 70%, at least 75%, at least 80%, at least 85%, at least 90%, at least 95%, at least 96%, at least 97%, at least 98%, or at least 99% identity thereto. The deletions may be any size. Suitably, the deletions are each at least 50 bp, at least 100 bp, at least 150 bp, at least 200 bp, at least 250 bp, at least 300 bp, at least 350 bp, or at least 400 bp in size. Suitably, the deletions are each from 50 to 500 bp, from 100 to 500 bp, from 150 to 500 bp, from 200 to 500 bp, from 250 to 500 bp, from 300 to 500 bp, from 350 to 500 bp, or from 400 to 500 bp in size.

In some embodiments, the promoter comprises or consists of a nucleotide sequence according to SEQ ID NO: 1, but wherein:
(i) position 1 to position n1 of SEQ ID NO: 1 is deleted, wherein n1 is an integer from 1 to 430; and/or
(ii) position n2 to position n3 of SEQ ID NO: 1 is deleted, wherein n3 ≥ n2, n2 is an integer from 508 to 1061, and n3 is an integer from 508 to 1061;
or a nucleotide sequence with at least 70%, at least 75%, at least 80%, at least 85%, at least 90%, at least 95%, at least 96%, at least 97%, at least 98%, or at least 99% identity thereto. In some embodiments:
(iii) the position corresponding to position 493 of SEQ ID NO: 1 is G, the position corresponding to position 1080 of SEQ ID NO: 1 is T, the position corresponding to position 1169 of SEQ ID NO: 1 is C, and/or the position corresponding to position 1249 of SEQ ID NO: 1 is G.

In preferred embodiments:
(iii) the position corresponding to position 493 of SEQ ID NO: 1 is G, the position corresponding to position 1080 of SEQ ID NO: 1 is T, the position corresponding to position 1169 of SEQ ID NO: 1 is C, and the position corresponding to position 1249 of SEQ ID NO: 1 is G.

Although the nucleotide positions above are referred to by reference to a position in SEQ ID NO: 1, the skilled person would be able to readily identify the corresponding nucleotide location in variants thereof, for example by aligning SEQ ID NO: 1 and the variant sequence. For example, position 493 of SEQ ID NO: 1 corresponds to position 436 of SEQ ID NO: 14; position 1080 of SEQ ID NO: 1 corresponds to position 1023 of SEQ ID NO: 14; position 1169 of SEQ ID NO: 1 corresponds to position 1112 of SEQ ID NO: 14; and position 1249 of SEQ ID NO: 1 corresponds to position 1192 of SEQ ID NO: 14.

For example, the promoter may comprise or consist of a nucleotide sequence having at least 70%, at least 75%, at least 80%, at least 85%, at least 90%, at least 95%, at least 96%, at least 97%, at least 98%, or at least 99% identity to a nucleotide sequence according to SEQ ID NO: 1, but wherein:
(i) position 1 to position n1 of SEQ ID NO: 1 is deleted, wherein n1 is an integer from 1 to 430; and/or
(ii) position n2 to position n3 of SEQ ID NO: 1 is deleted, wherein n3 ≥ n2, n2 is an integer from 508 to 1061, and n3 is an integer from 508 to 1061.

In some embodiments:
(iii) position 493 of SEQ ID NO: 1 is substituted with G, position 1080 of SEQ ID NO: 1 is substituted with T, position 1169 of SEQ ID NO: 1 is substituted with C, and/or position 1249 of SEQ ID NO: 1 is substituted with G.

In preferred embodiments:
(iii) position 493 of SEQ ID NO: 1 is substituted with G, position 1080 of SEQ ID NO: 1 is substituted with T, position 1169 of SEQ ID NO: 1 is substituted with C, and position 1249 of SEQ ID NO: 1 is substituted with G.

Suitably, n1 is an integer from 50 to 430, from 100 to 430, from 150 to 430, from 200 to 430, from 250 to 430, from 300 to 430, from 350 to 430, or from 400 to 430. In some embodiments, n1 is an integer from 100 to 430. In some embodiments, n1 = 430, i.e. position 1 to position 430 of SEQ ID NO: 1 is deleted in the promoter of the invention.

The difference between n3 and n2 specifies the size of the deletion. Suitably, n3 ≥ n2 + 49, n3 ≥ n2 + 99, n3 ≥ n2 + 149, n3 ≥ n2 + 199, n3 ≥ n2 + 249, n3 ≥ n2 + 299, n3 ≥ n2 + 349, n3 ≥ n2 + 399, n3 ≥ n2 + 449, n3 ≥ n2 + 499, or n3 ≥ n2 + 549. In some embodiments, n3 ≥ n2 + 49.

The values that n2 and n3 take determine where the deletion is. Suitably, n2 and n3 are each integers from 550 to 1050, n2 and n3 are each integers from 600 to 1000, n2 and n3 are each integers from 650 to 950, n2 and n3 are each integers from 700 to 900, n2 and n3 are integers from 750 to 850.

In some embodiments, n2 = 508 and n3 = 1061, i.e. position 508 to 1061 of SEQ ID NO: 1 is deleted in the promoter

### Promoter regions

The present inventors have determined the regions of the nephrin promoter which drive transgene expression.

A promoter typically comprises a "core" and a "proximal" region. The "core promoter region" may comprise a transcription start site, a RNA polymerase binding sites and a general transcription factor binding site. The "proximal promoter region" may comprise primary regulatory elements and specific transcription factor binding sites which are required, for example, to facilitate effective and controllable transcription. The size and components of both the core and proximal promoter regions typically vary in a gene specific manner. A promoter may also comprise a 5' untranslated region (5' UTR) (also known as a leader sequence) downstream of the core promoter region and upstream from the initiation codon. (See e.g. Figure 1).

The promoter may be a hybrid promoter. As used herein, a "hybrid promoter" comprises a combination of elements derived from different promoters. For example, a hybrid promoter may comprise a proximal promoter region derived from one pre-existing promoter and a core promoter from another pre-existing promoter to achieve the desired transgene expression. A muscle hybrid promoter is described in Piekarowicz, K., et al. (2019). Methods & clinical development, 15, 157-169.

In some embodiments, the promoter comprises (i) the nucleotide sequence shown as SEQ ID NO: 4, or a variant which is at least 70% identical to SEQ ID NO: 4. Without wishing to be bound by theory, it is considered that a nucleotide sequence having at least about 70% identity to SEQ ID NO: 4 may provide a proximal promoter region.

*Exemplary proximal promoter region (SEQ ID NO: 4)*

GGCCCTGGGGTCACGGAGGCTGGGGAGGCACCGAGGAACGCGCCTGGCATGTGCTGACAGGGAATTTT
ATGCTCCAG

Suitably, the variant may be at least 75%, at least 80%, at least 85%, at least 90%, at least 95%, at least 96%, at least 97%, at least 98%, at least 99% identical to SEQ ID NO: 4. The promoter may comprise a variant of SEQ ID NO: 4 shown as SEQ ID NO: 17. Suitably, a variant of SEQ ID NO: 4 comprises the substitution A63G (see SEQ ID NO: 17).

In some embodiments, the promoter comprises (i) the nucleotide sequence shown as SEQ ID NO: 17, or a variant which is at least 70% identical to SEQ ID NO: 17. Suitably, the variant may be at least 75%, at least 80%, at least 85%, at least 90%, at least 95%, at least 96%, at least 97%, at least 98%, at least 99% identical to SEQ ID NO: 17.

*Exemplary variant proximal promoter region (SEQ ID NO: 17)*

GGCCCTGGGGTCACGGAGGCTGGGGAGGCACCGAGGAACGCGCCTGGCATGTGCTGACAGGGGATTTT
ATGCTCCAG

In some embodiments, the promoter comprises (ii) the nucleotide sequence shown as SEQ ID NO: 5 or 18, or a variant which is at least 70% identical to SEQ ID NO: 5 or 18; the nucleotide sequence shown as SEQ ID NO: 6 or 19, or a variant which is at least 70% identical to SEQ ID NO: 6 or 19; and/or the nucleotide sequence shown as SEQ ID NO: 7 or 20, or a variant which is at least 70% identical to SEQ ID NO: 7 or 20.

In some embodiments, the promoter comprises (ii) the nucleotide sequence shown as SEQ ID NO: 5, or a variant which is at least 70% identical to SEQ ID NO: 5. Without wishing to be bound by theory, it is considered that a nucleotide sequence having at least about 70% identity to SEQ ID NO: 5 may provide a core promoter region.

*Exemplary core promoter region (SEQ ID NO: 5)* GAGCAAGACAGAGAGAGACACTCACAGGGAAG

Suitably, the variant may be at least 75%, at least 80%, at least 85%, at least 90%, at least 95%, at least 96%, at least 97%, at least 98%, at least 99% identical to SEQ ID NO: 5. The promoter may comprise a variant of SEQ ID NO: 5 shown as SEQ ID NO: 18. Suitably, a variant of SEQ ID NO: 5 comprises the substitution C19T (see SEQ ID NO: 18).

In some embodiments, the promoter comprises (ii) the nucleotide sequence shown as SEQ ID NO: 18, or a variant which is at least 70% identical to SEQ ID NO: 18. Suitably, the variant may be at least 75%, at least 80%, at least 85%, at least 90%, at least 95%, at least 96%, at least 97%, at least 98%, at least 99% identical to SEQ ID NO: 18.

*Exemplary variant core promoter region (SEQ ID NO: 18)*
GAGCAAGACAGAGAGAGATACTCACAGGGAAG

In some embodiments, the promoter comprises (ii) the nucleotide sequence shown as SEQ ID NO: 6, or a variant which is at least 70% identical to SEQ ID NO: 6. Without wishing to be bound by theory, it is considered that a nucleotide sequence having at least about 70% identity to SEQ ID NO: 6 may provide a 5'UTR.

*Exemplary 5'UTR (SEQ ID NO: 6)*

AGGGGAAGAGGAAAACGAGAAAGGAGGAGAGTAACGGAAAGAGATAAAAAAGAAAAGCAGGTGGCAG
AGACACAGAGAGAGGGACCCAGAGAAAGCCAGACAGACGCAGGTGGCTGGCAGCGGGCGCTGTGGGGG
TCACAGTAGGGGGACCTGTC

Suitably, the variant may be at least 75%, at least 80%, at least 85%, at least 90%, at least 95%, at least 96%, at least 97%, at least 98%, at least 99% identical to SEQ ID NO: 6. The promoter may comprise a variant of SEQ ID NO: 6 shown as SEQ ID NO: 19. Suitably, a variant of SEQ ID NO: 6 comprises the substitutions G76C and/or C156G (see SEQ ID NO: 19).

In some embodiments, the promoter comprises (ii) the nucleotide sequence shown as SEQ ID NO: 19, or a variant which is at least 70% identical to SEQ ID NO: 19. Suitably, the variant may be at least 75%, at least 80%, at least 85%, at least 90%, at least 95%, at least 96%, at least 97%, at least 98%, at least 99% identical to SEQ ID NO: 19.

*Exemplary variant 5'UTR (SEQ ID NO: 19)*

AGGGGAAGAGGAAAACGAGAAAGGAGGAGAGTAACGGAAAGAGATAAAAAAGAAAAGCAGGTGGCAG
AGACACACAGAGAGGGACCCAGAGAAAGCCAGACAGACGCAGGTGGCTGGCAGCGGGCGCTGTGGGGG
TCACAGTAGGGGGACCTGTG

In some embodiments, the promoter comprises (ii) the nucleotide sequence shown as SEQ ID NO: 7, or a variant which is at least 70% identical to SEQ ID NO: 7. Without wishing to be bound by theory, it is considered that a nucleotide sequence having at least about 70% identity to SEQ ID NO: 7 may provide a core promoter region and a 5'UTR.

*Exemplary core promoter region and 5'UTR (SEQ ID NO: 7)*

GAGCAAGACAGAGAGAGACACTCACAGGAAGAGGGAAGAGGAAAACGAGAAAGGAGGAGAGTAAC
GGAAAGAGATAAAAAAGAAAAGCAGGTGGCAGAGACACAGAGAGAGGGACCCAGAGAAAGCCAGACAG
ACGCAGGTGGCTGGCAGCGGGCGCTGTGGGGGTCACAGTAGGGGGACCTGTC

Suitably, the variant may be at least 75%, at least 80%, at least 85%, at least 90%, at least 95%, at least 96%, at least 97%, at least 98%, at least 99% identical to SEQ ID NO: 7. The promoter may comprise a variant of SEQ ID NO: 7 shown as SEQ ID NO: 20. Suitably, a variant of SEQ ID NO: 7 comprises the substitutions C19T, G108C and/or C188G (see SEQ ID NO: 20). Suitably, a variant of SEQ ID NO: 7 comprises the substitutions C19T, G108C and C188G (see SEQ ID NO: 20).

In some embodiments, the promoter comprises (ii) the nucleotide sequence shown as SEQ ID NO: 20, or a variant which is at least 70% identical to SEQ ID NO: 20. Suitably, the variant may be at least 75%, at least 80%, at least 85%, at least 90%, at least 95%, at least 96%, at least 97%, at least 98%, at least 99% identical to SEQ ID NO: 20.

*Exemplary variant core promoter region and 5'UTR (SEQ ID NO: 20)*

GAGCAAGACAGAGAGAGATACTCACAGGAAGAGGGAAGAGGAAAACGAGAAAGGAGGAGAGTAAC
GGAAAGAGATAAAAAAGAAAAGCAGGTGGCAGAGACACACAGAGAGGACCCAGAGAAAGCCAGACAG
ACGCAGGTGGCTGGCAGCGGGCGCTGTGGGGGTCACAGTAGGGGGACCTGTG

In some embodiments, the promoter comprises (iii) a nucleotide sequence having at least 70% identity to SEQ ID NO: 8, or one or more fragments thereof. Suitably, the promoter comprises a nucleotide sequence having at least about 70% identity to SEQ ID NO: 8, or one or more fragments thereof, immediately downstream of the proximal promoter region and/or immediately upstream of the core promoter region.

The promoter may comprise a nucleotide sequence that has at least 70%, at least 75%, at least 80%, at least 85%, at least 90%, at least 95%, at least 96%, at least 97%, at least 98% or at least 99% identity to SEQ ID NO: 8, or one or more fragments thereof. The promoter may comprise the nucleotide sequence of SEQ ID NO: 8, or one or more fragments thereof.

*Exemplary optional promoter region (SEQ ID NO: 8)* 
CTGGGCCAGCTGGAGGAGCCTGCTGGGCAGAGGCCAGAGCTGGGGCTCTGGAAGGTACCTGGGGA
GGTTGCACTGTGAGAATGAGCTCAAGCTGGGTCAGAGAGCAGGGCTGACTCTGCCAGTGCCTGCATCA
GCCTCATCGCTCTCCTAGGCTCCTGGCCTGCTGGACTCTGGGCTGCAGGTCCTTCTTGAAAGGCTGTG
AGTAGTGAGACAAGGAGCAGGAGTGAGGGGTGGCAGGAGAGAAGATAGAGATTGAGAGAGAGAGAGAG
AGAGACAGAGAGAGAGGAAGAGACAGAGACAAAAGGAGAGAGAACGGCTTAGACAAGGAGAGAAAGAT
GGAAAGATAAAGAGACTGGGCGCAGTGGCTCACGCCTGTAATCCCAACACTTGGGGAGGCCAAGGTGG
GAGGATGGCTTGAAGGAAAGAGTCTGAGATCAACCTGGCCAACATAGTGAGACCCCGTCTCTAAAAAA
AAAAAAGAAAAAAAAAAGAAAAAAGAAAAAAAAGTTTTTTAAAGAGACAGAGAAAGAGACTCAGAGA
TTGAGACTGA
 Suitably, the one or more fragments are (a) a 5' terminal fragment; and/or (b) a 3' terminal fragment. Suitably, the 5' terminal fragment may be immediately downstream of the proximal promoter region. Suitably, the 3' terminal fragment may be immediately upstream of the core promoter region. For example, the promoter may comprise
(a) a nucleotide sequence having at least 70% to positions 1 to x of SEQ ID NO: 8; and/or
(b) a nucleotide sequence having at least 70% identity to positions y to 554 of SEQ ID NO: 8;
wherein x and y are integers, and y > x.

The fragment(s) of SEQ ID NO: 8 may be any length. Suitably, the fragment(s) may have a length of about 500 bp or less, 450 bp or less, 400 bp or less, 350 bp or less, 300 bp or less, 250 bp or less, 200 bp or less, 150 bp or less, 100 bp or less, 50 bp or less, 40 bp or less, 30 bp or less, 20 bp or less, or 10 bp or less.

In some embodiments, the promoter does not comprise SEQ ID NO: 8.

In some embodiments, the promoter comprises a nucleotide sequence having at least 70% identity to SEQ ID NO: 9, or a fragment thereof. Suitably, the promoter comprises a nucleotide sequence having at least about 70% identity to SEQ ID NO: 9, or a fragment thereof, immediately upstream of the proximal promoter region.

The promoter may comprise a nucleotide sequence that has at least 70%, at least 75%, at least 80%, at least 85%, at least 90%, at least 95%, at least 96%, at least 97%, at least 98% or at least 99% identity to SEQ ID NO: 9, or a fragment thereof. The promoter may comprise the nucleotide sequence of SEQ ID NO: 9, or one or more fragments thereof.

*Exemplary optional upstream promoter region (SEQ ID NO: 9)*

CCTGCAGGGCCCACTAGTCTGTAATCCCAGCATTTGGGAGGCTGAGGCAGATGGATCACCTGAGGTC
AGGAGTTCGAGACCAGCCTGGCCAACATGATGAAACCCCGTCTCTAGTAAAAATACAAAAATTAGCCA
GGCATGGTGCTATATACCTGTAGTACCAGCTACTTGGGAGACAGAGGTGGGAGAATTACTTGAACCTG
GGAGGTTCAAGCCATGGGAGGTGGAAGTTGCAGTGAGCCGAGATGCCACTGCACTCCAGCCTGAGCAA
CAGAGCAAGACTATCTCAAGAAAAAAAAGAAAGAAAGAAAGGGACTTGCCAAGGTCATGTATCAGGGC
AAGGAAGAGCTGGGGGCCCAGCTGGCTGCTCCCCTGCTGAGCTGGGAGACCACCTTGATCTGACTTCT
CCCATCTTCCCAGCCTAAGCCA

Suitably, the fragment is a 3' terminal fragment. For example, the promoter may comprise a nucleotide sequence having at least 70% identity to positions z to 430 of SEQ ID NO: 9, wherein z is an integer.

The fragment of SEQ ID NO: 9 may be any length. Suitably, the fragment may have a length of about 400 bp or less, 350 bp or less, 300 bp or less, 250 bp or less, 200 bp or less, 150 bp or less, 100 bp or less, 50 bp or less, 40 bp or less, 30 bp or less, 20 bp or less, or 10 bp or less.

In some embodiments, the promoter does not comprise SEQ ID NO: 9.

In some embodiments, the promoter comprises or consists of from 5 to 3':
(i) a nucleotide sequence having at least 70%, at least 75%, at least 80%, at least 85%, at least 90%, at least 95%, at least 96%, at least 97%, at least 98%, at least 99%, or 100% identity to SEQ ID NO: 4 or 17;
(iii) optionally a nucleotide sequence having at least 70%, at least 75%, at least 80%, at least 85%, at least 90%, at least 95%, at least 96%, at least 97%, at least 98%, at least 99%, or 100% identity to SEQ ID NO: 8, or one or more fragments thereof; and
(ii) a nucleotide sequence having at least 70%, at least 75%, at least 80%, at least 85%, at least 90%, at least 95%, at least 96%, at least 97%, at least 98%, at least 99%, or 100% identity to SEQ ID NO: 5 or 18, a nucleotide sequence having at least 70%, at least 75%, at least 80%, at least 85%, at least 90%, at least 95%, at least 96%, at least 97%, at least 98%, at least 99%, or 100% identity to SEQ ID NO: 6 or 19, and/or a nucleotide sequence having at least 70%, at least 75%, at least 80%, at least 85%, at least 90%, at least 95%, at least 96%, at least 97%, at least 98%, at least 99%, or 100% identity to SEQ ID NO: 7 or 20.

In some embodiments, the promoter comprises or consists of from 5 to 3':
(i) a nucleotide sequence having at least 90% identity to SEQ ID NO: 4 or 17;
(iii) optionally a nucleotide sequence having at least 90% identity to SEQ ID NO: 8, or one or more fragments thereof; and
(ii) a nucleotide sequence having at least 90% identity to SEQ ID NO: 5 or 18, a nucleotide sequence having at least 90% identity to SEQ ID NO: 6 or 19, and/or a nucleotide sequence having at least 90% identity to SEQ ID NO: 7 or 20.

In some embodiments, the promoter comprises or consists of from 5 to 3':
(i) a nucleotide sequence having at least 70%, at least 75%, at least 80%, at least 85%, at least 90%, at least 95%, at least 96%, at least 97%, at least 98%, at least 99%, or 100% identity to SEQ ID NO: 4 or 17;
(iii) optionally (a) a nucleotide sequence having at least 70%, at least 75%, at least 80%, at least 85%, at least 90%, at least 95%, at least 96%, at least 97%, at least 98%, at least 99%, or 100% identity to a 5' terminal fragment of SEQ ID NO: 8; and/or (b) a nucleotide sequence having at least 70%, at least 75%, at least 80%, at least 85%, at least 90%, at least 95%, at least 96%, at least 97%, at least 98%, at least 99%, or 100% identity to a 3' terminal fragment of SEQ ID NO: 8; and
(ii) a nucleotide sequence having at least 70%, at least 75%, at least 80%, at least 85%, at least 90%, at least 95%, at least 96%, at least 97%, at least 98%, at least 99%, or 100% identity to SEQ ID NO: 5 or 18, a nucleotide sequence having at least 70%, at least 75%, at least 80%, at least 85%, at least 90%, at least 95%, at least 96%, at least 97%, at least 98%, at least 99%, or 100% identity to SEQ ID NO: 6 or 19, and/or a nucleotide sequence having at least 70%, at least 75%, at least 80%, at least 85%, at least 90%, at least 95%, at least 96%, at least 97%, at least 98%, at least 99%, or 100% identity to SEQ ID NO: 7 or 20.

In some embodiments, the promoter comprises or consists of from 5 to 3':
(i) a nucleotide sequence having at least 90% identity to SEQ ID NO: 4 or 17;
(iii) optionally (a) a nucleotide sequence having at least 90% identity to a 5' terminal fragment of SEQ ID NO: 8; and/or (b) a nucleotide sequence having at least 90% identity to a 3' terminal fragment of SEQ ID NO: 8; and
(ii) a nucleotide sequence having at least 90% identity to SEQ ID NO: 5 or 18, a nucleotide sequence having at least 90% identity to SEQ ID NO: 6 or 19, and/or a nucleotide sequence having at least 90% identity to SEQ ID NO: 7 or 20.

### Promoter elements

The present inventors have determined the functional elements of the nephrin promoter which drive transgene expression.

The promoter of the invention may comprise one or more of the following elements: (a) a retinoic acid receptor binding site; (b) a WT1 binding site; (c) an enhancer box; (d) a transcription factor binding region; and (e) a transcription initiation site.

Suitably, the promoter of the invention comprises all of the following elements: (a) a retinoic acid receptor binding site; (b) a WT1 binding site; (c) an enhancer box; (d) a transcription factor binding region; and (e) a transcription initiation site.

A retinoic acid receptor (RAR) binding site refers to a polynucleotide sequence which is capable of binding RAR alpha, RAR beta, and/or RAR gamma. The RAR binding site may comprise or consist of a nucleotide sequence shown as SEQ ID NO: 10, or a nucleotide sequence having one or two substitutions, deletions, or insertions compared to SEQ ID NO: 10. The substitutions, deletions, or insertions may be any substitution, deletion, or insertion of a single nucleotide such that the RAR binding site retains at least one of its endogenous functions.

*Exemplary RAR binding site (SEQ ID NO: 10)*
GGGGTCA

A WT1 binding site refers to a polynucleotide sequence which is capable of binding the zinc finger polypeptide encoded by the Wilms' tumor suppressor gene, WT1. The WT1 binding site may comprise or consist of a nucleotide sequence shown as SEQ ID NO: 11, or a nucleotide sequence having one, two or three substitutions, deletions, or insertions compared to SEQ ID NO: 11. The substitutions, deletions, or insertions may be any substitution, deletion, or insertion of a single nucleotide such that the WT1 binding region retains at least one of its endogenous functions.

*Exemplary WT1 binding site (SEQ ID NO: 11)*
CGGAGGCTGGGGAGGCA

An enhancer box refers to a DNA response element found in some eukaryotes that acts as a protein-binding site. The enhancer box may comprise or consist of a nucleotide sequence shown as SEQ ID NO: 12, or a nucleotide sequence having one or two substitutions, deletions, or insertions compared to SEQ ID NO: 12. The substitutions, deletions, or insertions may be any substitution, deletion, or insertion of a single nucleotide such that the enhancer box retains at least one of its endogenous functions.

*Exemplary enhancer box (SEQ ID NO: 12)*
ATGTG

One or more of (a) a retinoic acid receptor binding site; (b) a WT1 binding site; and (c) an enhancer box may be present in the proximal promoter region. Suitably, each of (a) a retinoic acid receptor binding site; (b) a WT1 binding site; and (c) an enhancer box are present in the proximal promoter region

In some embodiments, one or more of the following elements is present in (i) the nucleotide sequence having at least 70% identity to SEQ ID NO: 4 or 17: (a) a RAR binding site at a position corresponding approximately to position 7 to position 13 of SEQ ID NO: 4 or 17; (b) a WT1 binding site at a position corresponding approximately to position 14 to position 30 of SEQ ID NO: 4 or 17; and (c) an enhancer box at a position corresponding approximately to position 49 to position 53 of SEQ ID NO: 4 or 17. In some embodiments, each of the elements are present in (i) the nucleotide sequence having at least 70% identity to SEQ ID NO: 4 or 17.

In some embodiments, one or more of the following nucleotide sequences is present in (i) the nucleotide sequence having at least 70% identity to SEQ ID NO: 4 or 17: (a) GGGGTCA at a position corresponding to position 7 to position 13 of SEQ ID NO: 4 or 17; (b) CGGAGGCTGGGGAGGCA at a position corresponding to position 14 to position 30 of SEQ ID NO: 4 or 17; and (c) ATGTG at a position corresponding to position 49 to position 53 of SEQ ID NO: 4 or 17. In some embodiments, each of the nucleotide sequences are present in (i) the nucleotide sequence having at least 70% identity to SEQ ID NO: 4 or 17.

Suitably, the promoter may comprise a transcription factor binding region comprising or consisting of a nucleotide sequence shown as SEQ ID NO: 13 or a nucleotide sequence having one, two, three, four or five substitutions, deletions, or insertions compared to SEQ ID NO: 13. The substitutions, deletions, or insertions may be any substitution, deletion, or insertion of a single nucleotide such that the transcription factor binding region retains at least one of its endogenous functions. An exemplary variant is shown in SEQ ID NO: 43. Suitably, a variant of SEQ ID NO: 13 comprises the substitution C19T (see SEQ ID NO: 43).

*Exemplary transcription factor binding region (SEQ ID NO: 13)*
GAGCAAGACAGAGAGAGACACTCACAGGGA

Suitably, the promoter may comprise a transcription factor binding region comprising or consisting of a nucleotide sequence shown as SEQ ID NO: 43 or a nucleotide sequence having one, two, three, four or five substitutions, deletions, or insertions compared to SEQ ID NO: 43. The substitutions, deletions, or insertions may be any substitution, deletion, or insertion of a single nucleotide such that the transcription factor binding region retains at least one of its endogenous functions.

*Exemplary transcription factor binding region (SEQ ID NO: 43)*
GAGCAAGACAGAGAGAGATACTCACAGGGA

Other suitable transcription factor binding regions will be well known to those of skill in the art. For example, other suitable transcription factor binding regions include TACGAT (SEQ ID NO: 37), TATAAT (SEQ ID NO: 38), GATACT (SEQ ID NO: 39), TATGAT (SEQ ID NO: 40), and TATGTT (SEQ ID NO: 41).

Suitably, the promoter may comprise a transcription initiation site which comprises or consists of an "AG" dinucleotide.

Suitably, the transcription factor binding site is operably linked to the transcription initiation site. Suitably, the transcription factor binding site may be directly upstream of the transcription initiation site. Without wishing to be bound by theory, it is considered that the transcription factor binding site and the transcription initiation site may provide a core promoter region. Suitably the promoter may comprise a 5' untranslated region. The 5' untranslated region may comprise or consist of a nucleotide sequence having at least about 70%, 80%, 90%, 95% or 99% sequence identity to SEQ ID NO: 6 or 19. The 5' untranslated region may comprise or consist of SEQ ID NO: 6 or 19.

Suitably, the 5' untranslated region is operably linked to the transcription initiation site. Suitably, the 5' untranslated region may be directly downstream of the transcription initiation site.

In some embodiments, the promoter comprises or consists of from 5 to 3':
(i) a nucleotide sequence having at least 70%, at least 75%, at least 80%, at least 85%, at least 90%, at least 95%, at least 96%, at least 97%, at least 98%, at least 99%, or 100% identity to SEQ ID NO: 4 or 17, wherein each of the following elements is present: (a) a RAR binding site at a position corresponding approximately to position 7 to position 13 of SEQ ID NO: 4 or 17; (b) a WT1 binding site at a position corresponding approximately to position 14 to position 30 of SEQ ID NO: 4 or 17; and (c) an enhancer box at a position corresponding approximately to position 49 to position 53 of SEQ ID NO: 4 or 17;
(iii) optionally a nucleotide sequence having at least 70%, at least 75%, at least 80%, at least 85%, at least 90%, at least 95%, at least 96%, at least 97%, at least 98%, at least 99%, or 100% identity to SEQ ID NO: 8, or one or more fragments thereof; and (ii) a nucleotide sequence having at least 70%, at least 75%, at least 80%, at least 85%, at least 90%, at least 95%, at least 96%, at least 97%, at least 98%, at least 99%, or 100% identity to SEQ ID NO: 5 or 18, a nucleotide sequence having at least 70%, at least 75%, at least 80%, at least 85%, at least 90%, at least 95%, at least 96%, at least 97%, at least 98%, at least 99%, or 100% identity to SEQ ID NO: 6 or 19, or a nucleotide sequence having at least 70%, at least 75%, at least 80%, at least 85%, at least 90%, at least 95%, at least 96%, at least 97%, at least 98%, at least 99%, or 100% identity to SEQ ID NO: 7 or 20.

In some embodiments, the promoter comprises or consists of from 5 to 3':
(i) a nucleotide sequence having at least 90% identity to SEQ ID NO: 4 or 17, wherein each of the following elements is present: (a) a RAR binding site at a position corresponding approximately to position 7 to position 13 of SEQ ID NO: 4 or 17; (b) a WT1 binding site at a position corresponding approximately to position 14 to position 30 of SEQ ID NO: 4 or 17; and (c) an enhancer box at a position corresponding approximately to position 49 to position 53 of SEQ ID NO: 4 or 17;
(iii) optionally a nucleotide sequence having at least 90% identity to SEQ ID NO: 8, or one or more fragments thereof; and
(ii) a nucleotide sequence having at least 90% identity to SEQ ID NO: 5 or 18, a nucleotide sequence having at least 90% identity to SEQ ID NO: 6 or 19, or a nucleotide sequence having at least 90% identity to SEQ ID NO: 7 or 20.

### Exemplary promoters

SEQ ID NO: 2 provides an exemplary minimal nephrin promoter which is not covered by the subject-matter of the claims.
*Exemplary minimal nephrin promoter - 819 bp (SEQ ID NO: 2)* 
GGCCCTGGGGTCACGGAGGCTGGGAGGCACCGAGGAACGCGCCTGGCATGTGCTGACAGGAATTTT
ATGCTCCAGCTGGCCAGCTGGAGGAGCCTGCTGGGCAGAGGCCAGAGCTGGGGCTCTGGAAGGTA
CCTGGGGGAGGTTGCACTGTGAGAATGAGCTCAAGCTGGGTCAGAGAGCAGGGCTGACTCTGCCAGTG
CCTGCATCAGCCTCATCGCTCTCCTAGGCTCCTGGCCTGCTGGACTCTGGGCTGCAGGTCCTTCTTGA
AAGGCTGTGAGTAGTGAGACAAGGAGCAGGAGTGAGGGGTGGCAGGAGAGAAGATAGAGATTGAGAGA
GAGAGAGAGAGACAGAGAGAGAGGAAGAGACAGAGACAAAAGGAGAGAGAACGGCTTAGACAAGGA
GAGAAAGATGGAAAGATAAAGAGACTGGGCGCAGTGGCTCACGCCTGTAATCCCAACACTTGGGGAGG
CCAAGGTGGGAGGATGGCTTGAAGGAAAGAGTCTGAGATCAACCTGGCCAACATAGTGAGACCCCGTC
TCTAAAAAAAAAAAAGAAAAAAAAAAGAAAAAAGAAAAAAAAGTTTTTTAAAGAGACAGAGAAAGAG
ACTCAGAGATTGAGACTGAGAGCAAGACAGAGAGAGACACTCACAGGAAGAGGGGAAGAGGAAAACG
AGAAAGGGAGGAGAGTAACGGAAAGAGATAAAAAAGAAAAGCAGGTGGCAGAGACACAGAGAGAGGGA
CCCAGAGAAAGCCAGACAGACGCAGGTGGCTGGCAGCGGGCGCTGTGGGGGTCACAGTAGGGGGACCT
GTC
 SEQ ID NOs: 15, 46, 58, 59 and 60 provide exemplary minimal nephrin promoter variants which are not covered by the subject-matter of the claims.
*Exemplary minimal nephrin promoter variant - 819 bp (SEQ ID NO: 15)* 
GGCCCTGGGGTCACGGAGGCTGGGAGGCACCGAGGAACGCGCCTGGCATGTGCTGACAGGGGATTTT
ATGCTCCAGCTGGCCAGCTGGGAGGAGCCTGCTGGGCAGAGGCCAGAGCTGGGGGCTCTGGAAGGTA
CCTGGGGGAGGTTGCACTGTGAGAATGAGCTCAAGCTGGGTCAGAGAGCAGGGCTGACTCTGCCAGTG
CCTGCATCAGCCTCATCGCTCTCCTAGGCTCCTGGCCTGCTGGACTCTGGGCTGCAGGTCCTTCTTGA
AAGGCTGTGAGTAGTGAGACAAGGAGCAGGAGTGAGGGGTGGCAGGAGAGAAGATAGAGATTGAGAGA
GAGAGAGAGAGAGACAGAGAGAGAGGAAGAGACAGAGACAAAAGGAGAGAGAACGGCTTAGACAAG
GAGAGAAAGATGGAAAGATAAAGAGACTGGGCGCAGTGGCTCACGCCTGTAATCCCAACACTTGGGGA
GGCCAAGGTGGGAGGATGGCTTGAAGGAAAGAGTCTGAGATCAACCTGGCCAACATAGTGAGACCCCG
TCTCTAAAAAAAAAAGAAAAAAAAAAGAAAAAAGAAAAAAAAGTTTTTTAAAGAGACAGAGAAAGAG
ACTCAGAGATTGAGACTGAGAGCAAGACAGAGAGAGATACTCACAGGAAGAGGGGAAGAGGAAAACG
AGAAAGGAGGAGAGTAACGGAAAGAGATAAAAAAGAAAAGCAGGTGGCAGAGACACACAGAGAGGGA
CCCAGAGAAAGCCAGACAGACGCAGGTGGCTGGCAGCGGGCGCTGTGGGGGTCACAGTAGGGGGACCT
GTG
*Exemplary minimal nephrin promoter variant - 819 bp (SEQ ID NO: 46)* 
GGCCCTGGGGTCACGGAGGCTGGGAGGCACCGAGGAACGCGCCTGGCATGTGCTGACAGGGGATTTT
ATGCTCCAGCTGGGCCAGCTGGGAGGAGCCTGCTGGGCAGAGGCCAGAGCTGGGGGCTCTGGAAGGTA
CCTGGGGGAGGTTGCACTGTGAGAATGAGCTCAAGCTGGGTCAGAGAGCAGGGCTGACTCTGCCAGTG
CCTGCATCAGCCTCATCGCTCTCCTAGGCTCCTGGCCTGCTGGACTCTGGGCTGCAGGTCCTTCTTGA
AAGGCTGTGAGTAGTGAGACAAGGAGCAGGAGTGAGGGGTGGCAGGAGAGAAGATAGAGATTGAGAGA
GAGAGAGAGAGACAGAGAGAGAGGAAGAGACAGAGACAAAAGGAGAGAGAACGGCTTAGACAAGGA
GAGAAAGATGGAAAGATAAAGAGACTGGGCGCAGTGGCTCACGCCTGTAATCCCAACACTTGGGAGG
CCAAGGTGGGAGGATGGCTTGAAGGAAAGAGTCTGAGATCAACCTGGCCAACATAGTGAGACCCCGTC
TCTAAAAAAAAAAAAGAAAAAAAAAAGAAAAAAGAAAAAAAAGTTTTTTAAAGAGACAGAGAAAGAG
ACTCAGAGATTGAGACTGAGAGCAAGACAGAGAGAGATACTCACAGGAAGAGGGGAAGAGGAAAACG
AGAAAGGAGGAGAGTAACGGAAAGAGATAAAAAAGAAAAGCAGGTGGCAGAGACACAGAGAGAGGGA
CCCAGAGAAAGCCAGACAGACGCAGGTGGCTGGCAGCGGGCGCTGTGGGGGTCACAGTAGGGGGACCT
GTC
*Exemplary minimal nephrin promoter - 818 bp (SEQ ID NO: 58)* 
GGCCCTGGGGTCACGGAGGCTGGGAGGCACCGAGGAACGCGCCTGGCATGTGCTGACAGGAATTTT
ATGCTCCAGCTGGCCAGCTGGAGGAGCCTGCTGGGCAGAGGCCAGAGCTGGGGCTCTGGAAGGTA
CCTGGGGGAGGTTGCACTGTGAGAATGAGCTCAAGCTGGGTCAGAGAGCAGGGCTGACTCTGCCAGTG
CCTGCATCAGCCTCATCGCTCTCCTAGGCTCCTGGCCTGCTGGACTCTGGGCTGCAGGTCCTTCTTGA
AAGGCTGTGAGTAGTGAGACAAGGAGCAGGAGTGAGGGGTGGCAGGAGAGAAGATAGAGATTGAGAGA
GAGAGAGAGAGACAGAGAGAGAGGAAGAGACAGAGACAAAAGGAGAGAGAACGGCTTAGACAAGGA
GAGAAAGATGGAAAGATAAAGAGACTGGGCGCAGTGGCTCACGCCTGTAATCCCAACACTTGGGGAGG
CCAAGGTGGGAGGATGGCTTGAAGGAAAGAGTCTGAGATCAACCTGGCCAACATAGTGAGACCCCGTC
TCTAAAAAAAAAAAAGAAAAAAAAAAGAAAAAAGAAAAAAAAGTTTTTTAAAGAGACAGAGAAAGAG
ACTCAGAGATTGAGACTGAGAGCAAGACAGAGAGAGACACTCACAGGAAGAGGGGAAGAGGAAAACG
AGAAAGGGAGGAGAGTAACGGAAAGAGATAAAAAAGAAAAGCAGGTGGCAGAGACACAGAGAGAGGGA
CCCAGAGAAAGCCAGACAGACGCAGGTGGCTGGCAGCGGGCGCTGTGGGGGTCACAGTAGGGGGACCT
GT
*Exemplary minimal nephrin promoter variant - 818 bp (SEQ ID NO: 59)* 
GGCCCTGGGGTCACGGAGGCTGGGAGGCACCGAGGAACGCGCCTGGCATGTGCTGACAGGGGATTTT
ATGCTCCAGCTGGCCAGCTGGGAGGAGCCTGCTGGGCAGAGGCCAGAGCTGGGGGCTCTGGAAGGTA
CCTGGGGGAGGTTGCACTGTGAGAATGAGCTCAAGCTGGGTCAGAGAGCAGGGCTGACTCTGCCAGTG
CCTGCATCAGCCTCATCGCTCTCCTAGGCTCCTGGCCTGCTGGACTCTGGGCTGCAGGTCCTTCTTGA
AAGGCTGTGAGTAGTGAGACAAGGAGCAGGAGTGAGGGGTGGCAGGAGAGAAGATAGAGATTGAGAGA
GAGAGAGAGAGAGACAGAGAGAGAGGAAGAGACAGAGACAAAAGGAGAGAGAACGGCTTAGACAAG
GAGAGAAAGATGGAAAGATAAAGAGACTGGGCGCAGTGGCTCACGCCTGTAATCCCAACACTTGGGGA
GGCCAAGGTGGGAGGATGGCTTGAAGGAAAGAGTCTGAGATCAACCTGGCCAACATAGTGAGACCCCG
TCTCTAAAAAAAAAAGAAAAAAAAAAGAAAAAAGAAAAAAAAGTTTTTTAAAGAGACAGAGAAAGAG
ACTCAGAGATTGAGACTGAGAGCAAGACAGAGAGAGATACTCACAGGAAGAGGGGAAGAGGAAAACG
AGAAAGGGAGGAGAGTAACGGAAAGAGATAAAAAAGAAAAGCAGGTGGCAGAGACACACAGAGAGGGA
CCCAGAGAAAGCCAGACAGACGCAGGTGGCTGGCAGCGGGCGCTGTGGGGGTCACAGTAGGGGGACCT
GT
*Exemplary minimal nephrin promoter variant - 818 bp (SEQ ID NO: 60)*

GGCCCTGGGGTCACGGAGGCTGGGAGGCACCGAGGAACGCGCCTGGCATGTGCTGACAGGGGATTTT
ATGCTCCAGCTGGGCCAGCTGGAGGAGCCTGCTGGGCAGAGGCCAGAGCTGGGGGCTCTGGAAGGTA
CCTGGGGGAGGTTGCACTGTGAGAATGAGCTCAAGCTGGGTCAGAGAGCAGGGCTGACTCTGCCAGTG
CCTGCATCAGCCTCATCGCTCTCCTAGGCTCCTGGCCTGCTGGACTCTGGGCTGCAGGTCCTTCTTGA
AAGGCTGTGAGTAGTGAGACAAGGAGCAGGAGTGAGGGGTGGCAGGAGAGAAGATAGAGATTGAGAGA
GAGAGAGAGAGACAGAGAGAGAGGAAGAGACAGAGACAAAAGGAGAGAGAACGGCTTAGACAAGGA
GAGAAAGATGGAAAGATAAAGAGACTGGGCGCAGTGGCTCACGCCTGTAATCCCAACACTTGGGGAGG
CCAAGGTGGGAGGATGGCTTGAAGGAAAGAGTCTGAGATCAACCTGGCCAACATAGTGAGACCCCGTC
TCTAAAAAAAAAAAAGAAAAAAAAAAGAAAAAAGAAAAAAAAGTTTTTTAAAGAGACAGAGAAAGAG
ACTCAGAGATTGAGACTGAGAGCAAGACAGAGAGAGATACTCACAGGAAGAGGGGAAGAGGAAAACG
AGAAAGGGAGGAGAGTAACGGAAAGAGATAAAAAAGAAAAGCAGGTGGCAGAGACACAGAGAGAGGGA
CCCAGAGAAAGCCAGACAGACGCAGGTGGCTGGCAGCGGGCGCTGTGGGGGTCACAGTAGGGGGACCT
GT

In some embodiments, the promoter of the present invention comprises or consists of the nucleotide sequence shown as SEQ ID NO: 3, or a variant which is at least 90% identical to SEQ ID NO: 3.

In some embodiments, the promoter of the present invention comprises the nucleotide sequence shown as SEQ ID NO: 3, or a variant which is at least 90% identical to SEQ ID NO: 3 and wherein the promoter has a length of 1.1 kb or less.

In some embodiments, the promoter of the present invention consists of the nucleotide sequence shown as SEQ ID NO: 3, or a variant which is at least 90% identical to SEQ ID NO: 3.

*Exemplary minimal nephrin promoter -* 265 *bp (SEQ ID NO: 3)*

GGCCCTGGGGTCACGGAGGCTGGGAGGCACCGAGGAACGCGCCTGGCATGTGCTGACAGGAATTTT
ATGCTCCAGGAGCAAGACAGAGAGAGACACTCACAGGGAAGAGGGAAGAGGAAAACGAGAAAGGAG
GAGAGTAACGGAAAGAGATAAAAAAGAAAAGCAGGTGGCAGAGACACAGAGAGAGGGACCCAGAGAAA
GCCAGACAGACGCAGGTGGCTGGCAGCGGGCGCTGTGGGGGTCACAGTAGGGGGACCTGTC

Suitably, the variant may be at least 95%, at least 96%, at least 97%, at least 98%, at least 99% identical to SEQ ID NO: 3. Suitably, a variant of SEQ ID NO: 3 comprises one or more of the substitutions A63G, C96T, G185C, and C265G. Suitably, a variant of SEQ ID NO: 3 comprises the substitutions A63G, C96T, G185C, and C265G (cf SEQ ID NOs: 17-20). The promoter may comprise or consist of a variant of SEQ ID NO: 3 shown as SEQ ID NO: 16 or SEQ ID NO: 47.

In some embodiments, the promoter of the present invention comprises or consists of the nucleotide sequence shown as SEQ ID NO: 16, or a variant which is at least 90% identical to SEQ ID NO: 16.

In some embodiments, the promoter of the present invention comprises the nucleotide sequence shown as SEQ ID NO: 16, or a variant which is at least 90% identical to SEQ ID NO: 16 and wherein the promoter has a length of 1.1 kb or less.

In some embodiments, the promoter of the present invention consists of the nucleotide sequence shown as SEQ ID NO: 16, or a variant which is at least 90% identical to SEQ ID NO: 16.

Suitably, the variant may at least 95%, at least 96%, at least 97%, at least 98%, at least 99% identical to SEQ ID NO: 16.

*Exemplary minimal nephrin promoter variant - 265 bp (SEQ ID NO: 16)*

GGCCCTGGGGTCACGGAGGCTGGGAGGCACCGAGGAACGCGCCTGGCATGTGCTGACAGGGGATTTT
ATGCTCCAGGAGCAAGACAGAGAGAGATACTCACAGGGAAGAGGGAAGAGGAAAACGAGAAAGGGAG
GAGAGTAACGGAAAGAGATAAAAAAGAAAAGCAGGTGGCAGAGACACAGAGAGAGGGACCCAGAGAAA
GCCAGACAGACGCAGGTGGCTGGCAGCGGGCGCTGTGGGGGTCACAGTAGGGGGACCTGTC

The promoter of the present invention comprises or consists of the nucleotide sequence shown as SEQ ID NO: 47, or a variant which is at least 90% identical to SEQ ID NO: 47.

In some embodiments, the promoter of the present invention comprises the nucleotide sequence shown as SEQ ID NO: 47, or a variant which is at least 90% identical to SEQ ID NO: 47 and wherein the promoter has a length of 1.1 kb or less.

In some embodiments, the promoter of the present invention consists of the nucleotide sequence shown as SEQ ID NO: 47, or a variant which is at least 90% identical to SEQ ID NO: 47.

Suitably, the variant may be at least 95%, at least 96%, at least 97%, at least 98%, at least 99% identical to SEQ ID NO: 47.

*Exemplary minimal nephrin promoter variant - 265 bp (SEQ ID NO: 47)*

GGCCCTGGGGTCACGGAGGCTGGGAGGCACCGAGGAACGCGCCTGGCATGTGCTGACAGGGGATTTT
ATGCTCCAGGAGCAAGACAGAGAGAGATACTCACAGGGAAGAGGGAAGAGGAAAACGAGAAAGGGAG
GAGAGTAACGGAAAGAGATAAAAAAGAAAAGCAGGTGGCAGAGACACACAGAGAGGGACCCAGAGAAA
GCCAGACAGACGCAGGTGGCTGGCAGCGGGCGCTGTGGGGGTCACAGTAGGGGGACCTGTG

### Polynucleotides

The present invention provides a polynucleotide comprising the promoter of the present invention.

Polynucleotides of the invention may comprise DNA or RNA, preferably DNA. They may be single-stranded or double-stranded. Polynucleotides such as DNA polynucleotides may be produced recombinantly, synthetically or by any means available to those of skill in the art. They may also be cloned by standard techniques. The polynucleotide may be an isolated polynucleotide.

Longer polynucleotides will generally be produced using recombinant means, for example using polymerase chain reaction (PCR) cloning techniques. This will involve making a pair of primers (e.g. of about 15 to 30 nucleotides) flanking the target sequence which it is desired to clone, bringing the primers into contact with mRNA or cDNA obtained from an animal or human cell, performing a polymerase chain reaction under conditions which bring about amplification of the desired region, isolating the amplified fragment (e.g. by purifying the reaction mixture with an agarose gel) and recovering the amplified DNA. The primers may be designed to contain suitable restriction enzyme recognition sites so that the amplified DNA can be cloned into a suitable vector.

The polynucleotides may be modified by any method available in the art. Such modifications may be carried out in order to enhance the in vivo activity or lifespan of the polynucleotides of the invention.

### Protein-coding sequence

The promoter may be operably linked to one or more protein-coding sequences.

The protein-coding sequence may encode any polypeptide of interest. For example, the protein-coding sequence can encode any polypeptide associated with a glomerular disease. The protein-coding sequence may encode a polypeptide involved in with a GBM-associated genetic glomerular disease, such as Alport Syndrome. The protein-coding sequence may encode a polypeptide involved in podocyte-associated genetic glomerular disease.

Suitably, the protein-coding sequence may encode a COL4A3, COL4A4, COL4A5, NPHS2, CFH, CFL, CFHL1, C1INH, C4BP, MASP2, C3, C5aR1, C5, C5a, CD55, CD35, CD46, CD59, vitronectin, clusterin, ADCK4, ALG1, ARHGAP24, ARGHDIA, CD151, CD2AP, COQ2, COQ6, DGKE, E2F3, EMP2, KANK2, LAGE3, LMNA, LMX1B, MAF B, NUP85, NUP93, NXF5, OSGEP, PAX2, PDSS2, PMM2, PODXL, SCARB2, SGPL1, Smad7, TP53RK, TPRKB, VDR, WDR73, WT1, ZMPSTE24, APOL1, NPHS1, TRPC6, NUP107, NUP133, NUP160, ACTN4, INF2, ANKFY1, ANLN, CRB2, ITGA3, KANK1, KANK4, MAGI2, MYO1E, OCRL, PTPRO, SMARCAL1, SYNPO, TBC1D8B, XPO5, TNS2, NLRP3, or VEGFC polypeptide.

Suitably, the protein-coding sequence encodes a polypeptide which has a length of 1450 amino acids or more, 1500 amino acids or more, 1550 amino acids or more, 1600 amino acids or more, or 1650 amino acids or more.

For a protein-coding polynucleotide, it will be understood by a skilled person that numerous different polynucleotides can encode the same polypeptide as a result of the degeneracy of the genetic code. In addition, it is to be understood that skilled persons may, using routine techniques, make nucleotide substitutions that do not affect the polypeptide sequence encoded by the polynucleotides of the invention to reflect the codon usage of any particular host organism in which the polypeptides are to be expressed

The protein-coding sequence may be codon-optimised. Different cells differ in their usage of particular codons. This codon bias corresponds to a bias in the relative abundance of particular tRNAs in the cell type. By altering the codons in the sequence so that they are tailored to match with the relative abundance of corresponding tRNAs, it is possible to increase expression. By the same token, it is possible to decrease expression by deliberately choosing codons for which the corresponding tRNAs are known to be rare in the particular cell type.

Thus, an additional degree of translational control is available. Codon usage tables are known in the art for mammalian cells (e.g. humans), as well as for a variety of other organisms.

The protein coding nucleotide sequences disclosed herein may comprise or lack stop codons at their 3' end. Thus, the present disclosure encompasses the SEQ ID NOs disclosed herein with the stop codons present or absent.

### COL4A3, COL4A4 and COL4A5 polypeptides

The protein-coding sequence may encode a COL4A3, COL4A4 or COL4A5 polypeptide, or a fragment or derivative thereof.

COL4A3, COL4A4 and COL4A5 proteins are approximately 170-185 kDa homologous polypeptides containing collagenous Gly-X-Y repeat sequences frequently interrupted by non-collagenous sequences and forming a triple helix repeat. Each polypeptide also contains a large globular non-collagenous domain at the carboxyl-terminal end.

Alport syndrome (AS) is caused by pathogenic variants in the COL4A3, COL4A4 and COL4A5 genes, which result in abnormalities of the collagen IV α345 network of basement membranes.

The COL4A3, COL4A4 or COL4A5 polypeptide or a fragment or derivative thereof may be capable of forming a collagen IV α345 network.

Approximately 200-300 amino acids may be removed from each of the COL4A3, COL4A4 and COL4A5 polypeptides to produce a truncated transgene suitable for a mini-gene approach. The amino acids may be removed from the triple helix repeat. Preferably the amino acids are not removed from the non-collagenous region.

In some embodiments the COL4A3, COL4A4 and COL4A5 polypeptides are full-length polypeptides.

Preferably, the COL4A3, COL4A4 or COL4A5 polypeptide is human. An example human COL4A3 is the COL4A3 having the UniProtKB accession number Q01955. An example human COL4A4 is the COL4A3 having the UniProtKB accession number P53420. An example human COL4A5 is the COL4A5 having the UniProtKB accession number P29400.

Suitably, the COL4A3 peptide may comprise or consist of the polypeptide sequence shown as SEQ ID NO: 21, or a variant which is at least 70% identical to SEQ ID NO: 21. Suitably, the variant may be at least 75%, at least 80%, at least 85%, at least 90%, at least 95%, at least 96%, at least 97%, at least 98%, at least 99% identical to SEQ ID NO: 21.

Suitably, the COL4A4 peptide may comprise or consist of the polypeptide sequence shown as SEQ ID NO: 22, or a variant which is at least 70% identical to SEQ ID NO: 22. Suitably, the variant may be at least 75%, at least 80%, at least 85%, at least 90%, at least 95%, at least 96%, at least 97%, at least 98%, at least 99% identical to SEQ ID NO: 22.

Suitably, the COL4A5 peptide may comprise or consist of the polypeptide sequence shown as SEQ ID NO: 23, or a variant which is at least 70% identical to SEQ ID NO: 23. Suitably, the variant may be at least 75%, at least 80%, at least 85%, at least 90%, at least 95%, at least 96%, at least 97%, at least 98%, at least 99% identical to SEQ ID NO: 23.
*Exemplary COL4A3 amino acid sequence - Uniprot reference Q01955 (SEQ ID NO: 21)*
*Exemplary COL4A4 amino acid sequence - Uniprot reference P53420 (SEQ ID NO: 22)*
*Exemplary COL4A5 amino acid sequence - Uniprot reference P29400 (SEQ ID NO: 23)*

The protein-coding sequence may comprise or consist of a COL4A3, COL4A4 or COL4A5 transgene.

An example transgene encoding COL4A3 is provided in NM_000091.5. An example transgene encoding COL4A4 is provided in NM_000092.5. An example transgene encoding COL4A5 is provided in NM_000495.5.

Suitably, the COL4A3 transgene may comprise or consist of the polynucleotide sequence shown as SEQ ID NO: 24, or a variant which is at least 70% identical to SEQ ID NO: 24. Suitably, the variant may be at least 75%, at least 80%, at least 85%, at least 90%, at least 95%, at least 96%, at least 97%, at least 98%, at least 99% identical to SEQ ID NO: 24.

Suitably, the COL4A4 transgene may comprise or consist of the polynucleotide sequence shown as SEQ ID NO: 25, or a variant which is at least 70% identical to SEQ ID NO: 25. Suitably, the variant may be at least 75%, at least 80%, at least 85%, at least 90%, at least 95%, at least 96%, at least 97%, at least 98%, at least 99% identical to SEQ ID NO: 25.

Suitably, the COL4A5 transgene may comprise or consist of the polynucleotide sequence shown as SEQ ID NO: 26, or a variant which is at least 70% identical to SEQ ID NO: 26. Suitably, the variant may be at least 75%, at least 80%, at least 85%, at least 90%, at least 95%, at least 96%, at least 97%, at least 98%, at least 99% identical to SEQ ID NO: 26.
*Exemplary COL4A3 transgene sequence (SEQ ID NO: 24)* 
atgagcgcccggaccgcccccaggccgcaggtgctcctgctgccgctcctgctggtgctcctggcggc
ggcgcccgcagccagcaaggttgtgtctgtaaagacaaaggccagtgcttctgtgacgggccaaag
ggagaagggggagaaggctttcctggacccccggttctcctggccagaaaggattcacaggtcct
gaaggcttgcctggaccgcagggacccaagggctttccaggacttccaggactcacgggttccaaagg
tgtaaggggaataagtggattgccaggattttctggttctcctggacttccaggcaccccaggcaata
ccgggccttacggacttgtcggtgtaccaggatgcagtggttctaagggtgagcaggggtttccagga
ctcccagggacactgggctacccaggatcccgggtgctgctggtttgaaaggacaaaagggtgctcc
tgctaaagaagaagatatagaacttgatgcaaaaggcgaccccgggttgccaggggctccaggacccc
agggtttgccaggccctccaggttttcctggcctgttggcccacctggtcctccgggattctttggc
tttccaggagccatggacctagaggacctaagggtcacatgggtgaaagagtgataggacataaagg
agagcggggtgtgaaagggttaacaggacccccgggaccaccaggaacagttattgtgaccctaactg
gcccagataacagaacggacctcaagggggaaaagggagacaagggagcaatgggcgagcctggacct
cctggaccctcaggactgcctggagaatcatatggatctgaaaagggtgctcctggagaccctggcct
gcagggaaaacccggaaaagatggtgttcctggcttccctggaagtgagggagtcaagggcaacaggg
gtttccctgggttaatgggtgaagatggcattaagggacagaaaggggacattggccctccaggattt
cgtggtccaacagaatattatgacacataccaggaaaaggagatgaaggcactccaggcccaccagg
gcccagaggagctcgtggcccacaaggtcccagtggtcccccggagttcctggaagtcctggatcat
caaggcctggcctcagaggagcccctggatggccaggcctgaaaggaagtaaaggggaacgaggccgc
ccaggaaaggatgccatgggactcctgggtccccaggttgtgctggttcaccaggtcttccaggatc
accgggacctccaggaccgccaggtgacatcgtttttcgcaagggtccacctggagatcacggactgc
caggctatctagggtctccaggaatcccaggagttgatgggcccaaaggagaaccaggcctcctgtgt
acacagtgcccttatatcccaggcctcccggtctcccaggattgccaggttacatggtgtaaaagg
aatcccaggaagacaaggcgcagctggcttgaaaggaagcccaggtccccaggaaatacaggtcttc
caggatttccaggtttcccaggtgcccagggtgacccaggacttaaaggagaaaaaggtgaaacactt
cagcctgaggggcaagtgggtgtcccaggtgacccggggctcagaggccaacctgggagaaaggctt
ggatggaattcctggaactccgggagtgaaaggattaccaggacctaaaggcgaactggctctgagtg
gtgagaaaggggaccaaggtcctccaggggatcctggctcccctgggtccccaggacctgcaggacca
gctggaccacctggctacggaccccaaggagaacctggtctccagggcacgcaaggagttcctggagc
cccccggaccacccggagaagccggccctaggggagagctcagtgtttcaacaccagttccaggcccac
caggacctccagggccccctggccatcctggcccccaaggtccacctggtatccctggatccctgggg
aaatgtggagatcctggtcttccaggcctgatggtgaaccaggaattccaggaattggatttcctgg
gcctcctggacctaagggagaccaaggttttccaggtacaaaaggatcactgggttgtcctggaaaaa
tgggagagcctgggttacctggaaagccaggcctcccaggagccaaggagaaccagcagtagccatg
cctggaggaccaggaacaccaggttttccaggagaaagaggcaattctggggaacatggagaaattgg
actccctggacttccaggtctccctggaactccaggaaatgaagggcttgatggaccacgaggagatc
 
cagggcagcctggaccacctggagaacaaggacccccaggaaggtgcatagagggtcccaggggagcc
caaggacttccaggcttaaatggattgaaaggcaacaaggcagaagaggtaaaacgggccaaaggg
agacccaggaattccaggcttggatagatcaggatttcctggagaaactggatcaccaggaattccag
gtcatcaaggtgaaatggaccactgggtcaaagaggatatccaggaaatccgggaattttagggcca
ccaggtgaagatggagtgattgggatgatgggctttcctggagccattggccctccagggccccctgg
gaacccaggcacaccagggcagaggggagccctggaattccaggagtaaaggccagagaggaaccc
caggagccaaggggaacaaggagataaaggaaatcccggccttcagagatatccacgtaataggg
gacaaaggagaaccaggtctcaaaggattcgcaggaaatccaggtgagaaaggaaacagaggcgttcc
agggatgccaggtttaaaggcctcaaaggactacccggaccagcaggaccaccaggccccagaggag
atttgggcagcactgggaatcctggagaaccaggactgcgtggtataccaggaagcatggggaacatg
ggcatgccaggttctaaaggaaaaagggaactttgggattcccaggtcgagcaggaagaccaggcct
cccaggtattcatggtctccagggagataaggagagccaggttattcagaaggtacaaggccaggac
caccggaccaacggggatccaggactgccgggtgatatgggaaagaaaggagaaatgggcaacct
ggcccacctggacatttgggcctgctggacctgaggagcccctggaagtcctggaagtcctggcct
cccaggaaagccaggtcctcatggtgatttgggttttaaaggaatcaaaggcctcctgggccctccag
gaatcagaggccctccaggtcttccaggatttccaggatctcctggaccaatgggtataagaggtgac
caaggacgtgatggaattcctggtccagccggagaaaaggagaaacgggtttattgaggcccctcc
aggcccaagagggaaccctggtgctcaaggagccaaaggagacaggggagccccaggttttcctggcc
tcccgggcagaaaagggccatggagatgctggacctcgaggacccacaggcatagaaggattccca
gggccaccaggtctgcccggtgcaattatccctggccagacaggaaatcgtggtccaccaggctcaag
aggaagcccaggtgcgcctggtcccctggacctccagggagtcatgtaataggcataaaggagaca
aagggtctatgggccaccctggcccaaaaggtccacctggaactgcaggagacatgggaccaccaggt
cgtctgggagcaccaggtactccaggtcttccaggacccagaggtgatcctggattccaggggtttcc
aggcgtgaaaggagaaaaggtaatcctggatttctaggatccattggacctccaggaccaattgggc
caaaaggaccacctggtgtacgtggagaccctggcacacttaagattatctcccttccaggaagccca
gggccacctggcacacctggagaaccaggatgcaggagaacctgggccaccaggccacctggaaa
cctaggaccctgtgggccaagaggtaagccaggcaaggatggaaaaccaggaactcctggaccagctg
gagaaaaaggcaacaaaggttctaaaggagagccaggaccagctggatcagatggattgccaggtttg
aaaggaaaacgtggagacagtggatcacctgcaacctggacaacgagaggctttgtcttcacccgaca
cagtcaaaccacagcaattccttcatgtccagagggacagtgccactctacagtgggtttcttttc
tttttgtacaaggaaatcaacgagcccacggacaagaccttggaactcttggcagctgcctgcagcga
tttaccacaatgccattcttattctgcaatgtcaatgatgtatgtaattttgcatctcgaaatgatta
ttcatactggctgtcaacaccagctctgatgccaatgaacatggctcccattactggcagagcccttg
agccttatataagcagatgcactgtttgtgaaggtcctgcgatcgccatagccgttcacagccaaacc
actgacattcctccatgtcctcacggctggatttctctctggaaaggattttcattcatcatgttcac
aagtgcaggttctgagggcaccggcaagcactggcctcccctggctcctgcctggaagaattccgag
ccagcccatttctagaatgtcatggaagaggaacgtgcaactactattcaaattcctacagtttctgg
ctggcttcattaaacccagaaagaatgttcagaaagcctattccatcaactgtgaaagctggggaatt
agaaaaaataataagtcgctgtcaggtgtgcatgaagaaaagacactga
*Exemplary COL4A4 transgene sequence (SEQ ID NO: 25)* 
atgtggtctctgcacatagtactaatgaggtgctccttcagattgaccaagtccttggccacaggtcc
ctggtcacttatactcattctcttttctgtacaatatgtatatggagtggaaagaatacattggtc
cttgtggaggaagagattgctctgtttgccactgtgttcctgaaaagggtctcggggtccaccagga
ccaccagggccacaggtccaattggaccccctgggagccccaggacccattgggctttcaggagagaa
aggaatgagaggggaccgcggccctcctggagcagcaggggacaaaggagataaggtccaactggtg
ttcctggatttccaggtttagatggcatacctgggcacccagggcctcctggacccagaggcaaacct
ggtatgagtggccacaatggctcaagaggtgacccagggtttccaggaggaagaggagctcttggccc
aggaggcccccctaggccatcctggggaaaaggagaaaaaggaaattcagtgttcattttaggtgccg
ttaaaggtattcagggagacagaggggacccaggactgcctggcttaccaggatcttggggtgcagga
ggaccggcaggtcccacaggatatcctggagagccaggttagtgggacctccggccaaccagggcg
tccaggtttgaagggaaatcccggtgtgggagtaaagggcaaatggagacccggtgaggttggtc
agcaaggttctcctggacccaccctgttggtagagccacctgacttttgtctctataaaggagaaaag
ggtataaaaggaattcctggaatggttggactgccaggaccaccaggacgcaagggagaatctggtat
 
tggggcaaaaggagaaaaggtattcctggatttccagggcctcgggggatcctggttcctatggat
ctccaggttttccaggattaaagggagaactaggactggttggagatcctgggctatttggattaatt
ggcccaaaggggggatcctggaaatcgagggcacccaggaccaccaggtgttttggtgactccacctct
tccactcaaaggcccaccaggggacccagggttccctggccgctatggagaaacaggggatgttggac
cacctggtccccccaggtctcttgggcagaccaggggaagcctgtgcaggcatgataggacccccctggg
ccacaaggatttcctggtcttcctgggcttccaggagaagctggtattcctgggagacctgattctgc
tccaggaaaaccagggaagccaggatcacctggcttgcctggagcaccaggcctgcaggcctcccag
gatcaagtgtgatatactgtagtgttgggaaccccggaccacaaggaataaaaggcaaagttggtccc
ccaggaggaagaggcccaaaaggagaaaaggaaatgaaggactctgtgcctgtgagcctggacccat
gggcccccctggccctccaggacttcctgggaggcaggggagtaagggagacttggggctccctggct
ggcttggaacaaaaggtgacccaggacctcctggtgctgaaggacctccaggctaccaggaaagcat
ggtgcctctggaccacctggcaacaaagggcgaaggtgacatggttgtatcaagagttaaaggca
caaaggagaaagaggtcctgatggcccccaggatttccagggcagccaggatcacatggtcgggatg
gacatgctggagaaaaaggggatccaggacctccaggggatcatgaagatgcgaccccaggtggtaaa
ggatttcctggacctctgggcccccccaggcaaagcaggacctgtggggcccccaggactgggatttcc
tggtccaccaggagagcgaggccacccaggagttccaggccacccaggtgtgagggccctgatggct
tgaagggtcagaaaggtgacacaatttcttgcaacgtaacctaccctgggaggcatggccctccaggt
tttgatggacctccaggtccgaagggatttccaggtccccaaggtgcccctgggctgagtggttcaga
tgggcataaaggcagacctggcacaccaggaacagcggaaataccaggtccacctggttttcgtggtg
acatggagatccgggttttggaggtgaaaaggggtcctcccctgttggcccccaggccctcccggc
tcaccaggagtgaatggtcagaaaggaatcccgggagaccctgcatttggtcacctgggacccccggg
aaagaggggtctttcaggagtgccaggataaaaggacccagaggtgatccgggatgtccagggctg
aagggccagctggcattcctggattcctaggtctcaaaggtcccaaaggcagagagggacatgctggg
tttccaggtgtcccaggtccacctggccattcctgtgaaagaggtgctccagggataccaggcaacc
gggactccctgggtatccaggtagcccaggtgctccaggtgggaaaggacagccgggagatgtggggc
ctcccggccagctggaatgaaaggcctccccggactcccaggacggcctggggcacatggtcccccca
ggcctcccaggaatcccaggtccctttggagatgatggctacctggtcctccaggtccaaaggacc
ccgggggctgcctggtttcccaggttttcccggagaaagaggaaagcctggtgcagaggatgtcctg
gcgcaaaggagaacctggagagaaggcatgtctggccttcctggagaccggggactgagagggcc
aaaggagccataggacctcccggagatgaaggagaaatggctatcatttcacaaaaggaacacctgg
ggaacctggacctcctggagatgatggattcccaggagaaagaggtgataaaggaactcccggatgc
aagggagaagaggagagccgggaagatacggaccacctggatttcacagaggggaacctggtgagaaa
ggtcagccaggcctcctggacccccaggccctccaggctcaactggtctaagagggttcattggttt
tccaggacttccaggtgaccaggtgagccaggttctccaggtcccccctggattttcaggaattgatg
gagcaagaggacctaaaggaaacaaaggtgaccctgccagtcactttggtccacctggtccaaagggt
gagccaggtagccctggatgtccagggcattttggagcatccggagagcagggcttgcctggtattca
agggcccagaggatcacctggaaggccagggccacctggctcctctggaccaccagggtgcccaggtg
atcacgggatgcctggctgaggggacagccaggagaaatggagaccctgggccaagaggcctccag
gggatccaggataccaggtcctccgggaataaaaggtccctccggatcacctggcctgaacggctt
gcatggattgaaaggtcagaaaggaactaaaggtgcttcaggtttgcatgatgtggggccacctggtc
cagtgggaatacctggctaaaagggagagaggagaccctgggagcccaggaatctctcctccaggt
cctcgtggaaagaaaggtcccccaggacccccagggagttcaggaccacctggtcctgcaggtgccac
aggaagagctcctaaggacattcctgacccggtccacctggagatcagggacctcctggtcctgatg
gcccaagaggagcacctgggcctccaggcctccctgggagtgttgaccttctgagagggagccaggt
gactgtggtctaccaggccaccaggtcccccctggcccaccaggccctccaggatacaaaggctttcc
aggatgtgatggaaaagatggccagaaaggaccagtgggattcccgggaccgcaggaccacatggat
ttcctgggccacctggagagaagggtttacctggacctccagggagaaaaggcccactggtcttccg
ggtcccagaggtgaaccggggccacctgcagatgtggatgactgtccccgaatcccaggccttcctgg
ggcgccaggcatgagaggaccagaaggagccatggggctccctggaatgagaggcccctcaggaccag
ggtgcaaaggagagcctgggctggatggcaggagggggtgtggatggcgtccctgggtctcctgggcct
cccggacgtaaaggtgacacaggagaagacggctaccctggaggaccaggcctcctggtcccattgg
ggatcctgggcccaaagggtttggccctggatacctcggtggcttcctcctggttctccacagtcaga
cggaccaggagcccacctgccccctggcatgcccaggctctggactgggtatagtctgttatacctg
gaagggcaagagaaagctcacaatcaagaccttggtctggcagggtcttgccttcccgtatttagcac
gctgccctttgcctactgcaacatccaccaggtgtgccactatgcccagagaaacgacagatcctact
ggctggccagcgctgcgcccctccccatgatgccactctctgaagaggcgatccgcccctatgtcagc
 
cgctgtgcggtatgcgaggccccggcccaggcggtggcggtgcacagccaggaccagtccatcccccc
atgtccgcagacctggaggagcctctggatcgggtattcattcctgatgcacacaggagctggggacc
aaggaggagggcaggcccttatgtcacctggcagctgcctggaagatttcagagcagcaccattcctt
gaatgccagggccggcagggaacttgccactttttcgcaaataagtatagcttctggctcacaacggt
gaaagcagacttgcagttttcctctgctccagcaccagacaccttaaaagaaagccaggcccaacgcc
agaaaatcagccggtgccaggtctgcgtgaagtatagctag
*Exemplary COL4A5 transgene sequence (SEQ ID NO: 26)*

atgaaactgcgtggagtcagcctggctgccggcttgttcttactggccctgagtctttgggggcagcc
tgcagaggctgcggcttgctatgggtgttctccaggatcaaagtgtgactgcagtggcataaaagggg
aaaagggagagagagggtttccaggtttggaaggacacccaggattgcctggatttccaggtccagaa
gggcctccgggcctcggggacaaaggtgatgatggaattccaggccaccaggaccaaaggaat
cagaggtcctcctggacttcctggatttccaggacaccaggtcttcctggaatgccaggccacgatg
ggccccaggacctcaaggtattcccggatgcaatggaaccaaggagaacgtggatttccaggcagt
cccggttttcctggtttacaggtcctccaggacccctgggatcccaggtatgaaggtgaaccagg
tagtataattatgtcatcactgccaggaccaaagggtaatccaggatatccaggtcctcctggaatac
aaggcctacctggtcccactggtataccagggccaattggtcccccaggaccaccaggtttgatgggc
cctcctggtccaccaggacttccaggacctaagggaatatggcttaaatttccagggacccaaagg
tgaaaaaggtgagcaaggtcttcagggcccacctgggccacctgggcagatcagtgaacagaaaagac
caattgatgtagagtttcagaaaggagatcagggacttcctggtgaccgaggcctcctggacctcca
gggatacgtggtcctccaggtccccaggtggtgagaaaggtgagaagggtgagcaaggagagccagg
caaaagaggtaaaccaggcaaagatggagaaaatggccaaccaggaattcctggtttgcctggtgatc
ctggttaccctggtgaacccggaagggatggtgaaaaggccaaaaaggtgacactggcccacctgga
cctcctggacttgtaattcctagacctggactggtataactataggagaaaaaggaaacattgggtt
gcctgggttgcctggagaaaaaggagagcgaggatttcctggaatacaggtccacctggccttcctg
gacctccagggggctgcagttatgggtcctcctggccctcctggatttcctggagaaaggggtcagaaa
ggtgatgaaggaccacctggaatttccattcctggacctcctggacttgacggacagcctggggctcc
tgggcttccaggcctcctggccctgctggccctcacattcctcctagtgatgagatatgtgaaccag
gccctccaggcccccaggatctccaggtgataaaggactccaaggagaacaaggagtgaaaggtgac
aaaggtgacacttgcttcaactgcattggaactggtatttcagggcctccaggtcaacctggtttgcc
aggtctcccaggtcctccaggatctcttggtttccctggacagaaaggggaaaaaggacaagctggtg
caactggtcccaaaggattaccaggcattccaggagctccaggtgctccaggctttcctggatctaaa
ggtgaacctggtgatatcctcacttttccaggaatgaagggtgacaaaggagagttgggttcccctgg
agctccaggcttcctggtttacctggcactcctggacaggatggattgccaggcttcctggcccga
aaggagagcctggtggaattacttttaagggtgaaagaggtcccctgggaacccaggtttaccaggc
ctcccagggaatatagggcctatgggtccccctggtttcggccctccaggcccagtaggtgaaaaagg
catacaaggtgtggcaggaaatccaggccagccaggaataccaggtcctaaagggatccaggtcaga
ctataacccagccgggaagcctggcttgcctggtaacccaggcagagatggtgatgtaggtcttcca
ggtgaccctggacttccaggcaaccaggcttgccagggatacctggtagcaaaggagaaccaggtat
ccctggaattgggcttcctggaccacctggtcccaaaggctttcctggaattccaggacctccaggag
cacctgggacacctggaagaattggtctagaaggccctcctggcccacccggctttccaggaccaaag
ggtgaaccaggatttgcattacctggcccacctggcccaccaggacttccaggtttcaaaggagcact
tggtccaaaaggtgatcgtggtttcccaggacctccgggtcctccaggacgcactggcttagatggc
tccctggaccaaaaggtgatgttggaccaaatggacaacctggaccaatggacctcctggctgcca
ggaataggtgttcaggaccaccaggaccaccaggattcctgggccaataggtcaacctggtttaca
tggaataccaggagagaaggggggatccaggacctcctggacttgatgttccaggacccccaggtgaaa
gaggcagtccagggatccccggagcacctggtcctataggacctccaggatcaccaggcttccagga
aaagcaggtgcctctggatttccaggtaccaaaggtgaaatgggtatgatgggacctccaggcccacc
aggacctttgggaattcctggcaggagtggtgtacctggtcttaaaggtgatgatggcttgcagggtc
agccaggacttcctggccctacaggagaaaaaggtagtaaaggagagcctggccttccaggccctcct
ggaccaatggatccaaatcttctgggctcaaaaggagagaaggggggaacctggcttaccaggtatacc
tggagtttcaggccaaaaggttatcagggtttgcctggagacccagggcaacctggactgagtggac
aacctggattaccaggaccaccaggtcccaaaggtaaccctggtctccctggacagccaggtcttata
ggacctcctggacttaaaggaaccatcggtgatatgggttttccaggcctcagggtgtggaaggcc

tcctggaccttctggagttcctggacaacctggctccccaggattacctggacagaaaggcgacaaag
gtgatcctggtatttcaagcattggtcttccaggtcttcctggtccaaaggtgagcctggtctgcct
ggatacccagggaaccctggtatcaaaggttctgtgggagatcctggtttgcccggattaccaggaac
cctggagcaaaaggacaaccaggccttcctggattcccaggaaccccaggccctcctggaccaaaag
gtattagtggccctcctgggaaccccggccttccaggagaacctggtcctgtaggtggtggaggtcat
cctgggcaaccagggcctccaggcgaaaaggcaaacccggtcaagatggtattcctggaccagctgg
acagaagggtgaaccaggtcaaccaggctttggaaacccaggacccctggacttccaggactttctg
gccaaaagggtgatggaggattacctgggattccaggaaatcctggccttccaggtccaaagggcgaa
ccaggctttcacggtttccctggtgtgcaggtcccccaggccctcctggttctccgggtccagctct
ggaaggacctaaaggcaaccctgggccccaaggtcctcctgggagaccaggtctaccaggtccagaag
gtcctccaggtctccctggaaatggaggtattaaaggagagaagggaaatccaggccaacctgggcta
cctggcttgcctggtttgaaaggagatcaaggaccaccaggactccagggtaatcctggccggccggg
tctcaatggaatgaaaggagatcctggtctccctggtgttccaggattcccaggcatgaaaggaccca
gtggagtacctggatcagctggccctgaggggaaccgggacttattggtcctccaggtcctcctgga
ttacctggtccttcaggacagagtatcataattaaaggagatgctggtcctccaggaatccctggcca
gcctgggctaaagggtctaccaggaccccaaggacctcaaggcttaccaggtccaactggccctccag
gagatcctggacgcaatggactccctggctttgatggtgcaggagggcgcaaaggagacccaggtctg
ccaggacagccaggtacccgtggtttggatggtccccctggtccagatggattgcaaggtcccccagg
tccccctggaacctcctctgttgcacatggatttcttattacacgccacagccagacaacggatgcac
cacaatgcccacagggaacacttcaggtctatgaaggcttttctctcctgtatgtacaaggaaataaa
agagcccacggtcaagacttggggacggctggcagctgccttcgtcgctttagtaccatgcctttcat
gttctgcaacatcaataatgtttgcaactttgcttcaagaaatgactattcttactggctctctaccc
cagagcccatgccaatgagcatgcaacccctaaagggccagagcatccagccattcattagtcgatgt
gcagtatgtgaagctccagctgtggtgatcgcagttcacagtcagacgatccagattccccattgtcc
tcagggatgggattctctgtggattggttattccttcatgatgcatacaagtgcaggggcagaaggct
caggtcaagccctagcctcccctggttcctgcttggaagagtttcgttcagctcccttcatcgaatgt
catgggagggtacctgtaactactatgccaactcctacagcttttggctggcaactgtagatgtgtc
agacatgttcagtaaacctcagtcagaaacgctgaaagcaggagacttgaggacacgaattagccgat
gtcaagtgtgcatgaagaggacataa

The COL4A3, COL4A4 or COL4A5 transgene may comprise an intron or intronic sequences, which can be used to improve gene expression. The COL4A3, COL4A4 or COL4A5 transgene may comprise a protein tag, such as a hemagglutinin (HA) tag. HA can be used as an epitope tag and has been shown not to interfere with bioactivity or biodistribution of proteins to which it has been added. The protein tag can facilitate detection, isolation, and purification of the transgene. Other suitable protein tags may include Myc tags, polyhistidine tags and flag tags.

In some embodiments, a promoter comprising or consisting of a nucleotide sequence having at least 70%, at least 75%, at least 80%, at least 85%, at least 90%, at least 95%, at least 96%, at least 97%, at least 98%, at least 99%, or 100% identity to SEQ ID NO: 47 is operably linked to the protein-coding sequence encoding a COL4A3, COL4A4 or COL4A5 polypeptide, or a fragment or derivative thereof.

In some embodiments, a promoter consisting of the nucleotide sequence of SEQ ID NO: 47 is operably linked to the protein-coding sequence encoding a COL4A3, COL4A4 or COL4A5 polypeptide, or a fragment or derivative thereof.

### Nephrotic syndrome (NS)-associated transgene

The protein-coding sequence may comprise or consist of a NS-associated transgene.

Nephrotic syndrome (NS) is a chronic kidney disease characterized by significant proteinuria, hypoalbuminemia, oedema and hyperlipidemia. The NS-associated transgene may be a gene associated with a monogenic form of NS and expressed in podocytes.

Suitable NS-associated transgenes include *NPHS2, ADCK4, ALG1, ARHGAP24, ARGHDIA,* CD151, CD2AP, COQ2, COQ6, *DGKE, E2F3, EMP2, KANK2, LAGE3, LMNA, LMX1B, MAFB, NUP85, NUP93, NXF5, OSGEP, PAX2, PDSS2, PMM2, PODXL, SCARB2, SGPL1, Smad7, TP53RK, TPRKB, VDR, WDR73, WT1, ZMPSTE24, APOL1, NPHS1, TRPC6, NUP107, NUP133, NUP160, ACTN4, INF2, ANKFY1, ANLN, CRB2, ITGA3, KANK1, KANK4, MAGI2, MYO1E, OCRL, PTPRO, SMARCAL1, SYNPO, TBC1D8B, XPO5, TNS2* and *NLRP3.*

In some embodiments, a promoter comprising or consisting of a nucleotide sequence having at least 70%, at least 75%, at least 80%, at least 85%, at least 90%, at least 95%, at least 96%, at least 97%, at least 98%, at least 99%, or 100% identity to SEQ ID NO: 47 is operably linked to the NS-associated transgene.

In some embodiments, a promoter consisting of the nucleotide sequence of SEQ ID NO: 47 is operably linked to the NS-associated transgene.

### NPHS2

The protein-coding sequence may encode NPHS2, or a fragment and/or variant thereof.

"NPHS2" is the abbreviated name of the polypeptide encoded by the *NPHS2* gene and is also known as podocin. NPHS2 is a 42kDa hairpin like membrane-associated podocyte-specific protein that is a key component of the protein complex at the slit diaphragm; the cell-cell junction between adjacent podocyte foot processes. It localises to lipid rafts and interacts with other important slit diaphragm proteins like nephrin, CD2AP and TRPC6. It is essential in the maintenance of the slit diaphragm, and consequently the integrity of the glomerular filtration barrier.

A fragment and/or variant of NPHS2 may retain NPHS2 activity. For example, a fragment and/or variant of podocin may regulate glomerular permeability. Suitably, a fragment and/or variant of NPHS2 may have the same or similar activity to NPHS2, e.g. may have at least 40%, at least 50%, at least 60%, at least 70%, at least 80%, at least 90%, at least 95%, or at least 100% of the activity of NPHS2.

A person skilled in the art would be able to generate fragments and/or variants using conservative substitutions, based on the known structural and functional features of NPHS2 (see e.g. Tabassum, A., et al., 2014. Interdisciplinary Sciences: Computational Life Sciences, 6(1), pp.32-39), and/or based on known variants (see e.g. NCBI Gene ID: 7827 and NCBI HomoloGene: 22826). Suitably, a fragment and/or variant of NPHS2 comprises a transmembrane domain, with two cytoplasmic domains at the N- and C-terminus.

The *NPHS2* gene is conserved in chimpanzee, Rhesus monkey, dog, cow, mouse, and rat. The NPHS2 may be a human NPHS2. Suitably, the NPHS2 may comprise or consist of a polypeptide sequence of UniProtKB accession Q9NP85, or a fragment and/or variant thereof.

In some embodiments, the NPHS2 comprises or consists of an amino acid sequence which is at least 70% identical to SEQ ID NO: 55 or a fragment thereof. Suitably, the NPHS2 comprises or consists of an amino acid sequence which is at least 75%, at least 80%, at least 85%, at least 90%, at least 95%, at least 96%, at least 97%, at least 98% or at least 99% identical to SEQ ID NO: 55 or a fragment thereof.

In some embodiments, the NPHS2 comprises or consists of SEQ ID NO: 55 or a fragment thereof. Exemplary NPHS2 amino acid sequence (SEQ ID NO: 55)

In some embodiments, the NPHS2 transgene comprises or consists of a nucleotide sequence which is at least 70% identical to SEQ ID NO: 56 or a fragment thereof. Suitably, the NPHS2 transgene comprises or consists of a nucleotide sequence which is at least 75%, at least 80%, at least 85%, at least 90%, at least 95%, at least 96%, at least 97%, at least 98% or at least 99% identical to SEQ ID NO: 56 or a fragment thereof.

In some embodiments, the NPHS2 transgene, comprises or consists of the nucleotide sequence SEQ ID NO: 56 or a fragment thereof. 
ATGGAGAGGAGGGCGCGGAGCTCCTCCAGGGAGTCCCGCGGGCGAGGCGGCAGGACTCCGCACAAGGAG
AACAAGAGGGCAAAGGCCGAGAGGAGCGGCGGGGGCCGCGGGCGCCAGGAGGCTGGGCCCGAGCCGTCG
GGCTCCGGACGGGCGGGGACCCCGGGGGAGCCCCGAGCGCCCGCCGCCACGGTGGTGGACGTGGATGAG
GTCCGAGGCTCCGGCGAGGAGGGCACCGAGGTGGTGGCGCTGTTGGAGAGCGAGCGGCCCGAGGAAGGT
ACCAAATCCTCCGGCTTAGGGGCCTGTGAGTGGCTTCTTGTCCTCATTTCCCTGCTCTTCATCATCATG
ACCTTCCCTTTTTCCATCTGGTTCTGCGTAAAGGTTGTACAAGAGTATGAAAGAGTAATTATATTCCGA
CTGGGACATCTGCTTCCTGGAAGAGCCAAAGGCCCTGGTCTTTTCTTTTTTTTGCCCTGCCTGGATACC
TACCACAAGGTTGACCTTCGTCTCCAAACTCTGGAGATACCTTTTCATGAGATCGTGACCAAAGACATG
 
TTTATAATGGAGATAGATGCCATTTGCTACTACCGAATGGAAAATGCCTCTCTTCTCCTAAGCAGTCTT
GCTCATGTATCTAAAGCTGTGCAATTCCTTGTGCAAACCACTATGAAGCGTCTCCTAGCACATCGATCC
CTCACTGAAATTCTTCTAGAGAGGAAGAGCATCGCCCAAGATGCAAAGGTTGCCTTGGATTCAGTGACC
TGTATTTGGGGAATCAAAGTGGAGAGAATAGAAATTAAAGATGTGAGGTTGCCAGCTGGGCTTCAGCAC
TCACTGGCTGTGGAGGCTGAAGCGCAAAGACAAGCCAAAGTGCGGATGATTGCTGCAGAAGCGGAAAAG
GCTGCTTCTGAGTCCCTGAGGATGGCAGCTGAGATTCTGTCAGGCACCCCTGCTGCTGTTCAGCTTCGA
TACCTCCACACCCTTCAGTCTCTGTCCACAGAGAAGCCTTCCACTGTGGTTTTACCTTTGCCATTTGAC
CTACTGAATTGCCTGTCTTCTCCCAGCAACAGAACTCAGGGAAGCCTCCCCTTCCCAAGTCCTTCCAAA
CCTGTTGAGCCACTAAATCCTAAAAAGAAAGACTCTCCCATGTTA
 Exemplary NPHS2 transgene sequence (SEQ ID NO: 56)

In some embodiments, the NPHS2 transgene comprises or consists of a nucleotide sequence which is at least 70% identical to SEQ ID NO: 57 or a fragment thereof. Suitably, the NPHS2 transgene comprises or consists of a nucleotide sequence which is at least 75%, at least 80%, at least 85%, at least 90%, at least 95%, at least 96%, at least 97%, at least 98% or at least 99% identical to SEQ ID NO: 57 or a fragment thereof.

In some embodiments, the NPHS2 transgene, comprises or consists of the nucleotide sequence SEQ ID NO: 57 or a fragment thereof. 
ATGGAGAGGAGGGCGCGGAGCTCCTCCAGGGAGTCCCGCGGCGAGGCGGCAGGACTCCGCACAAGGAG
AACAAGAGGGCAAAGGCCGAGAGGAGCGGCGGAGGCCGCGGGCGCCAGGAGGCTGGGCCCGAGCCGTCG
GGCTCCGGACGGGCGGGGACCCCGGGGGAGCCCCGAGCGCCCGCCGCCACGGTGGTGGACGTGGATGAG
GTCCGAGGCTCCGGCGAGGAGGGCACCGAGGTGGTGGCGCTGTTGGAGAGCGAGCGGCCCGAGGAAGGT
ACCAAATCCTCCGGCTTAGGGGCCTGTGAGTGGCTTCTTGTCCTCATTTCCCTGCTCTTCATCATCATG
ACCTTCCCTTTTTCCATCTGGTTCTGCGTAAAGGTTGTACAAGAGTATGAAAGAGTAATTATATTCCGA
CTGGGACATCTGCTTCCTGGAAGAGCCAAAGGCCCTGGTCTTTTCTTTTTTTGCCCTGCCTGGATACC
TACCACAAGGTTGACCTTCGTCTCCAAACTCTGGAGATACCTTTTCATGAGATCGTGACCAAAGACATG
TTTATAATGGAGATAGATGCCATTTGCTACTACCGAATGGAAAATGCCTCTCTTCTCCTAAGCAGTCTT
GCTCATGTATCTAAAGCTGTGCAATTCCTTGTGCAAACCACTATGAAGCGTCTCCTAGCACATCGATCC
CTCACTGAAATTCTTCTAGAGAGGAAGAGCATCGCCCAAGATGCAAAGGTTGCCTTGGATTCAGTGACC
TGTATTTGGGGAATCAAAGTGGAGAGAATAGAAATTAAAGATGTGAGGTTGCCAGCTGGGCTTCAGCAC
TCACTGGCTGTGGAGGCTGAAGCGCAAAGACAAGCCAAAGTGCGGATGATTGCTGCAGAAGCGGAAAAG
GCTGCTTCTGAGTCCCTGAGGATGGCAGCTGAGATTCTGTCAGGCACCCCTGCTGCCGTTCAGCTTCGA
TACCTCCACACCCTTCAGTCTCTGTCCACAGAGAAGCCTTCCACTGTGGTTTTACCTTTGCCATTTGAC
CTACTGAATTGCCTGTCTTCTCCCAGCAACAGAACTCAGGGAAGCCTCCCCTTCCCAAGTCCTTCCAAA
CCTGTTGAGCCACTAAATCCTAAAAAGAAAGACTCTCCCATGTTATAG
 Exemplary NPHS2 transgene sequence (SEQ ID NO: 57)

In some embodiments, a promoter comprising or consisting of a nucleotide sequence having at least 70%, at least 75%, at least 80%, at least 85%, at least 90%, at least 95%, at least 96%, at least 97%, at least 98%, at least 99%, or 100% identity to SEQ ID NO: 47 is operably linked to the protein-coding sequence encoding NPHS2, or a fragment and/or variant thereof.

In some embodiments, a promoter consisting of the nucleotide sequence of SEQ ID NO: 47 is operably linked to the protein-coding sequence encoding NPHS2, or a fragment and/or variant thereof.

### Vascular endothelial growth factor (VEGF)C transgene

The protein-coding sequence may comprise or consist of a vascular endothelial growth factor (VEGF)C transgene.

VEGFC is a lymphangiogenic growth factor, which is known to signal via two receptors, VEGFR-3 (Flt4) and VEGFR-2 (Flk4). VEGFC is produced by cells in a prepropeptide form, which dimerises before being cleaved into a tetramer.

The VEGFC transgene may comprises a polynucleotide encoding any form of VEGFC, such as the prepropeptide form, the tetramer form, the intermediate form, or fully processed mature VEGFC.

If desired, polynucleotides encoding different forms of VEGFC polypeptides may be used in any combination. Preferably the VEGFC transgene comprises a polynucleotide encoding one or more polypeptides having VEGFC biological activity, i.e., peptides that can bind to and activate VEGFR-2 and/or VEGRF-3. More preferably, the VEGFC transgene comprises a polynucleotide encoding a polypeptide comprising the VEGFC homology domain and having VEGFC biological activity, i.e., a polypeptide that can bind to and activate VEGFR-2 and/or VEGRF-3. Further details of suitable VEGFC polynucleotides and polypeptides include those described in WO 2015/022447 and US 2014/0087002.

The VEGFC polynucleotide may comprise the VEGFC open reading frame (ORF) sequence of SEQ ID NO: 36. The VEGFC polynucleotide may comprise a nucleic acid sequence which has at least 70%, at least 75%, at least 80%, at least 85%, at least 90%, at least 95%, at least 96%, at least 97%, at least 98%, or at least 99% sequence identity to the VEGFC ORF sequence of SEQ ID NO: 36. The variant sequence may encode a VEGFC polypeptide that has retained the capability to bind and activate VEGFR-2 and VEGFR-3.

*Exemplary VEGFC polynucleotide (SEQ ID NO: 36)*

ATGCACTTGCTGGGCTTCTTCTCTGTGGCGTGTTCTCTGCTCGCCGCTGCGCTGCTCCCGGGTCCTCG
CGAGGCGCCCGCCGCCGCCGCCTTCGAGTCCGGACTCGACCTCTCGGACGCGGAGCCCGACGCGG
GCGAGGCCACGGCTTATGCAAGCAAAGATCTGGAGGAGCAGTTACGGTCTGTGTCCAGTGTAGATGAA
CTCATGACTGTACTCTACCCAGAATATTGGAAAATGTACAAGTGTCAGCTAAGGAAAGGAGGCTGGCA
ACATAACAGAGAACAGGCCAACCTCAACTCAAGGACAGAAGAGACTATAAAATTTGCTGCAGCACATT
ATAATACAGAGATCTTGAAAAGTATTGATAATGAGTGGAGAAAGACTCAATGCATGCCACGGGAGGTG
TGTATAGATGTGGGGAAGGAGTTTGGAGTCGCGACAAACACCTTCTTTAAACCTCCATGTGTGTCCGT
CTACAGATGTGGGGGTTGCTGCAATAGTGAGGGGCTGCAGTGCATGAACACCAGCACGAGCTACCTCA
GCAAGACGTTATTTGAAATTACAGTGCCTCTCTCTCAAGGCCCCAAACCAGTAACAATCAGTTTTGCC
AATCACACTTCCTGCCGATGCATGTCTAAACTGGATGTTTACAGACAAGTTCATTCCATTATTAGACG
TTCCCTGCCAGCAACACTACCACAGTGTCAGGCAGCGAACAAGACCTGCCCCACCAATTACATGTGGA
ATAATCACATCTGCAGATGCCTGGCTCAGGAAGATTTTATGTTTTCCTCGGATGCTGGAGATGACTCA
ACAGATGGATTCCATGACATCTGTGGACCAAACAAGGAGCTGGATGAAGAGACCTGTCAGTGTGTCTG
CAGAGCGGGGCTTCGGCCTGCCAGCTGTGGACCCCACAAAGAACTAGACAGAAACTCATGCCAGTGTG
TCTGTAAAAACAAACTCTTCCCCAGCCAATGTGGGGCCAACCGAGAATTTGATGAAAACACATGCCAG

TGTGTATGTAAAAGAACCTGCCCCAGAAATCAACCCCTAAATCCTGGAAAATGTGCCTGTGAATGTAC
AGAAAGTCCACAGAAATGCTTGTTAAAAGGAAAGAAGTTCCACCACCAAACATGCAGCTGTTACAGAC
GGCCATGTACGAACCGCCAGAAGGCTTGTGAGCCAGGATTTTCATATAGTGAAGAAGTGTGTCGTTGT
GTCCCTTCATATTGGAAAAGACCACAAATGAGC

In some embodiments, a promoter comprising or consisting of a nucleotide sequence having at least 70%, at least 75%, at least 80%, at least 85%, at least 90%, at least 95%, at least 96%, at least 97%, at least 98%, at least 99%, or 100% identity to SEQ ID NO: 47 is operably linked to the VEGF(C) transgene.

In some embodiments, a promoter consisting of the nucleotide sequence of SEQ ID NO: 47 is operably linked to the VEGF(C) transgene.

### Complement proteins and complement inhibitors

The protein-coding sequence may encode a complement protein, or a fragment and/or variant thereof.

As used herein, a "complement protein" is a protein which is part of the complement system. The complement system, also known as complement cascade, is a central part of the innate immunity that serves as a first line of defence against foreign and altered host cells. The complement system is composed of plasma proteins produced mainly by the liver or membrane proteins expressed on cell surface. Complement operates in plasma, in tissues, or within cells. Complement proteins collaborate as a cascade to opsonize pathogens and induce a series of inflammatory responses helping immune cells to fight infection and maintain homeostasis (Merle, N.S., et al., 2015. Frontiers in immunology, 6, 262).

There are three pathways of complement activation: the classical, the alternative, and the lectin pathways. The three complement pathways differ in their mechanisms of target recognition but converge in the activation of the central component C3. After this activation, C5 is cleaved, and the assembly of the membrane attack complex (MAC) is initiated. The enzymatic cleavage of C3 and C5 leads to the production and release of anaphylotoxins C3a and C5a.

Suitably, the complement protein is selected from the list consisting of CFI, CFH, FHL-1, C1INH, C4BP, MASP2, C3, C5aR1, C5, C5a, CD55, CD35, CD46, CD59, vitronectin, and clusterin, or fragments and/or variants thereof.

The protein-coding sequence may encode an inhibitor of the complement system, or a fragment and/or variant thereof.

As used herein, an "inhibitor of the complement system" or "complement inhibitor" is a protein which prevents activation of the complement system. Complement is tightly controlled by these inhibitors, which naturally protect self cells and tissues from unwanted complement activation. Complement inhibitors can regulate complement activation in different stages of the classical, lectin, and alternative pathways. Suitably, the complement inhibitor is a naturally-occurring complement inhibitor, or a fragment and/or variant thereof. Preferably, the inhibitor of the complement system is an inhibitor of the complement system in humans.

Complement inhibitors are grouped into two categories: soluble inhibitors and membrane-bound inhibitors. Preferably, the inhibitor of the complement system is a soluble complement inhibitor. Soluble complement inhibitors include C1 inhibitor (C1INH), complement factor I (CFI), complement factor H (CFH), complement factor H-like protein 1 (FHL-1), C4 binding protein (C4BP), clusterin and vitronectin. Membrane-bound regulators include CD46, CD55, CD59, CD35 and CUB and Sushi multiple domain 1 (CSMD1).

The inhibitor of the complement system may be selected from: CFI, CFH, FHL-1, C1INH, C4BP, CD46, CD55, CD59, CD35, vitronectin, clusterin, and CSMD1, or fragments and/or variants thereof.

Preferably, the inhibitor of the complement system is selected from: CFI, CFH, and FHL-1, or fragments and/or variants thereof.

### CFI

The protein-coding sequence may encode CFI, or a fragment and/or variant thereof.

Complement factor I (CFI) is a trypsin-like serine protease that inhibits the complement system by cleaving three peptide bonds in the alpha-chain of C3b and two bonds in the alpha-chain of C4b thereby inactivating these proteins.

CFI is a glycoprotein heterodimer consisting of a disulfide linked heavy chain and light chain. The heavy chain has four domains: an FI membrane attack complex (FIMAC) domain, CD5 domain, and low density lipoprotein receptor 1 and 2 (LDLr1 and LDLr2) domains. The heavy chain plays an inhibitory role in maintaining the enzyme inactive until it meets the complex formed by the substrate (either C3b or C4b) and a cofactor protein (Factor H, C4b-binding protein, complement receptor 1, and membrane cofactor protein). Upon binding of the enzyme to the substrate:cofactor complex, the heavy:light chain interface is disrupted, and the enzyme activated by allostery. The light chain contains only the serine protease domain. This domain contains the catalytic triad His-362, Asp-411, and Ser-507, which is responsible for specific cleavage of C3b and C4b.

The CFI or a fragment and/or variant thereof may be capable of cleaving C3b into iC3b and/or may be capable of cleaving iC3b into C3d,g.

The fragment and/or variant of CFI may retain at least 50%, 60%, 70%, 80%, 90%, 95% or 100% of the C3b-inactivating and iC3b-degradation activity of native CFl. The C3b-inactivating and iC3b-degradation activity of the fragment and/or variant of CFI and native CFI, may be determined using any suitable method known to those of skill in the art. For example, using a proteolytic assay.

Preferably, the CFI is a human CFI. An example human CFI is the CFI having the UniProtKB accession number P05156.

Suitably, the CFI may comprise or consist of the polypeptide sequence shown as SEQ ID NO: 49, or a variant which is at least 70% identical to SEQ ID NO: 49.

*Illustrative CFI polypeptide sequence* (SEQ ID NO: 49):

MKLLHVFLLFLCFHLRFCKVTYTSQEDLVEKKCLAKKYTHLSCDKVFCQPWQRCIEGTCVCKLPYQCP
KNGTAVCATNRRSFPTYCQQKSLECLHPGTKFLNNGTCTAEGKFSVSLKHGNTDSEGIVEVKLVDQDK
TMFICKSSWSMREANVACLDLGFQQGADTQRRFKLSDLSINSTECLHVHCRGLETSLAECTFTKRRTM
GYQDFADVVCYTQKADSPMDDFFQCVNGKYISQMKACDGINDCGDQSDELCCKACQGKGFHCKSGVCI
PSQYQCNGEVDCITGEDEVGCAGFASVTQEETEILTADMDAERRRIKSLLPKLSCGVKNRMHIRRKRI
VGGKRAQLGDLPWQVAIKDASGITCGGIYIGGCWILTAAHCLRASKTHRYQIWTTVVDWIHPDLKRIV
IEYVDRIIFHENYNAGTYQNDIALIEMKKDGNKKDCELPRSIPACVPWSPYLFQPNDTCIVSGWGREK
DNERVFSLQWGEVKLISNCSKFYGNRFYEKEMECAGTYDGSIDACKGDSGGPLVCMDANNVTYVWGVV
SWGENCGKPEFPGVYTKVANYFDWISYHVGRPFISQYNV

Suitably, the variant may be at least 75%, at least 80%, at least 85%, at least 90%, at least 95%, at least 96%, at least 97%, at least 98%, at least 99% identical to SEQ ID NO: 49.

An example nucleotide sequence encoding CFI is NM_000204.5. Suitably, a protein-coding sequence encoding CFI may comprise or consist of the polynucleotide sequence shown as SEQ ID NO: 50, or a variant which is at least 70% identical to SEQ ID NO: 50.

*Illustrative CFI polynucleotide sequence* (SEQ ID NO: 50):

ATGAAGCTTCTTCATGTTTTCCTGTTATTTCTGTGCTTCCACTTAAGGTTTTGCAAGGTCACTTATAC
ATCTCAAGAGGATCTGGTGGAGAAAAAGTGCTTAGCAAAAAAATATACTCACCTCTCCTGCGATAAAG
TCTTCTGCCAGCCATGGCAGAGATGCATTGAGGGCACCTGTGTTTGTAAACTACCGTATCAGTGCCCA
AAGAATGGCACTGCAGTGTGTGCAACTAACAGGAGAAGCTTCCCAACATACTGTCAACAAAAGAGTTT
GGAATGTCTTCATCCAGGGACAAAGTTTTTAAATAACGGAACATGCACAGCCGAAGGAAAGTTTAGTG
TTTCCTTGAAGCATGGAAATACAGATTCAGAGGGAATAGTTGAAGTAAAACTTGTGGACCAAGATAAG
ACAATGTTCATATGCAAAAGCAGCTGGAGCATGAGGGAAGCCAACGTGGCCTGCCTTGACCTTGGGTT
TCAACAAGGTGCTGATACTCAAAGAAGGTTTAAGTTGTCTGATCTCTCTATAAATTCCACTGAATGTC
TACATGTGCATTGCCGAGGATTAGAGACCAGTTTGGCTGAATGTACTTTTACTAAGAGAAGAACTATG
GGTTACCAGGATTTCGCTGATGTGGTTTGTTATACACAGAAAGCAGATTCTCCAATGGATGACTTCTT
TCAGTGTGTGAATGGGAAATACATTTCTCAGATGAAAGCCTGTGATGGTATCAATGATTGTGGAGACC

AAAGTGATGAACTGTGTTGTAAAGCATGCCAAGGCAAAGGCTTCCATTGCAAATCGGGTGTTTGCATT
CCAAGCCAGTATCAATGCAATGGTGAGGTGGACTGCATTACAGGGGAAGATGAAGTTGGCTGTGCAGG
CTTTGCATCTGTGACTCAAGAAGAAACAGAAATTTTGACTGCTGACATGGATGCAGAAAGAAGACGGA
TAAAATCATTATTACCTAAACTATCTTGTGGAGTTAAAAACAGAATGCACATTCGAAGGAAACGAATT
GTGGGAGGAAAGCGAGCACAACTGGGAGACCTCCCATGGCAGGTGGCAATTAAGGATGCCAGTGGAAT
CACCTGTGGGGGAATTTATATTGGTGGCTGTTGGATTCTGACTGCTGCACATTGTCTCAGAGCCAGTA
AAACTCATCGTTACCAAATATGGACAACAGTAGTAGACTGGATACACCCCGACCTTAAACGTATAGTA
ATTGAATACGTGGATAGAATTATTTTCCATGAAAACTACAATGCAGGCACTTACCAAAATGACATCGC
TTTGATTGAAATGAAAAAAGACGGAAACAAAAAAGATTGTGAGCTGCCTCGTTCCATCCCTGCCTGTG
TCCCCTGGTCTCCTTACCTATTCCAACCTAATGATACATGCATCGTTTCTGGCTGGGGACGAGAAAAA
GATAACGAAAGAGTCTTTTCACTTCAGTGGGGTGAAGTTAAACTAATAAGCAACTGCTCTAAGTTTTA
CGGAAATCGTTTCTATGAAAAAGAAATGGAATGTGCAGGTACATATGATGGTTCCATCGATGCCTGTA
AAGGGGACTCTGGAGGCCCCTTAGTCTGTATGGATGCCAACAATGTGACTTATGTCTGGGGTGTTGTG
AGTTGGGGGGAAAACTGTGGAAAACCAGAGTTCCCAGGTGTTTACACCAAAGTGGCCAATTATTTTGA
CTGGATTAGCTACCATGTAGGAAGGCCTTTTATTTCTCAGTACAATGTATAA

Suitably, the variant may be at least 75%, at least 80%, at least 85%, at least 90%, at least 95%, at least 96%, at least 97%, at least 98%, at least 99% identical to SEQ ID NO: 50.

In some embodiments, a promoter comprising or consisting of a nucleotide sequence having at least 70%, at least 75%, at least 80%, at least 85%, at least 90%, at least 95%, at least 96%, at least 97%, at least 98%, at least 99%, or 100% identity to SEQ ID NO: 47 is operably linked to the protein-coding sequence encoding CFI, or a fragment and/or variant thereof.

In some embodiments, a promoter consisting of the nucleotide sequence of SEQ ID NO: 47 is operably linked to the protein-coding sequence encoding CFI, or a fragment and/or variant thereof.

### CFH

The protein-coding sequence may encode CFH, or a fragment and/or variant thereof.

Complement factor H (CFH) regulates complement activation on self cells and surfaces. CFH competes for binding of complement factor B (CFB) to C3b, acts as a cofactor for CFI-catalysed proteolytic cleavage of C3b, and accelerates the irreversible dissociation of C3bBb and C3b2Bb into their separate components. Thus, CFH not only inhibits formation of the convertases but it also shortens the lifespan of any convertase complex that forms.

CFH is a large (155 kDa) soluble glycoprotein. CFH is composed from a total of 20 domains, each containing approximately 60 amino acid residues and termed complement control protein modules (CCPs) or short consensus repeats that are joined by short linkers consisting of 3-8 residues. The CCP modules are numbered from 1-20 (from the N-terminus of the protein): CCPs 1-4 and CCPs 19-20 engage with C3b while CCPs 7 and CCPs 19-20 bind to GAGs and sialic acid.

The CFH or a fragment and/or variant thereof may be capable of binding C3b and/or C3d; and/or acting as a cofactor for the CFI-catalysed proteolytic cleavage of C3b; and/or increasing the irreversible dissociation of C3bBb and C3b2Bb into their separate components. The fragment and/or variant of CFH may retain at least 50%, 60%, 70%, 80%, 90%, 95% or 100% of the activity of native CFH. The activity of the fragment and/or variant of CFH and native CFH may be determined using any suitable method known to those of skill in the art.

Preferably, the CFH is a human CFH. An example human CFH is the CFH having the UniProtKB accession number P08603.

Suitably, the CFH may comprise or consist of the polypeptide sequence shown as SEQ ID NO: 51, or a variant which is at least 70% identical to SEQ ID NO: 51.

*Illustrative CFH polypeptide sequence* (SEQ ID NO: 51):

MRLLAKIIICLMLWAICVAEDCNELPPRRNTEILTGSWSDQTYPEGTQAIYKCRPGYRSLGNVIMVCRK
GEWVALNPLRKCQKRPCGHPGDTPFGTFTLTGGNVFEYGVKAVYTCNEGYQLLGEINYRECDTDGWTN
DIPICEVVKCLPVTAPENGKIVSSAMEPDREYHFGQAVRFVCNSGYKIEGDEEMHCSDDGFWSKEKPK
CVEISCKSPDVINGSPISQKIIYKENERFQYKCNMGYEYSERGDAVCTESGWRPLPSCEEKSCDNPYI
PNGDYSPLRIKHRTGDEITYQCRNGFYPATRGNTAKCTSTGWIPAPRCTLKPCDYPDIKHGGLYHENM
RRPYFPVAVGKYYSYYCDEHFETPSGSYWDHIHCTQDGWSPAVPCLRKCYFPYLENGYNQNYGRKFVQ
GKSIDVACHPGYALPKAQTTVTCMENGWSPTPRCIRVKTCSKSSIDIENGFISESQYTYALKEKAKYQ
CKLGYVTADGETSGSITCGKDGWSAQPTCIKSCDIPVFMNARTKNDFTWFKLNDTLDYECHDGYESNT
GSTTGSIVCGYNGWSDLPICYERECELPKIDVHLVPDRKKDQYKVGEVLKFSCKPGFTIVGPNSVQCY
HFGLSPDLPICKEQVQSCGPPPELLNGNVKEKTKEEYGHSEVVEYYCNPRFLMKGPNKIQCVDGEWTT
LPVCIVEESTCGDIPELEHGWAQLSSPPYYYGDSVEFNCSESFTMIGHRSITCIHGVWTQLPQCVAID
KLKKCKSSNLIILEEHLKNKKEFDHNSNIRYRCRGKEGWIHTVCINGRWDPEVNCSMAQIQLCPPPPQ
IPNSHNMTTTLNYRDGEKVSVLCQENYLIQEGEEITCKDGRWQSIPLCVEKIPCSQPPQIEHGTINSS
RSSQESYAHGTKLSYTCEGGFRISEENETTCYMGKWSSPPQCEGLPCKSPPEISHGVVAHMSDSYQYG
EEVTYKCFEGFGIDGPAIAKCLGEKWSHPPSCIKTDCLSLPSFENAIPMGEKKDVYKAGEQVTYTCAT
YYKMDGASNVTCINSRWTGRPTCRDTSCVNPPTVQNAYIVSRQMSKYPSGERVRYQCRSPYEMFGDEE
VMCLNGNWTEPPQCKDSTGKCGPPPPIDNGDITSFPLSVYAPASSVEYQCQNLYQLEGNKRITCRNGQ
WSEPPKCLHPCVISREIMENYNIALRWTAKQKLYSRTGESVEFVCKRGYRLSSRSHTLRTTCWDGKLE
YPTCAKR

Suitably, the variant may be at least 75%, at least 80%, at least 85%, at least 90%, at least 95%, at least 96%, at least 97%, at least 98%, at least 99% identical to SEQ ID NO: 51.

An example nucleotide sequence encoding CFH is NM_000186.4. Suitably, a protein-coding sequence encoding CFH may comprise or consist of the polynucleotide sequence shown as SEQ ID NO: 52, or a variant which is at least 70% identical to SEQ ID NO: 52.

*Illustrative CFH polynucleotide sequence* (SEQ ID NO: 52):

ATGAGACTTCTAGCAAAGATTATTTGCCTTATGTTATGGGCTATTTGTGTAGCAGAAGATTGCAATGA
ACTTCCTCCAAGAAGAAATACAGAAATTCTGACAGGTTCCTGGTCTGACCAAACATATCCAGAAGGCA
CCCAGGCTATCTATAAATGCCGCCCTGGATATAGATCTCTTGGAAATGTAATAATGGTATGCAGGAAG
GGAGAATGGGTTGCTCTTAATCCATTAAGGAAATGTCAGAAAAGGCCCTGTGGACATCCTGGAGATAC

TCCTTTTGGTACTTTTACCCTTACAGGAGGAAATGTGTTTGAATATGGTGTAAAAGCTGTGTATACAT
GTAATGAGGGGTATCAATTGCTAGGTGAGATTAATTACCGTGAATGTGACACAGATGGATGGACCAAT
GATATTCCTATATGTGAAGTTGTGAAGTGTTTACCAGTGACAGCACCAGAGAATGGAAAAATTGTCAG
TAGTGCAATGGAACCAGATCGGGAATACCATTTTGGACAAGCAGTACGGTTTGTATGTAACTCAGGCT
ACAAGATTGAAGGAGATGAAGAAATGCATTGTTCAGACGATGGTTTTTGGAGTAAAGAGAAACCAAAG
TGTGTGGAAATTTCATGCAAATCCCCAGATGTTATAAATGGATCTCCTATATCTCAGAAGATTATTTA
TAAGGAGAATGAACGATTTCAATATAAATGTAACATGGGTTATGAATACAGTGAAAGAGGAGATGCTG
TATGCACTGAATCTGGATGGCGTCCGTTGCCTTCATGTGAAGAAAAATCATGTGATAATCCTTATATT
CCAAATGGTGACTACTCACCTTTAAGGATTAAACACAGAACTGGAGATGAAATCACGTACCAGTGTAG
AAATGGTTTTTATCCTGCAACCCGGGGAAATACAGCAAAATGCACAAGTACTGGCTGGATACCTGCTC
CGAGATGTACCTTGAAACCTTGTGATTATCCAGACATTAAACATGGAGGTCTATATCATGAGAATATG
CGTAGACCATACTTTCCAGTAGCTGTAGGAAAATATTACTCCTATTACTGTGATGAACATTTTGAGAC
TCCGTCAGGAAGTTACTGGGATCACATTCATTGCACACAAGATGGATGGTCGCCAGCAGTACCATGCC
TCAGAAAATGTTATTTTCCTTATTTGGAAAATGGATATAATCAAAATCATGGAAGAAAGTTTGTACAG
GGTAAATCTATAGACGTTGCCTGCCATCCTGGCTACGCTCTTCCAAAAGCGCAGACCACAGTTACATG
TATGGAGAATGGCTGGTCTCCTACTCCCAGATGCATCCGTGTCAAAACATGTTCCAAATCAAGTATAG
ATATTGAGAATGGGTTTATTTCTGAATCTCAGTATACATATGCCTTAAAAGAAAAAGCGAAATATCAA
TGCAAACTAGGATATGTAACAGCAGATGGTGAAACATCAGGATCAATTACATGTGGGAAAGATGGATG
GTCAGCTCAACCCACGTGCATTAAATCTTGTGATATCCCAGTATTTATGAATGCCAGAACTAAAAATG
ACTTCACATGGTTTAAGCTGAATGACACATTGGACTATGAATGCCATGATGGTTATGAAAGCAATACT
GGAAGCACCACTGGTTCCATAGTGTGTGGTTACAATGGTTGGTCTGATTTACCCATATGTTATGAAAG
AGAATGCGAACTTCCTAAAATAGATGTACACTTAGTTCCTGATCGCAAGAAAGACCAGTATAAAGTTG
GAGAGGTGTTGAAATTCTCCTGCAAACCAGGATTTACAATAGTTGGACCTAATTCCGTTCAGTGCTAC
CACTTTGGATTGTCTCCTGACCTCCCAATATGTAAAGAGCAAGTACAATCATGTGGTCCACCTCCTGA
ACTCCTCAATGGGAATGTTAAGGAAAAAACGAAAGAAGAATATGGACACAGTGAAGTGGTGGAATATT
ATTGCAATCCTAGATTTCTAATGAAGGGACCTAATAAAATTCAATGTGTTGATGGAGAGTGGACAACT
TTACCAGTGTGTATTGTGGAGGAGAGTACCTGTGGAGATATACCTGAACTTGAACATGGCTGGGCCCA
GCTTTCTTCCCCTCCTTATTACTATGGAGATTCAGTGGAATTCAATTGCTCAGAATCATTTACAATGA
TTGGACACAGATCAATTACGTGTATTCATGGAGTATGGACCCAACTTCCCCAGTGTGTGGCAATAGAT
AAACTTAAGAAGTGCAAATCATCAAATTTAATTATACTTGAGGAACATTTAAAAAACAAGAAGGAATT
CGATCATAATTCTAACATAAGGTACAGATGTAGAGGAAAAGAAGGATGGATACACACAGTCTGCATAA
ATGGAAGATGGGATCCAGAAGTGAACTGCTCAATGGCACAAATACAATTATGCCCACCTCCACCTCAG
ATTCCCAATTCTCACAATATGACAACCACACTGAATTATCGGGATGGAGAAAAAGTATCTGTTCTTTG
CCAAGAAAATTATCTAATTCAGGAAGGAGAAGAAATTACATGCAAAGATGGAAGATGGCAGTCAATAC
CACTCTGTGTTGAAAAAATTCCATGTTCACAACCACCTCAGATAGAACACGGAACCATTAATTCATCC
AGGTCTTCACAAGAAAGTTATGCACATGGGACTAAATTGAGTTATACTTGTGAGGGTGGTTTCAGGAT
ATCTGAAGAAAATGAAACAACATGCTACATGGGAAAATGGAGTTCTCCACCTCAGTGTGAAGGCCTTC
CTTGTAAATCTCCACCTGAGATTTCTCATGGTGTTGTAGCTCACATGTCAGACAGTTATCAGTATGGA
GAAGAAGTTACGTACAAATGTTTTGAAGGTTTTGGAATTGATGGGCCTGCAATTGCAAAATGCTTAGG
AGAAAAATGGTCTCACCCTCCATCATGCATAAAAACAGATTGTCTCAGTTTACCTAGCTTTGAAAATG
CCATACCCATGGGAGAGAAGAAGGATGTGTATAAGGCGGGTGAGCAAGTGACTTACACTTGTGCAACA
TATTACAAAATGGATGGAGCCAGTAATGTAACATGCATTAATAGCAGATGGACAGGAAGGCCAACATG
CAGAGACACCTCCTGTGTGAATCCGCCCACAGTACAAAATGCTTATATAGTGTCGAGACAGATGAGTA
AATATCCATCTGGTGAGAGAGTACGTTATCAATGTAGGAGCCCTTATGAAATGTTTGGGGATGAAGAA
GTGATGTGTTTAAATGGAAACTGGACGGAACCACCTCAATGCAAAGATTCTACAGGAAAATGTGGGCC
CCCTCCACCTATTGACAATGGGGACATTACTTCATTCCCGTTGTCAGTATATGCTCCAGCTTCATCAG
TTGAGTACCAATGCCAGAACTTGTATCAACTTGAGGGTAACAAGCGAATAACATGTAGAAATGGACAA
TGGTCAGAACCACCAAAATGCTTACATCCGTGTGTAATATCCCGAGAAATTATGGAAAATTATAACAT
AGCATTAAGGTGGACAGCCAAACAGAAGCTTTATTCGAGAACAGGTGAATCAGTTGAATTTGTGTGTA
AACGGGGATATCGTCTTTCATCACGTTCTCACACATTGCGAACAACATGTTGGGATGGGAAACTGGAG
TATCCAACTTGTGCAAAAAGATAG

Suitably, the variant may be at least 75%, at least 80%, at least 85%, at least 90%, at least 95%, at least 96%, at least 97%, at least 98%, at least 99% identical to SEQ ID NO: 52.

The CFH fragment may be a splice variant. For example, complement factor H-like protein 1 (FHL-1) is a CFH gene splice variant, which is almost identical to the N-terminal 7 domains of CFH (CCPs 1-7).

In some embodiments, a promoter comprising or consisting of a nucleotide sequence having at least 70%, at least 75%, at least 80%, at least 85%, at least 90%, at least 95%, at least 96%, at least 97%, at least 98%, at least 99%, or 100% identity to SEQ ID NO: 47 is operably linked to the protein-coding sequence encoding CFH, or a fragment and/or variant thereof.

In some embodiments, a promoter consisting of the nucleotide sequence of SEQ ID NO: 47 is operably linked to the protein-coding sequence encoding CFH, or a fragment and/or variant thereof.

### FHL-1

The protein-coding sequence may encode FHL-1, or a fragment and/or variant thereof.

FHL-1 or a fragment and/or variant thereof may be capable of binding C3b and/or C3d. The fragment and/or variant of FHL-1 may retain at least 50%, 60%, 70%, 80%, 90%, 95% or 100% of the activity of native FHL-1. The activity of the fragment and/or variant of FHL-1 and native FHL-1 may be determined using any suitable method known to those of skill in the art.

Preferably, the FHL-1 is a human FHL-1. An example human FHL-1 is the FHL-1 having the NCBI Reference Sequence: NP_001014975.1.

Suitably, the FHL-1 may comprise or consist of the polypeptide sequence shown as SEQ ID NO: 53, or a variant which is at least 70% identical to SEQ ID NO: 53.

*Illustrative FHL-1 polypeptide sequence* (SEQ ID NO: 53):

MRLLAKIICLMLWAICVAEDCNELPPRRNTEILTGSWSDQTYPEGTQAIYKCRPGYRSLGNVIMVCRK
GEWVALNPLRKCQKRPCGHPGDTPFGTFTLTGGNVFEYGVKAVYTCNEGYQLLGEINYRECDTDGWTN
DIPICEVVKCLPVTAPENGKIVSSAMEPDREYHFGQAVRFVCNSGYKIEGDEEMHCSDDGFWSKEKPK
CVEISCKSPDVINGSPISQKIIYKENERFQYKCNMGYEYSERGDAVCTESGWRPLPSCEEKSCDNPYI
PNGDYSPLRIKHRTGDEITYQCRNGFYPATRGNTAKCTSTGWIPAPRCTLKPCDYPDIKHGGLYHENM
RRPYFPVAVGKYYSYYCDEHFETPSGSYWDHIHCTQDGWSPAVPCLRKCYFPYLENGYNQNHGRKFVQ
GKSIDVACHPGYALPKAQTTVTCMENGWSPTPRCIRVSFTL

Suitably, the variant may be at least 75%, at least 80%, at least 85%, at least 90%, at least 95%, at least 96%, at least 97%, at least 98%, at least 99% identical to SEQ ID NO: 53.

An example nucleotide sequence encoding FHL-1 is NM_001014975.2. Suitably, a protein-coding sequence encoding FHL-1 may comprise or consist of the polynucleotide sequence shown as SEQ ID NO: 54, or a variant which is at least 70% identical to SEQ ID NO: 54.

*Illustrative FHL-1 polynucleotide sequence* (SEQ ID NO: 54):

ATGAGACTTCTAGCAAAGATTATTTGCCTTATGTTATGGGCTATTTGTGTAGCAGAAGATTGCAATGA
ACTTCCTCCAAGAAGAAATACAGAAATTCTGACAGGTTCCTGGTCTGACCAAACATATCCAGAAGGCA
CCCAGGCTATCTATAAATGCCGCCCTGGATATAGATCTCTTGGAAATGTAATAATGGTATGCAGGAAG
GGAGAATGGGTTGCTCTTAATCCATTAAGGAAATGTCAGAAAAGGCCCTGTGGACATCCTGGAGATAC
TCCTTTTGGTACTTTTACCCTTACAGGAGGAAATGTGTTTGAATATGGTGTAAAAGCTGTGTATACAT
GTAATGAGGGGTATCAATTGCTAGGTGAGATTAATTACCGTGAATGTGACACAGATGGATGGACCAAT
GATATTCCTATATGTGAAGTTGTGAAGTGTTTACCAGTGACAGCACCAGAGAATGGAAAAATTGTCAG
TAGTGCAATGGAACCAGATCGGGAATACCATTTTGGACAAGCAGTACGGTTTGTATGTAACTCAGGCT
ACAAGATTGAAGGAGATGAAGAAATGCATTGTTCAGACGATGGTTTTTGGAGTAAAGAGAAACCAAAG
TGTGTGGAAATTTCATGCAAATCCCCAGATGTTATAAATGGATCTCCTATATCTCAGAAGATTATTTA
TAAGGAGAATGAACGATTTCAATATAAATGTAACATGGGTTATGAATACAGTGAAAGAGGAGATGCTG
TATGCACTGAATCTGGATGGCGTCCGTTGCCTTCATGTGAAGAAAAATCATGTGATAATCCTTATATT
CCAAATGGTGACTACTCACCTTTAAGGATTAAACACAGAACTGGAGATGAAATCACGTACCAGTGTAG
AAATGGTTTTTATCCTGCAACCCGGGGAAATACAGCAAAATGCACAAGTACTGGCTGGATACCTGCTC
CGAGATGTACCTTGAAACCTTGTGATTATCCAGACATTAAACATGGAGGTCTATATCATGAGAATATG
CGTAGACCATACTTTCCAGTAGCTGTAGGAAAATATTACTCCTATTACTGTGATGAACATTTTGAGAC
TCCGTCAGGAAGTTACTGGGATCACATTCATTGCACACAAGATGGATGGTCGCCAGCAGTACCATGCC
TCAGAAAATGTTATTTTCCTTATTTGGAAAATGGATATAATCAAAATCATGGAAGAAAGTTTGTACAG
GGTAAATCTATAGACGTTGCCTGCCATCCTGGCTACGCTCTTCCAAAAGCGCAGACCACAGTTACATG
TATGGAGAATGGCTGGTCTCCTACTCCCAGATGCATCCGTGTCAGCTTTACCCTCTGA

Suitably, the variant may be at least 75%, at least 80%, at least 85%, at least 90%, at least 95%, at least 96%, at least 97%, at least 98%, at least 99% identical to SEQ ID NO: 54.

In some embodiments, a promoter comprising or consisting of a nucleotide sequence having at least 70%, at least 75%, at least 80%, at least 85%, at least 90%, at least 95%, at least 96%, at least 97%, at least 98%, at least 99%, or 100% identity to SEQ ID NO: 47 is operably linked to the protein-coding sequence encoding FHL-1, or a fragment and/or variant thereof.

In some embodiments, a promoter consisting of the nucleotide sequence of SEQ ID NO: 47 is operably linked to the protein-coding sequence encoding FHL-1, or a fragment and/or variant thereof.

### Other regulatory elements

In addition to the promoter of the invention, the polynucleotide may comprise one or more further regulatory sequences which may act pre- or post-transcriptionally. Suitably, the protein-coding sequence may be operably linked to one or more further regulatory sequences. The one or more further regulatory sequences may facilitate expression of the protein in glomerular cells (e.g. podocytes).

"Regulatory sequences" are any sequences which facilitate expression of the polypeptides, e.g. act to increase expression of a transcript or to enhance mRNA stability. Suitable further regulatory sequences include for example enhancer elements, post-transcriptional regulatory elements and polyadenylation sites.

Suitably, the polynucleotide of the present invention does not comprise any further regulatory sequences (except the promoter of the invention).

### Enhancers

The polynucleotide of the invention may comprise an enhancer. Suitably, the enhancer may be operably linked to the protein-coding sequence. The enhancer may facilitate expression of the protein in glomerular cells (e.g. podocytes). Suitably, the enhancer is a mammalian enhancer e.g. a human enhancer.

An "enhancer" is a region of DNA that can be bound by proteins (activators) to increase the likelihood that transcription of a particular gene will occur. Enhancers are cis-acting. They can be located up to 1 Mbp (1,000,000 bp) away from the gene, upstream or downstream from the start site. Any suitable enhancer may be used, the selection of which may be readily made by the skilled person.

The polynucleotide of the invention may comprise a tissue specific enhancer. As used herein, a "tissue-specific enhancer" is an enhancer which preferentially facilitates expression of a gene in specific cells or tissues. Suitably, a tissue-specific enhancer may facilitate higher expression of a gene in specific cells-types as compared to other cell-types. Higher expression may be measured for example by measuring the expression of a transgene, e.g. GFP, operably linked to the enhancer, wherein expression of the transgene correlates with the ability of the enhancer to facilitate expression of a gene. For example, a tissue-specific enhancer may be an enhancer which facilitates gene expression levels at least 10% higher, at least 20% higher, at least 30% higher, at least 40% higher, at least 50% higher, at least 100% higher, at least 200% higher, at least 300% higher, at least 400% higher, at least 500% higher, or at least 1000% higher in a specific cell-type compared to expression levels in other cell types.

Suitable tissue-specific enhancer will be well known to those of skill in the art. The enhancer may be a kidney-specific enhancer, preferably a glomerular-specific enhancer, more preferably a podocyte-specific enhancer. Suitably, the enhancer may be operably linked to the protein-coding sequence.

Suitably, the enhancer may be or may be derived from an enhancer associated with a gene with selective expression in human kidney, glomerular cells, and/or podocytes. Methods to identify the enhancer regions associated with genes will be well known to those of skill in the art.

Suitably, the podocyte-specific enhancer is a NPHS1 or a NPHS2 enhancer, or a fragment or derivative thereof. Suitably, the podocyte-specific enhancer is a NPHS1 enhancer, or a fragment or derivative thereof.

A NPHS1 enhancer has been described in Guo, G., et al., 2004. Journal of the American Society of Nephrology, 15(11), pp.2851-2856. A 186-bp fragment from the human NPHS1 promoter was capable of directing podocyte-specific expression of a β-galactosidase transgene when placed in front of a heterologous minimal promoter in transgenic mice.

Suitably, the NPHS1 enhancer may comprise or consist of the nucleotide sequence shown as SEQ ID NO: 27, or a variant which is at least 70% identical to SEQ ID NO: 27.

*Exemplary NPHS1 enhancer (SEQ ID NO: 27):*

ctgctgagctgggagaccaccttgatctgacttctcccatcttcccagcctaagccaggccctggggt
cacggaggctggggaggcaccgaggaacgcgcctggcatgtgctgacaggggatttatgctccagct
gggccagctgggaggagcctgctgggcagaggccagagctgggggctctg

Suitably, the variant may be at least 75%, at least 80%, at least 85%, at least 90%, at least 95%, at least 96%, at least 97%, at least 98% or at least 99% identical to SEQ ID NO: 27.

Suitably, the polynucleotide of the present invention does not comprise an enhancer.

### Kozak sequence

The polynucleotide of the invention may comprise a Kozak sequence. Suitably, the Kozak sequence may be operably linked to the protein-coding sequence. A Kozak sequence may be inserted before the start codon of the protein to improve the initiation of translation.

Suitable Kozak sequences will be well known to those of skill in the art.

Suitably, the Kozak sequence may comprise or consist of the nucleotide sequence shown as SEQ ID NO: 28, or a variant which is at least 65% identical to SEQ ID NO: 28.

*Exemplary Kozak sequence (SEQ ID NO: 28):*

### GCCGCCACCAUGG

Suitably, the variant may be at least 75%, at least 85%, or at least 90% identical to SEQ ID NO: 28.

Suitably, the polynucleotide of the present invention does not comprise a Kozak sequence.

### Post-transcriptional regulatory elements

The polynucleotide of the invention may comprise a post-transcriptional regulatory element. Suitably, the post-transcriptional regulatory element may be operably linked to the protein-coding sequence. The post-transcriptional regulatory element may improve gene expression.

The polynucleotide may comprise a Woodchuck Hepatitis Virus Post-transcriptional Regulatory Element (WPRE). Suitably, the WPRE may be operably linked to the protein-coding sequence.

The WPRE sequence may have substitutions, deletions, or insertions within the X-antigen promoter and/or the initiation codon of the X-antigen. This may prevent the production of a functional X-antigen.

Suitably, the WPRE may comprise or consist of the nucleotide sequence shown as SEQ ID NO: 29, or a variant which is at least 70% identical to SEQ ID NO: 29.

*Exemplary WPRE (SEQ ID NO: 29):*

aatcaacctctggattacaaaatttgtgaaagattgactggtattcttaactatgttgctccttttac
gctatgtggatacgctgctttaatgcctttgtatcatgctattgcttcccgtatggctttcattttct
cctccttgtataaatcctggttgctgtctctttatgaggagttgtggcccgttgtcaggcaacgtggc
gtggtgtgcactgtgtttgctgacgcaacccccactggttggggcattgccaccacctgtcagctcct
ttccgggactttcgctttccccctccctattgccacggcggaactcatcgccgcctgccttgcccgct
gctggacaggggctcggctgttgggcactgacaattccgtggtgttgtcggggaaatcatcgtccttt
ccttggctgctcgcctgtgttgccacctggattctgcgcgggacgtccttctgctacgtcccttcggc
cctcaatccagcggaccttccttcccgcggcctgctgccggctctgcggcctcttccgcgtcttcgcc
ttcgccctcagacgagtcggatctccctttgggccgcctccccgc

Suitably, the variant may be at least 75%, at least 80%, at least 85%, at least 90%, at least 95%, at least 96%, at least 97%, at least 98%, at least 99% identical to SEQ ID NO: 29.

Suitably, the polynucleotide of the present invention does not comprise a post-transcriptional regulatory element.

### Polyadenylation signal

The polynucleotide of the invention may comprise a polyadenylation signal. Suitably, the polyadenylation signal may be operably linked to the protein-coding sequence. The polyadenylation signal may improve gene expression.

Suitable polyadenylation signals include the early SV40 polyadenylation signal (SV40pA), a chicken beta-globin polyadenylation signal, a bovine growth hormone polyadenylation signal (bGH), or a soluble neuropilin-1 polyadenylation signal. In some embodiments, the polyadenylation signal is an early SV40 polyadenylation signal (SV40pA) or a chicken beta-globin polyadenylation signal. Preferably, the polyadenylation signal is an early SV40 polyadenylation signal (SV40pA).

Suitably, the polyadenylation signal may comprise or consist of the nucleotide sequence shown as SEQ ID NO: 30, or a variant which is at least 70% identical to SEQ ID NO: 30. Suitably, the variant may be at least 75%, at least 80%, at least 85%, at least 90%, at least 95%, at least 96%, at least 97%, at least 98%, at least 99% identical to SEQ ID NO: 30.

*Exemplary SV40pA signal sequence (SEQ ID NO: 30):*

aacttgtttattgcagcttataatggttacaaataaagcaatagcatcacaaatttcacaaataaagc
atttttttcactgcattctagttgtggtttgtccaaactcatcaatgtatcttatcatgtctggatc

Suitably, the polyadenylation signal may comprise or consist of the nucleotide sequence shown as SEQ ID NO: 31, or a variant which is at least 70% identical to SEQ ID NO: 31. Suitably, the variant may be at least 75%, at least 80%, at least 85%, at least 90%, at least 95%, at least 96%, at least 97%, at least 98%, at least 99% identical to SEQ ID NO: 31.

*Exemplary bGH poly(A) signal sequence (SEQ ID NO: 31):*

ctgtgccttctagttgccagccatctgttgtttgcccctcccccgtgccttccttgaccctggaaggt
gccactcccactgtcctttcctaataaaatgaggaaattgcatcgcattgtctgagtaggtgtcattc
tattctggggggtggggtggggcaggacagcaagggggaggattgggaagacaatagcaggcatgctg
gggatgcggtgggctctatgg

Suitably, the polyadenylation signal may comprise or consist of the nucleotide sequence shown as SEQ ID NO: 32, or a variant which is at least 70% identical to SEQ ID NO: 32. Suitably, the variant may be at least 75%, at least 80%, at least 85%, at least 90%, at least 95%, at least 96%, at least 97%, at least 98%, at least 99% identical to SEQ ID NO: 32.

*Exemplary soluble neuropilin-1 polyadenylation signal (SEQ ID NO: 32):*
aaataaaatacgaaatg

Suitably, the polyadenylation signal may comprise or consist of the nucleotide sequence shown as SEQ ID NO: 42, or a variant which is at least 70% identical to SEQ ID NO: 42. Suitably, the variant may be at least 75%, at least 80%, at least 85%, at least 90%, at least 95%, at least 96%, at least 97%, at least 98%, at least 99% identical to SEQ ID NO: 42.

*Exemplary chicken beta-globin polyadenylation signal (SEQ ID NO: 42)*
caataaaagatctttattttcattagatctgtgtgttggttttttgtgtg

### Vectors

The present invention provides a vector comprising the polynucleotide of the present invention.

A vector is a tool that allows or facilitates the transfer of an entity from one environment to another. The four major types of vectors are plasmids, viral vectors, cosmids, and artificial chromosomes.

Preferably, the vector of the present invention is a viral vector. The vector of the invention is preferably an adeno-associated viral (AAV) vector, although it is contemplated that other viral vectors may be used.

The vector of the present invention may be in the form of a viral vector particle. Preferably, the viral vector of the present invention is in the form of an AAV vector particle.

Methods of preparing and modifying viral vectors and viral vector particles, such as those derived from AAV, are well known in the art. Suitable methods are described in Ayuso, E., et al., 2010. Current gene therapy, 10(6), pp.423-436, Merten, O.W., et al., 2016. Molecular Therapy-Methods & Clinical Development, 3, p.16017; and Nadeau, I. and Kamen, A., 2003. Biotechnology advances, 20(7-8), pp.475-489.

The vector of the present invention may be capable of transducing kidney cells. In some embodiments, the vector of the present invention is capable of specifically transducing kidney cells.

The vector of the present invention is preferably capable of transducing glomerular cells (e.g. podocytes). In some embodiments, the vector of the present invention is capable of specifically transducing glomerular cells (e.g. podocytes). The vector of the present invention is preferably capable of transducing podocytes. In some embodiments, the vector of the present invention is capable of specifically transducing podocytes.

### Adeno-associated viral (AAV) vectors

The vector of the present invention may be an adeno-associated viral (AAV) vector. The vector of the present invention may be in the form of an AAV vector particle.

### AAV genome

The AAV vector or AAV vector particle may comprise an AAV genome or a fragment or derivative thereof.

An AAV genome is a polynucleotide sequence, which may encode functions needed for production of an AAV particle. These functions include those operating in the replication and packaging cycle of AAV in a host cell, including encapsidation of the AAV genome into an AAV particle. Naturally occurring AAVs are replication-deficient and rely on the provision of helper functions in trans for completion of a replication and packaging cycle. Accordingly, the AAV genome of the AAV vector of the invention is typically replication-deficient.

The AAV genome may be in single-stranded form (ssAAV), either positive or negative-sense, or alternatively in double-stranded form (dsAAV). The use of a double-stranded form allows bypass of the DNA replication step in the target cell and so can accelerate transgene expression. The maximum packaging capacity of the single-stranded form is larger than the double-stranded form. Suitably, the AAV genome is in single-stranded form.

AAVs occurring in nature may be classified according to various biological systems. The AAV genome may be from any naturally derived serotype, isolate or clade of AAV.

AAV may be referred to in terms of their serotype. A serotype corresponds to a variant subspecies of AAV which, owing to its profile of expression of capsid surface antigens, has a distinctive reactivity which can be used to distinguish it from other variant subspecies. Typically, an AAV vector particle having a particular AAV serotype does not efficiently cross-react with neutralising antibodies specific for any other AAV serotype. AAV serotypes include AAV1, AAV2, AAV3, AAV4, AAV5, AAV6, AAV7, AAV8, AAV9, AAV10 and AAV11. In some embodiments, the AAV vector of the invention may be an AAV3B, LK03, AAV9, or AAV8 serotype. In some embodiments, the AAV vector of the invention may be an AAV3B, LK03, or AAV9 serotype.

AAV may also be referred to in terms of clades or clones. This refers to the phylogenetic relationship of naturally derived AAVs, and typically to a phylogenetic group of AAVs which can be traced back to a common ancestor, and includes all descendants thereof. Additionally, AAVs may be referred to in terms of a specific isolate, i.e. a genetic isolate of a specific AAV found in nature. The term genetic isolate describes a population of AAVs which has undergone limited genetic mixing with other naturally occurring AAVs, thereby defining a recognisably distinct population at a genetic level.

Typically, the AAV genome of a naturally derived serotype, isolate or clade of AAV comprises at least one inverted terminal repeat sequence (ITR). An ITR sequence acts in cis to provide a functional origin of replication and allows for integration and excision of the vector from the genome of a cell. ITRs may be the only sequences required in cis next to the therapeutic gene.

The AAV genome may also comprise packaging genes, such as rep and/or cap genes which encode packaging functions for an AAV particle. A promoter may be operably linked to each of the packaging genes. Specific examples of such promoters include the p5, p19 and p40 promoters. For example, the p5 and p19 promoters are generally used to express the rep gene, while the p40 promoter is generally used to express the cap gene. The rep gene encodes one or more of the proteins Rep78, Rep68, Rep52 and Rep40 or variants thereof. The cap gene encodes one or more capsid proteins such as VP1, VP2 and VP3 or variants thereof. These proteins make up the capsid of an AAV particle, which determines the AAV serotype. VP1, VP2, and VP3 may be produced by alternate mRNA splicing (Trempe, J.P. and Carter, B.J., 1988. Journal of virology, 62(9), pp.3356-3363). Thus, VP1, VP2 and VP3 may have identical sequences, but wherein VP2 is truncated at the N-terminus relative to VP1, and VP3 is truncated at the N-terminus relative to VP2.

The AAV genome may be the full genome of a naturally occurring AAV. For example, a vector comprising a full AAV genome may be used to prepare an AAV vector or vector particle.

Preferably, the AAV genome is derivatised for the purpose of administration to patients. Such derivatisation is standard in the art and the invention encompasses the use of any known derivative of an AAV genome, and derivatives which could be generated by applying techniques known in the art. The AAV genome may be a derivative of any naturally occurring AAV. Suitably, the AAV genome is a derivative of AAV1, AAV2, AAV3, AAV4, AAV5, AAV6, AAV7, AAV8, AAV9, AAV10, or AAV11. Suitably, the AAV genome is a derivative of AAV2.

Derivatives of an AAV genome include any truncated or modified forms of an AAV genome which allow for expression of a transgene from an AAV vector of the invention *in vivo.* Typically, it is possible to truncate the AAV genome significantly to include minimal viral sequence yet retain the above function. This is preferred for safety reasons to reduce the risk of recombination of the vector with wild-type virus, and also to avoid triggering a cellular immune response by the presence of viral gene proteins in the target cell.

Typically, a derivative will include at least one inverted terminal repeat sequence (ITR), preferably more than one ITR, such as two ITRs or more. One or more of the ITRs may be derived from AAV genomes having different serotypes, or may be a chimeric or mutant ITR. A preferred mutant ITR is one having a deletion of a trs (terminal resolution site). This deletion allows for continued replication of the genome to generate a single-stranded genome which contains both coding and complementary sequences, i.e. a self-complementary AAV (scAAV) genome. This allows for bypass of DNA replication in the target cell, and so enables accelerated transgene expression. However, the maximum packaging capacity of a scAAV is reduced. Suitably, the AAV genome is not a scAAV genome.

The AAV genome may comprise one or more ITR sequences from any naturally derived serotype, isolate or clade of AAV or a variant thereof. The AAV genome may comprise at least one, such as two, AAV1, AAV2, AAV3, AAV4, AAV5, AAV6, AAV7, AAV8, AAV9, AAV10, or AAV11 ITRs, or variants thereof. Suitably, the AAV genome may comprise at least one, such as two, AAV2 ITRs.

The inclusion of one or more ITRs is preferred to aid concatamer formation of the AAV vector in the nucleus of a host cell, for example following the conversion of single-stranded vector DNA into double-stranded DNA by the action of host cell DNA polymerases. The formation of such episomal concatamers protects the AAV vector during the life of the host cell, thereby allowing for prolonged expression of the transgene in vivo.

Suitably, ITR elements will be the only sequences retained from the native AAV genome in the derivative. A derivative will preferably not include the rep and/or cap genes of the native genome and any other sequences of the native genome. This is preferred for the reasons described above, and also to reduce the possibility of integration of the vector into the host cell genome. Additionally, reducing the size of the AAV genome allows for increased flexibility in incorporating other sequence elements (such as regulatory elements) within the vector in addition to the transgene.

The following portions could therefore be removed in a derivative of the invention: one inverted terminal repeat (ITR) sequence, the replication (rep) and capsid (cap) genes. However, derivatives may additionally include one or more rep and/or cap genes or other viral sequences of an AAV genome. Naturally occurring AAV integrates with a high frequency at a specific site on human chromosome 19, and shows a negligible frequency of random integration, such that retention of an integrative capacity in the AAV vector may be tolerated in a therapeutic setting.

The invention additionally encompasses the provision of sequences of an AAV genome in a different order and configuration to that of a native AAV genome. The invention also encompasses the replacement of one or more AAV sequences or genes with sequences from another virus or with chimeric genes composed of sequences from more than one virus. Such chimeric genes may be composed of sequences from two or more related viral proteins of different viral species.

### AAV serotype and capsid proteins

The AAV vector particle may be encapsidated by capsid proteins. The serotype may facilitate the transduction of glomerular cells (e.g. podocytes), for example specific transduction of glomerular cells (e.g. podocytes).

The AAV vector particle may be a kidney-specific vector particle. Preferably, the AAV vector particle is a glomerular-specific (e.g. podocyte-specific) vector particle. The AAV vector particle may be encapsidated by a glomerular-specific (e.g. podocyte-specific) capsid. The AAV vector particle may comprise a glomerular-specific (e.g. podocyte-specific) capsid protein.

Suitably, the AAV vector particles may be transcapsidated forms wherein an AAV genome or derivative having an ITR of one serotype is packaged in the capsid of a different serotype. The AAV vector particle also includes mosaic forms wherein a mixture of unmodified capsid proteins from two or more different serotypes makes up the viral capsid. The AAV vector particle also includes chemically modified forms bearing ligands adsorbed to the capsid surface. For example, such ligands may include antibodies for targeting a particular cell surface receptor.

Where a derivative comprises capsid proteins i.e. VP1, VP2 and/or VP3, the derivative may be a chimeric, shuffled or capsid-modified derivative of one or more naturally occurring AAVs. In particular, the invention encompasses the provision of capsid protein sequences from different serotypes, clades, clones, or isolates of AAV within the same vector (i.e. a pseudotyped vector). The AAV vector may be in the form of a pseudotyped AAV vector particle.

Chimeric, shuffled or capsid-modified derivatives will be typically selected to provide one or more desired functionalities for the AAV vector. Thus, these derivatives may display increased efficiency of gene delivery, decreased immunogenicity (humoral or cellular), an altered tropism range and/or improved targeting of podocytes compared to an AAV vector comprising a naturally occurring AAV genome. Increased efficiency of gene delivery may be effected by improved receptor or co-receptor binding at the cell surface, improved internalisation, improved trafficking within the cell and into the nucleus, improved uncoating of the viral particle and improved conversion of a single-stranded genome to double-stranded form. Increased efficiency may also relate to an altered tropism range or targeting of podocytes, such that the vector dose is not diluted by administration to tissues where it is not needed.

Chimeric capsid proteins include those generated by recombination between two or more capsid coding sequences of naturally occurring AAV serotypes. This may be performed for example by a marker rescue approach in which non-infectious capsid sequences of one serotype are co-transfected with capsid sequences of a different serotype, and directed selection is used to select for capsid sequences having desired properties. The capsid sequences of the different serotypes can be altered by homologous recombination within the cell to produce novel chimeric capsid proteins.

Chimeric capsid proteins also include those generated by engineering of capsid protein sequences to transfer specific capsid protein domains, surface loops or specific amino acid residues between two or more capsid proteins, for example between two or more capsid proteins of different serotypes.

Shuffled or chimeric capsid proteins may also be generated by DNA shuffling or by error-prone PCR. Hybrid AAV capsid genes can be created by randomly fragmenting the sequences of related AAV genes e.g. those encoding capsid proteins of multiple different serotypes and then subsequently reassembling the fragments in a self-priming polymerase reaction, which may also cause crossovers in regions of sequence homology. A library of hybrid AAV genes created in this way by shuffling the capsid genes of several serotypes can be screened to identify viral clones having a desired functionality. Similarly, error prone PCR may be used to randomly mutate AAV capsid genes to create a diverse library of variants which may then be selected for a desired property.

The sequences of the capsid genes may also be genetically modified to introduce specific deletions, substitutions or insertions with respect to the native wild-type sequence. In particular, capsid genes may be modified by the insertion of a sequence of an unrelated protein or peptide within an open reading frame of a capsid coding sequence, or at the N- and/or C-terminus of a capsid coding sequence. The unrelated protein or peptide may advantageously be one which acts as a ligand for a particular cell type, thereby conferring improved binding to a target cell or improving the specificity of targeting of the vector to a particular cell population. The unrelated protein may also be one which assists purification of the viral particle as part of the production process, i.e. an epitope or affinity tag. The site of insertion will typically be selected so as not to interfere with other functions of the viral particle e.g. internalisation, trafficking of the viral particle.

The capsid protein may be an artificial or mutant capsid protein. The term "artificial capsid" as used herein means that the capsid particle comprises an amino acid sequence which does not occur in nature or which comprises an amino acid sequence which has been engineered (e.g. modified) from a naturally occurring capsid amino acid sequence. In other words the artificial capsid protein comprises a mutation or a variation in the amino acid sequence compared to the sequence of the parent capsid from which it is derived where the artificial capsid amino acid sequence and the parent capsid amino acid sequences are aligned.

The capsid protein may comprise a mutation or modification relative to the wild type capsid protein which improves the ability to transduce podocytes relative to an unmodified or wild type viral particle. Improved ability to transduce podocytes may be measured for example by measuring the expression of a transgene, e.g. GFP, carried by the AAV vector particle, wherein expression of the transgene in podocytes correlates with the ability of the AAV vector particle to transduce podocytes.

The AAV vector particle may be an AAV3B, LK03, AAV9, or AAV8 vector particle. The present inventors have shown that AAV vector particles with AAV3B, LK03, AAV9 and AAV8 serotypes can transduce podocytes. Preferably, the AAV vector particle is an AAV3B vector particle or an LK03 vector particle. More preferably, the AAV vector particle is an AAV3B vector particle.

The AAV vector particle may comprise an AAV3B, LK03, AAV9, or AAV8 capsid protein. Preferably, the AAV vector particle comprises an AAV3B capsid protein or an LK03 capsid protein. More preferably, the AAV vector particle comprises an AAV3B capsid protein.

The AAV vector particle may comprise AAV3B, LK03, AAV9, or AAV8 capsid proteins VP1, VP2 and VP3. Preferably, the AAV vector particle comprises AAV3B or LK03 capsid proteins VP1, VP2 and VP3. More preferably, the AAV vector particle comprises AAV3B capsid proteins VP1, VP2 and VP3.

The AAV vector particle may comprise one or more AAV2 ITR sequences and AAV3B capsid proteins, LK03 capsid proteins, AAV9 capsid proteins, or AAV8 capsid proteins. Preferably, the AAV vector particle comprises one or more AAV2 ITR sequences and AAV3B or LK03 capsid proteins. More preferably, the AAV vector particle comprises one or more AAV2 ITR sequences and AAV3B capsid proteins.

The AAV vector particle may have an AAV2 genome and AAV3B capsid proteins (AAV2/3B), an AAV2 genome and LK03 capsid proteins, an AAV2 genome and AAV9 capsid proteins (AAV2/9), or an AAV2 genome and AAV8 capsid proteins (AAV2/8). Preferably, the AAV vector particle comprises an AAV2 genome and AAV3B or LK03 capsid proteins. More preferably, the AAV vector particle comprises an AAV2 genome and AAV3B capsid proteins.

The nomenclature AAVX/Y may denote a pseudotyped AAV, for example where the ITR sequences are from AAVX and flank a cassette harbouring a payload which is encapsidated into serotype AAVY (i.e. with AAVY capsid proteins).

### AAV3B serotype

The AAV vector particle may comprise an AAV3B capsid protein. Suitably, the AAV vector particle may be encapsidated by AAV3B capsid proteins.

Two distinct AAV3 isolates (AAV3A and AAV3B) have been cloned. In comparison with vectors based on other AAV serotypes, it is thought that AAV3 vectors inefficiently transduce most cell types. However, AAV3B may efficiently transduce podocytes. AA3B has been described in Rutledge, E.A., et al., 1998. Journal of virology, 72(1), pp.309-319.

The AAV vector particle may comprise an AAV3B VP1 capsid protein, an AAV3B VP2 capsid protein, and/or an AAV3B VP3 capsid protein. Suitably, the AAV vector particle may be encapsidated by AAV3B VP1 capsid proteins, AAV3B VP2 capsid proteins, and/or AAV3B VP3 capsid proteins. Suitably, the AAV vector particle may be encapsidated by AAV3B VP1, VP2, and VP3 capsid proteins.

Suitably, the AAV3B VP1 capsid protein may comprise or consist of the amino acid sequence shown as SEQ ID NO: 33, or a variant which is at least 90% identical to SEQ ID NO: 33.

*Exemplary AAV3B VP1 capsid protein (SEQ ID NO: 33):*

MAADGYLPDWLEDNLSEGIREWWALKPGVPQPKANQQHQDNRRGLVLPGYKYLGPGNGLDKGEPVNEA
DAAALEHDKAYDQQLKAGDNPYLKYNHADAEFQERLQEDTSFGGNLGRAVFQAKKRILEPLGLVEEAA
KTAPGKKRPVDQSPQEPDSSSGVGKSGKQPARKRLNFGQTGDSESVPDPQPLGEPPAAPTSLGSNTMA
SGGGAPMADNNEGADGVGNSSGNWHCDSQWLGDRVITTSTRTWALPTYNNHLYKQISSQSGASNDNHY
FGYSTPWGYFDFNRFHCHFSPRDWQRLINNNWGFRPKKLSFKLFNIQVKEVTQNDGTTTIANNLTSTV
QVFTDSEYQLPYVLGSAHQGCLPPFPADVFMVPQYGYLTLNNGSQAVGRSSFYCLEYFPSQMLRTGNN
FQFSYTFEDVPFHSSYAHSQSLDRLMNPLIDQYLYYLNRTQGTTSGTTNQSRLLFSQAGPQSMSLQAR
NWLPGPCYRQQRLSKTANDNNNSNFPWTAASKYHLNGRDSLVNPGPAMASHKDDEEKFFPMHGNLIFG
KEGTTASNAELDNVMITDEEEIRTTNPVATEQYGTVANNLQSSNTAPTTRTVNDQGALPGMVWQDRDV
YLQGPIWAKIPHTDGHFHPSPLMGGFGLKHPPPQIMIKNTPVPANPPTTFSPAKFASFITQYSTGQVS
VEIEWELQKENSKRWNPEIQYTSNYNKSVNVDFTVDTNGVYSEPRPIGTRYLTRN

Suitably, the variant may be at least 95%, at least 96%, at least 97%, at least 98% or at least 99% identical to SEQ ID NO: 33.

Suitably, the AAV3B VP2 and VP3 capsid proteins may be N-terminal truncations of SEQ ID NO: 33, or N-terminal truncations of a variant which is at least 90% identical, at least 95%, at least 96%, at least 97%, at least 98% or at least 99% identical to SEQ ID NO: 33.

### LK03 serotype

The AAV vector particle may comprise an LK03 capsid protein. Suitably, the AAV vector particle may be encapsidated by LK03 capsid proteins.

The AAV-LK03 cap sequence consists of fragments from seven different wild-type serotypes (AAV1, 2, 3B, 4, 6, 8, 9) and is described in Lisowski, L., et al., 2014. Nature, 506(7488), pp.382-386. The present inventors have demonstrated that AAV-LK03 vectors can achieve high transduction of close to 100% in human podocytes *in vitro.*

The AAV vector particle may comprise an LK03 VP1 capsid protein, an LK03 VP2 capsid protein, and/or an LK03 VP3 capsid protein. Suitably, the AAV vector particle may be encapsidated by LK03 VP1 capsid proteins, LK03 VP2 capsid proteins, and/or LK03 VP3 capsid proteins. Suitably, the AAV vector particle may be encapsidated by LK03 VP1, VP2, and VP3 capsid proteins.

Suitably, the LK03 VP1 capsid protein may comprise or consist of the amino acid sequence shown as SEQ ID NO: 34, or a variant which is at least 90% identical to SEQ ID NO: 34.

*Exemplary LK03 VP1 capsid protein (SEQ ID NO: 34):*

MAADGYLPDWLEDNLSEGIREWWALQPGAPKPKANQQHQDNARGLVLPGYKYLGPGNGLDKGEPVNAA
DAAALEHDKAYDQQLKAGDNPYLKYNHADAEFQERLKEDTSFGGNLGRAVFQAKKRLLEPLGLVEEAA
KTAPGKKRPVDQSPQEPDSSSGVGKSGKQPARKRLNFGQTGDSESVPDPQPLGEPPAAPTSLGSNTMA
SGGGAPMADNNEGADGVGNSSGNWHCDSQWLGDRVITTSTRTWALPTYNNHLYKQISSQSGASNDNHY
FGYSTPWGYFDFNRFHCHFSPRDWQRLINNNWGFRPKKLSFKLFNIQVKEVTQNDGTTTIANNLTSTV
QVFTDSEYQLPYVLGSAHQGCLPPFPADVFMVPQYGYLTLNNGSQAVGRSSFYCLEYFPSQMLRTGNN
FQFSYTFEDVPFHSSYAHSQSLDRLMNPLIDQYLYYLNRTQGTTSGTTNQSRLLFSQAGPQSMSLQAR
NWLPGPCYRQQRLSKTANDNNNSNFPWTAASKYHLNGRDSLVNPGPAMASHKDDEEKFFPMHGNLIFG
KEGTTASNAELDNVMITDEEEIRTTNPVATEQYGTVANNLQSSNTAPTTRTVNDQGALPGMVWQDRDV
YLQGPIWAKIPHTDGHFHPSPLMGGFGLKHPPPQIMIKNTPVPANPPTTFSPAKFASFITQYSTGQVS
VEIEWELQKENSKRWNPEIQYTSNYNKSVNVDFTVDTNGVYSEPRPIGTRYLTRPL

Suitably, the variant may be at least 95%, at least 96%, at least 97%, at least 98% or at least 99% identical to SEQ ID NO: 34.

Suitably, the LK03 VP2 and VP3 capsid proteins may be N-terminal truncations of SEQ ID NO: 34, or N-terminal truncations of a variant which is at least 90% identical, at least 95%, at least 96%, at least 97%, at least 98% or at least 99% identical to SEQ ID NO: 34.

### AA V9 serotype

The AAV vector particle may comprise an AAV9 capsid protein. Suitably, the AAV vector particle may be encapsidated by AAV9 capsid proteins.

The present inventors have demonstrated that AAV9 vectors can achieve high transduction in human podocytes *in vitro.*

The AAV vector particle may comprise an AAV9 VP1 capsid protein, an AAV9 VP2 capsid protein, and/or an AAV9 VP3 capsid protein. Suitably, the AAV vector particle may be encapsidated by AAV9 VP1 capsid proteins, AAV9 VP2 capsid proteins, and/or AAV9 VP3 capsid proteins. Suitably, the AAV vector particle may be encapsidated by AAV9 VP1, VP2, and VP3 capsid proteins.

Suitably, the AAV9 VP1 capsid protein may comprise or consist of the amino acid sequence shown as SEQ ID NO: 35, or a variant which is at least 90% identical to SEQ ID NO: 35.

*Exemplary AAV9 VP1 capsid protein (SEQ ID NO: 35):*

MAADGYLPDWLEDNLSEGIREWWALKPGAPQPKANQQHQDNARGLVLPGYKYLGPGNGLDKGEPVNAA
DAAALEHDKAYDQQLKAGDNPYLKYNHADAEFQERLKEDTSFGGNLGRAVFQAKKRLLEPLGLVEEAA
KTAPGKKRPVEQSPQEPDSSAGIGKSGAQPAKKRLNFGQTGDTESVPDPQPIGEPPAAPSGVGSLTMA
SGGGAPVADNNEGADGVGSSSGNWHCDSQWLGDRVITTSTRTWALPTYNNHLYKQISNSTSGGSSNDN
AYFGYSTPWGYFDFNRFHCHFSPRDWQRLINNNWGFRPKRLNFKLFNIQVKEVTDNNGVKTIANNLTS
TVQVFTDSDYQLPYVLGSAHEGCLPPFPADVFMIPQYGYLTLNDGSQAVGRSSFYCLEYFPSQMLRTG
NNFQFSYEFENVPFHSSYAHSQSLDRLMNPLIDQYLYYLSKTINGSGQNQQTLKFSVAGPSNMAVQGR
NYIPGPSYRQQRVSTTVTQNNNSEFAWPGASSWALNGRNSLMNPGPAMASHKEGEDRFFPLSGSLIFG
KQGTGRDNVDADKVMITNEEEIKTTNPVATESYGQVATNHQSAQAQAQTGWVQNQGILPGMVWQDRDV
YLQGPIWAKIPHTDGNFHPSPLMGGFGMKHPPPQILIKNTPVPADPPTAFNKDKLNSFITQYSTGQVS
VEIEWELQKENSKRWNPEIQYTSNYYKSNNVEFAVNTEGVYSEPRPIGTRYLTRNL

Suitably, the variant may be at least 95%, at least 96%, at least 97%, at least 98% or at least 99% identical to SEQ ID NO: 35.

Suitably, the AAV9 VP2 and VP3 capsid proteins may be N-terminal truncations of SEQ ID NO: 35, or N-terminal truncations of a variant which is at least 90% identical, at least 95%, at least 96%, at least 97%, at least 98% or at least 99% identical to SEQ ID NO: 35.

### Other viral vectors

### Retroviral and lentiviral vectors

The vector of the present invention may be a retroviral vector or a lentiviral vector. The vector of the present invention may be a retroviral vector particle or a lentiviral vector particle.

A retroviral vector may be derived from or may be derivable from any suitable retrovirus. A large number of different retroviruses have been identified. Examples include murine leukaemia virus (MLV), human T-cell leukaemia virus (HTLV), mouse mammary tumour virus (MMTV), Rous sarcoma virus (RSV), Fujinami sarcoma virus (FuSV), Moloney murine leukaemia virus (Mo-MLV), FBR murine osteosarcoma virus (FBR MSV), Moloney murine sarcoma virus (Mo-MSV), Abelson murine leukaemia virus (A-MLV), avian myelocytomatosis virus-29 (MC29) and avian erythroblastosis virus (AEV).

Retroviruses may be broadly divided into two categories, "simple" and "complex". Retroviruses may be even further divided into seven groups. Five of these groups represent retroviruses with oncogenic potential. The remaining two groups are the lentiviruses and the spumaviruses.

The basic structure of retrovirus and lentivirus genomes share many common features such as a 5' LTR and a 3' LTR. Between or within these are located a packaging signal to enable the genome to be packaged, a primer binding site, integration sites to enable integration into a host cell genome, and gag, pol and env genes encoding the packaging components - these are polypeptides required for the assembly of viral particles. Lentiviruses have additional features, such as rev and RRE sequences in HIV, which enable the efficient export of RNA transcripts of the integrated provirus from the nucleus to the cytoplasm of an infected target cell.

In the provirus, these genes are flanked at both ends by regions called long terminal repeats (LTRs). The LTRs are responsible for proviral integration and transcription. LTRs also serve as enhancer-promoter sequences and can control the expression of the viral genes.

The LTRs themselves are identical sequences that can be divided into three elements: U3, R and U5. U3 is derived from the sequence unique to the 3' end of the RNA. R is derived from a sequence repeated at both ends of the RNA. U5 is derived from the sequence unique to the 5' end of the RNA. The sizes of the three elements can vary considerably among different retroviruses.

In a defective retroviral vector genome gag, pol and env may be absent or not functional.

In a typical retroviral vector, at least part of one or more protein coding regions essential for replication may be removed from the virus. This makes the viral vector replication-defective. Portions of the viral genome may also be replaced by a library encoding candidate modulating moieties operably linked to a regulatory control region and a reporter moiety in the vector genome in order to generate a vector comprising candidate modulating moieties which is capable of transducing a target host cell and/or integrating its genome into a host genome.

Lentivirus vectors are part of the larger group of retroviral vectors. In brief, lentiviruses can be divided into primate and non-primate groups. Examples of primate lentiviruses include but are not limited to human immunodeficiency virus (HIV), the causative agent of human acquired immunodeficiency syndrome (AIDS); and simian immunodeficiency virus (SIV). Examples of non-primate lentiviruses include the prototype "slow virus" visna/maedi virus (VMV), as well as the related caprine arthritis-encephalitis virus (CAEV), equine infectious anaemia virus (EIAV), and the more recently described feline immunodeficiency virus (FIV) and bovine immunodeficiency virus (BIV).

The lentivirus family differs from retroviruses in that lentiviruses have the capability to infect both dividing and non-dividing cells. In contrast, other retroviruses, such as MLV, are unable to infect non-dividing or slowly dividing cells such as those that make up, for example, muscle, brain, lung and liver tissue.

A lentiviral vector, as used herein, is a vector which comprises at least one component part derivable from a lentivirus. Preferably, that component part is involved in the biological mechanisms by which the vector infects cells, expresses genes or is replicated.

The lentiviral vector may be a "primate" vector. The lentiviral vector may be a "non-primate" vector (i.e. derived from a virus which does not primarily infect primates, especially humans). Examples of non-primate lentiviruses may be any member of the family of lentiviridae which does not naturally infect a primate.

As examples of lentivirus-based vectors, HIV-1- and HIV-2-based vectors are described below.

The HIV-1 vector contains cis-acting elements that are also found in simple retroviruses. It has been shown that sequences that extend into the gag open reading frame are important for packaging of HIV-1. Therefore, HIV-1 vectors often contain the relevant portion of gag in which the translational initiation codon has been mutated. In addition, most HIV-1 vectors also contain a portion of the env gene that includes the RRE. Rev binds to RRE, which permits the transport of full-length or singly spliced mRNAs from the nucleus to the cytoplasm. In the absence of Rev and/or RRE, full-length HIV-1 RNAs accumulate in the nucleus. Alternatively, a constitutive transport element from certain simple retroviruses such as Mason-Pfizer monkey virus can be used to relieve the requirement for Rev and RRE. Efficient transcription from the HIV-1 LTR promoter requires the viral protein Tat.

Most HIV-2-based vectors are structurally very similar to HIV-1 vectors. Similar to HIV-1-based vectors, HIV-2 vectors also require RRE for efficient transport of the full-length or singly spliced viral RNAs.

Preferably, the viral vector used in the present invention has a minimal viral genome.

By "minimal viral genome" it is to be understood that the viral vector has been manipulated so as to remove the non-essential elements and to retain the essential elements in order to provide the required functionality to infect, transduce and deliver a nucleotide sequence of interest to a target host cell. Further details of this strategy can be found in WO 1998/017815.

Preferably, the plasmid vector used to produce the viral genome within a host cell/packaging cell will have sufficient lentiviral genetic information to allow packaging of an RNA genome, in the presence of packaging components, into a viral particle which is capable of infecting a target cell, but is incapable of independent replication to produce infectious viral particles within the final target cell. Preferably, the vector lacks a functional gag-pol and/or env gene and/or other genes essential for replication.

However, the plasmid vector used to produce the viral genome within a host cell/packaging cell will also include transcriptional regulatory control sequences operably linked to the lentiviral genome to direct transcription of the genome in a host cell/packaging cell. These regulatory sequences may be the natural sequences associated with the transcribed viral sequence (i.e. the 5' U3 region), or they may be a heterologous promoter, such as another viral promoter (e.g. the CMV promoter).

The vectors may be self-inactivating (SIN) vectors in which the viral enhancer and promoter sequences have been deleted. SIN vectors can be generated and transduce non-dividing cells in vivo with an efficacy similar to that of wild-type vectors. The transcriptional inactivation of the long terminal repeat (LTR) in the SIN provirus should prevent mobilisation by replication-competent virus. This should also enable the regulated expression of genes from internal promoters by eliminating any cis-acting effects of the LTR.

The vectors may be integration-defective. Integration defective lentiviral vectors (IDLVs) can be produced, for example, either by packaging the vector with catalytically inactive integrase (such as an HIV integrase bearing the D64V mutation in the catalytic site) or by modifying or deleting essential att sequences from the vector LTR, or by a combination of the above.

### Adenoviral vectors

The vector of the present invention may be an adenoviral vector. The vector of the present invention may be an adenoviral vector particle.

The adenovirus is a double-stranded, linear DNA virus that does not go through an RNA intermediate. There are over 50 different human serotypes of adenovirus divided into 6 subgroups based on the genetic sequence homology. The natural targets of adenovirus are the respiratory and gastrointestinal epithelia, generally giving rise to only mild symptoms. Serotypes 2 and 5 (with 95% sequence homology) are most commonly used in adenoviral vector systems and are normally associated with upper respiratory tract infections in the young.

Adenoviruses have been used as vectors for gene therapy and for expression of heterologous genes. The large (36 kb) genome can accommodate up to 8 kb of foreign insert DNA and is able to replicate efficiently in complementing cell lines to produce very high titres of up to 10¹². Adenovirus is thus one of the best systems to study the expression of genes in primary non-replicative cells.

The expression of viral or foreign genes from the adenovirus genome does not require a replicating cell. Adenoviral vectors enter cells by receptor mediated endocytosis. Once inside the cell, adenovirus vectors rarely integrate into the host chromosome. Instead, they function episomally (independently from the host genome) as a linear genome in the host nucleus. Hence, the use of recombinant adenovirus alleviates the problems associated with random integration into the host genome.

### Herpes simplex viral vector

The vector of the present invention may be a herpes simplex viral vector. The vector of the present invention may be a herpes simplex viral vector particle.

Herpes simplex virus (HSV) is a neurotropic DNA virus with favorable properties as a gene delivery vector. HSV is highly infectious, so HSV vectors are efficient vehicles for the delivery of exogenous genetic material to cells. Viral replication is readily disrupted by null mutations in immediate early genes that in vitro can be complemented in trans enabling straightforward production of high-titre pure preparations of non-pathogenic vector. The genome is large (152 Kb) and many of the viral genes are dispensable for replication in vitro, allowing their replacement with large or multiple transgenes. Latent infection with wild-type virus results in episomal viral persistence in sensory neuronal nuclei for the duration of the host lifetime. The vectors are non-pathogenic, unable to reactivate and persist long-term. The latency active promoter complex can be exploited in vector design to achieve long-term stable transgene expression in the nervous system. HSV vectors transduce a broad range of tissues because of the wide expression pattern of the cellular receptors recognized by the virus. Increasing understanding of the processes involved in cellular entry has allowed targeting the tropism of HSV vectors.

### Other viral vectors

Other suitable viral vectors include those described in Lundstrom, K., 2018. Diseases, 6(2), p.42.

The vector of the present invention may be an alphaviral vector. The vector of the present invention may be an alphaviral vector particle. The vector of the present invention may be a flaviviral vector. The vector of the present invention may be a flaviviral vector particle.

Self-amplifying ssRNA viruses comprise of alphaviruses (e.g. Semliki Forest virus, Sindbis virus, Venezuelan equine encephalitis virus, and M1) and flaviviruses (e.g. Kunjin virus, West Nile virus, and Dengue virus) possessing a genome of positive polarity. Alphaviruses have been mainly applied in preclinical gene therapy studies for cancer treatment. Alphavirus vectors can be delivered in the form of naked RNA, layered plasmid DNA vectors and recombinant replication-deficient or -proficient particles.

The vector of the present invention may be a rhabdoviral vector. The vector of the present invention may be a rhabdoviral vector particle. The vector of the present invention may be a measles viral vector. The vector of the present invention may be a measles viral vector particle.

Rhabdoviruses (e.g. rabies and vesicular stomatitis virus) and measles viruses carry negative strand genomes. Among rhabdoviruses, recombinant vesicular stomatitis virus (VSV) has been applied for preclinical gene therapy studies. Measles viruses (e.g. MV-Edm) have found a number of gene therapy applications.

The vector of the present invention may be a Newcastle disease viral vector. The vector of the present invention may be a Newcastle disease viral vector particle.

The ssRNA paramyxovirus Newcastle disease virus (NDV) replicates specifically in tumour cells and has therefore been frequently applied for cancer gene therapy.

The vector of the present invention may be a poxviral vector. The vector of the present invention may be a poxviral vector particle.

The characteristic feature of poxviruses is their dsDNA genome, which can generously accommodate more than 30 kb of foreign DNA. Poxviruses have found several applications as gene therapy vectors. For instance, vaccinia virus vectors have demonstrated potential for treatment of cancer. Vaccinia virus is large enveloped poxvirus that has an approximately 190 kb linear, double-stranded DNA genome. Vaccinia virus can accommodate up to approximately 25 kb of foreign DNA, which also makes it useful for the delivery of large genes. A number of attenuated vaccinia virus strains are known in the art that are suitable for gene therapy applications, for example the MVA and NYVAC strains.

The vector of the present invention may be a picornaviral vector. The vector of the present invention may be a picornaviral vector particle.

Picornoviruses are non-enveloped ssRNA viruses. Coxsackieviruses belonging to Picornaviridae, have been applied as oncolytic vectors.

### Variants, derivatives, analogues, homologues and fragments

In the context of the invention, a "variant" of any given sequence is a sequence in which the specific sequence of residues (whether amino acid or nucleic acid residues) has been modified in such a manner that the polypeptide or polynucleotide in question retains at least one of its endogenous functions. A variant sequence can be obtained by addition, deletion, substitution, modification, replacement and/or variation of at least one residue present in the naturally occurring polypeptide or polynucleotide. For example, a variant promoter sequence retains at least some level of the activity and specificity of the promoter sequence from which it is obtained.

The term "derivative" as used herein in relation to proteins or polypeptides includes any substitution of, variation of, modification of, replacement of, deletion of and/or addition of one (or more) amino acid residues from or to the sequence, providing that the resultant protein or polypeptide retains at least one of its endogenous functions.

Typically, amino acid substitutions may be made, for example from 1, 2 or 3, to 10 or 20 substitutions, provided that the modified sequence retains the required activity or ability. Amino acid substitutions may include the use of non-naturally occurring analogues.

Proteins used in the invention may also have deletions, insertions or substitutions of amino acid residues which produce a silent change and result in a functionally equivalent protein. Deliberate amino acid substitutions may be made on the basis of similarity in polarity, charge, solubility, hydrophobicity, hydrophilicity and/or the amphipathic nature of the residues as long as the endogenous function is retained. For example, negatively charged amino acids include aspartic acid and glutamic acid; positively charged amino acids include lysine and arginine; and amino acids with uncharged polar head groups having similar hydrophilicity values include asparagine, glutamine, serine, threonine and tyrosine.

Conservative substitutions may be made, for example according to the table below. Amino acids in the same block in the second column and preferably in the same line in the third column may be substituted for each other:

| | | |
|---|---|---|
| ALIPHATIC | Non-polar | G A P |
| | | I L V |
| | Polar - uncharged | C S T M |
| | | N Q |
| | Polar - charged | D E |
| | | K R H |
| AROMATIC | | F W Y |

The term "homologue" as used herein means a variant having a certain homology with the wild type amino acid sequence or the wild type nucleotide sequence. The term "homology" can be equated with "identity".

In the present context, a homologous sequence is taken to include an amino acid sequence which may be at least 50%, 55%, 65%, 75%, 85% or 90% identical, preferably at least 95%, 96% or 97% or 98% or 99% identical to the subject sequence. Typically, the homologues will comprise the same active sites etc. as the subject amino acid sequence. Although homology can also be considered in terms of similarity (i.e. amino acid residues having similar chemical properties/functions), in the context of the present invention it is preferred to express homology in terms of sequence identity.

In the present context, a homologous sequence is taken to include a nucleotide sequence which may be at least 50%, 55%, 65%, 75%, 85% or 90% identical, preferably at least 95%, 96% or 97% or 98% or 99% identical to the subject sequence. Although homology can also be considered in terms of similarity, in the context of the present invention it is preferred to express homology in terms of sequence identity.

Preferably, reference to a sequence which has a percent identity to any one of the SEQ ID NOs detailed herein refers to a sequence which has the stated percent identity over the entire length of the SEQ ID NO referred to.

Homology comparisons can be conducted by eye, or more usually, with the aid of readily available sequence comparison programs. These commercially available computer programs can calculate percent homology or identity between two or more sequences.

Percent homology may be calculated over contiguous sequences, i.e. one sequence is aligned with the other sequence and each amino acid or nucleotide in one sequence is directly compared with the corresponding amino acid or nucleotide in the other sequence, one residue at a time. This is called an "ungapped" alignment. Typically, such ungapped alignments are performed only over a relatively short number of residues.

Although this is a very simple and consistent method, it fails to take into consideration that, for example, in an otherwise identical pair of sequences, one insertion or deletion in the amino acid or nucleotide sequence may cause the following residues or codons to be put out of alignment, thus potentially resulting in a large reduction in percent homology when a global alignment is performed. Consequently, most sequence comparison methods are designed to produce optimal alignments that take into consideration possible insertions and deletions without penalising unduly the overall homology score. This is achieved by inserting "gaps" in the sequence alignment to try to maximise local homology.

However, these more complex methods assign "gap penalties" to each gap that occurs in the alignment so that, for the same number of identical amino acids or nucleotides, a sequence alignment with as few gaps as possible, reflecting higher relatedness between the two compared sequences, will achieve a higher score than one with many gaps. "Affine gap costs" are typically used that charge a relatively high cost for the existence of a gap and a smaller penalty for each subsequent residue in the gap. This is the most commonly used gap scoring system. High gap penalties will of course produce optimised alignments with fewer gaps. Most alignment programs allow the gap penalties to be modified. However, it is preferred to use the default values when using such software for sequence comparisons. For example when using the GCG Wisconsin Bestfit package the default gap penalty for amino acid sequences is -12 for a gap and -4 for each extension.

Calculation of maximum percent homology therefore firstly requires the production of an optimal alignment, taking into consideration gap penalties. A suitable computer program for carrying out such an alignment is the GCG Wisconsin Bestfit package (University of Wisconsin, USA; Devereux et al. (1984) Nucleic Acids Research 12: 387). Examples of other software that can perform sequence comparisons include, but are not limited to, the BLAST package (see Ausubel et al. (1999) ibid - Ch. 18), FASTA (Atschul et al. (1990) J. Mol. Biol. 403-410), EMBOSS Needle (Madeira, F., et al., 2019. Nucleic acids research, 47(W1), pp.W636-W641) and the GENEWORKS suite of comparison tools. Both BLAST and FASTA are available for offline and online searching (see Ausubel et al. (1999) ibid, pages 7-58 to 7-60). However, for some applications, it is preferred to use the GCG Bestfit program. Another tool, BLAST 2 Sequences, is also available for comparing protein and nucleotide sequences (FEMS Microbiol. Lett. (1999) 174(2):247-50; FEMS Microbiol. Lett. (1999) 177(1):187-8).

Although the final percent homology can be measured in terms of identity, the alignment process itself is typically not based on an all-or-nothing pair comparison. Instead, a scaled similarity score matrix is generally used that assigns scores to each pairwise comparison based on chemical similarity or evolutionary distance. An example of such a matrix commonly used is the BLOSUM62 matrix (the default matrix for the BLAST suite of programs). GCG Wisconsin programs generally use either the public default values or a custom symbol comparison table if supplied (see the user manual for further details). For some applications, it is preferred to use the public default values for the GCG package, or in the case of other software, the default matrix, such as BLOSUM62.

Once the software has produced an optimal alignment, it is possible to calculate percent homology, preferably percent sequence identity. The software typically does this as part of the sequence comparison and generates a numerical result. The percent sequence identity may be calculated as the number of identical residues as a percentage of the total residues in the SEQ ID NO referred to.

"Fragments" are also variants and the term typically refers to a selected region of the polypeptide or polynucleotide that is of interest either functionally or, for example, in an assay. "Fragment" thus refers to an amino acid or nucleic acid sequence that is a portion of a full-length polypeptide or polynucleotide.

Such variants, derivatives, homologues and fragments may be prepared using standard recombinant DNA techniques such as site-directed mutagenesis. Where insertions are to be made, synthetic DNA encoding the insertion together with 5' and 3' flanking regions corresponding to the naturally-occurring sequence either side of the insertion site may be made. The flanking regions will contain convenient restriction sites corresponding to sites in the naturally-occurring sequence so that the sequence may be cut with the appropriate enzyme(s) and the synthetic DNA ligated into the cut. The DNA is then expressed in accordance with the invention to make the encoded protein. These methods are only illustrative of the numerous standard techniques known in the art for manipulation of DNA sequences and other known techniques may also be used.

### Cells

In one aspect, the present invention provides a cell comprising the polynucleotide or the vector of the invention. The cell may be an isolated cell. The cell may be a human cell, suitably an isolated human cell.

Vectors comprising polynucleotides used in the invention may be introduced into cells using a variety of techniques known in the art, such as transfection, transduction and transformation. Suitably, the vector of the present invention is introduced into the cell by transfection or transduction.

The cell may be any cell type known in the prior art.

Suitably, the cell may be a producer cell. The term "producer cell" includes a cell that produces viral particles, after transient transfection, stable transfection or vector transduction of all the elements necessary to produce the viral particles or any cell engineered to stably comprise the elements necessary to produce the viral particles. Suitable producer cells will be well known to those of skill in the art. Suitable producer cell lines include HEK 293 (e.g. HEK 293T), HeLa, and A549 cell lines.

Suitably, the cell may be a packaging cell. The term "packaging cell" includes a cell which contains some or all of the elements necessary for packaging an infectious recombinant virus. The packaging cell may lack a recombinant viral vector genome. Typically, such packaging cells contain one or more vectors which are capable of expressing viral structural proteins. Cells comprising only some of the elements required for the production of enveloped viral particles are useful as intermediate reagents in the generation of viral particle producer cell lines, through subsequent steps of transient transfection, transduction or stable integration of each additional required element. These intermediate reagents are encompassed by the term "packaging cell". Suitable packaging cells will be well known to those of skill in the art.

Suitably, the cell may be a kidney cell or glomerular cell, for example a podocyte. Suitably, the cell may be an immortalized kidney cell or glomerular cell, for example an immortalized podocyte. Suitable podocyte cell lines will be well known to those of skill in the art, for example CIHP-1. Methods to generate immortalized podocytes will be well known to those of skill in the art. Suitable methods are described in Ni, L., et al., 2012. Nephrology, 17(6), pp.525-531.

### Pharmaceutical composition

In one aspect, the present invention provides pharmaceutical composition comprising the polynucleotide or vector of the invention or the cell of the invention.

A pharmaceutical composition is a composition that comprises or consists of a therapeutically effective amount of a pharmaceutically active agent i.e. the vector. It preferably includes a pharmaceutically acceptable carrier, diluent or excipient (including combinations thereof).

By "pharmaceutically acceptable" is included that the formulation is sterile and pyrogen free. The carrier, diluent, and/or excipient must be "acceptable" in the sense of being compatible with the vector and not deleterious to the recipients thereof. Typically, the carriers, diluents, and excipients will be saline or infusion media which will be sterile and pyrogen free, however, other acceptable carriers, diluents, and excipients may be used.

Acceptable carriers, diluents, and excipients for therapeutic use are well known in the pharmaceutical art. The choice of pharmaceutical carrier, excipient or diluent can be selected with regard to the intended route of administration and standard pharmaceutical practice. The pharmaceutical compositions may comprise as - or in addition to - the carrier, excipient or diluent any suitable binder(s), lubricant(s), suspending agent(s), coating agent(s) or solubilising agent(s).

Examples of pharmaceutically acceptable carriers include, for example, water, salt solutions, alcohol, silicone, waxes, petroleum jelly, vegetable oils, polyethylene glycols, propylene glycol, liposomes, sugars, gelatin, lactose, amylose, magnesium stearate, talc, surfactants, silicic acid, viscous paraffin, perfume oil, fatty acid monoglycerides and diglycerides, petroethral fatty acid esters, hydroxymethyl-cellulose, polyvinylpyrrolidone, and the like.

The vector, cell, or pharmaceutical composition according to the present invention may be administered in a manner appropriate for treating and/or preventing the diseases described herein. The quantity and frequency of administration will be determined by such factors as the condition of the subject, and the type and severity of the subject's disease, although appropriate dosages may be determined by clinical trials. The pharmaceutical composition may be formulated accordingly.

The vector, cell or pharmaceutical composition according to the present invention may be administered parenterally, for example, intravenously, or by infusion techniques. The vector, cell or pharmaceutical composition may be administered in the form of a sterile aqueous solution which may contain other substances, for example, enough salts or glucose to make the solution isotonic with blood. The aqueous solution may be suitably buffered (preferably to a pH of from 3 to 9). The pharmaceutical composition may be formulated accordingly. The preparation of suitable parenteral formulations under sterile conditions is readily accomplished by standard pharmaceutical techniques well-known to those skilled in the art.

The vector, cell or pharmaceutical composition according to the present invention may be administered systemically, for example by intravenous injection.

The vector, cell or pharmaceutical composition according to the present invention may be administered locally, for example by targeting administration to the kidney. Suitably, the vector, cell or pharmaceutical composition may be administered by injection into the renal artery or by ureteral or subcapsular injection.

The pharmaceutical compositions may comprise vectors or cells of the invention in infusion media, for example sterile isotonic solution. The pharmaceutical composition may be enclosed in ampoules, disposable syringes or multiple dose vials made of glass or plastic.

The vector, cell or pharmaceutical composition may be administered in a single or in multiple doses. Particularly, the vector, cell or pharmaceutical composition may be administered in a single, one off dose. The pharmaceutical composition may be formulated accordingly.

The vector, cell or pharmaceutical composition may be administered at varying doses (e.g. measured in vector genomes (vg) per kg). The physician in any event will determine the actual dosage which will be most suitable for any individual subject and it will vary with the age, weight and response of the particular subject. Typically, however, for the AAV vectors of the invention, doses of 10¹⁰ to 10¹⁴ vg/kg, or 10¹¹ to 10¹³ vg/kg may be administered.

The pharmaceutical composition may further comprise one or more other therapeutic agents.

Disclosed herein but not part of the invention is the use of kits comprising the vector, cells and/or pharmaceutical composition of the present invention. Preferably said kits are for use in the methods and used as described herein, e.g., the therapeutic methods as described herein. Preferably said kits comprise instructions for use of the kit components.

### Methods for treating and/or preventing disease

In one aspect, the present invention provides the vector, cell or pharmaceutical composition according to the present invention for use as a medicament.

In one aspect, disclosed herein is the use of the vector, cell or pharmaceutical composition according to the present invention in the manufacture of a medicament.

### Glomerular diseases

The vector, cell or pharmaceutical composition according to the present invention may be used to treat glomerular diseases in a subject. Suitably, the subject is a human subject.

In one aspect, the present invention provides the vector, cell or pharmaceutical composition according to the present invention for use in preventing or treating a glomerular disease.

In a related aspect, disclosed herein is the use of the vector, cell or pharmaceutical composition according to the present invention for the manufacture of a medicament for preventing or treating a glomerular disease.

Glomerular diseases can be classified as either nephrotic or nephritic. Nephrotic syndrome often involves factors that affect the integrity of podocyte-podocyte or podocyte-GBM interactions. By contrast, factors involved in the etiology of nephritic syndrome can vary, but may include circulating platelets and white blood cells, the GBM, resident glomerular endothelial cells, and mesangial cells. (Chiang, C.K. and Inagi, R., 2010. Nature Reviews Nephrology, 6(9), p.539).

Suitably, the glomerular disease is a genetic glomerular disease, i.e. a glomerular disease which is inherited. Genetic glomerular diseases include podocyte-associated genetic glomerular diseases, such as nephrotic syndrome, and GBM-associated glomerular diseases, such as Alport Syndrome.

Suitably, the glomerular disease is a podocyte-associated genetic glomerular disease. Podocyte-associated genetic glomerular diseases include Congenital nephrotic syndrome of the Finnish type, Congenital nephrotic syndrome type 2, Familial nephrotic syndrome type 3, Frasier syndrome and Denys-Drash syndrome, Schimke immuno-osseous dysplasia, Nephrotic syndrome caused by mutations in CD2AP, Nephrotic syndrome caused by mutations in actinin-4, Nephrotic syndrome caused by mutations in TRPC6, and Epstein and Fechtner syndrome. Suitably, the glomerular disease is nephrotic syndrome.

Suitably, the glomerular disease is a GBM-associated genetic glomerular disease. Podocyte-associated genetic glomerular diseases include X-linked Alport syndrome, Autosomal recessive Alport syndrome, Autosomal dominant Alport syndrome, Thin basement membrane diseases, Pierson syndrome, and Nail-patella syndrome. Suitably, the glomerular disease is Alport syndrome (AS). AS is also known as familial nephritis, hereditary nephritis, thin basement membrane disease and thin basement membrane nephropathy.

### EXAMPLES

The invention will now be further described by way of Examples, which are meant to serve to assist one of ordinary skill in the art in carrying out the invention and are not intended in any way to limit the scope of the invention.

### Example 1 - design, construction, and testing of minimal nephrin promoters

### Design of minimal nephrin promoters

A human NPHS1 promoter has been described in Moeller et al. 2002 J Am Soc Nephrol, 13(6):1561-7 and Wong MA et al. 2000 Am J Physiol Renal Physiol, 279(6):F1027-32. This NPHS1 promoter is a 1.2kb fragment and appears to be podocyte-specific. This is referred to hereafter as a "FL" nephrin promoter and is shown in **Figure 1A****.**

The FL nephrin promoter was initially cut to 822 bp (819 bp excluding start codon) by deleting the N-terminal sequence. This is referred to hereafter as a "midi" nephrin promoter and is shown in **Figure 1B****.** The "midi" nephrin promoter is not covered by the subject-matter of the claims and is included as a reference example.

The midi nephrin promoter was further cut to 268 bp (265 bp excluding start codon) by removing putative general transcription domains from the central region. This is referred to hereafter as a "mini" nephrin promoter and is shown in **Figure 1C****.** The "mini" nephrin promoter is covered by the subject-matter of the claims.

### Construction of vector constructs

### Midi nephrin promoter

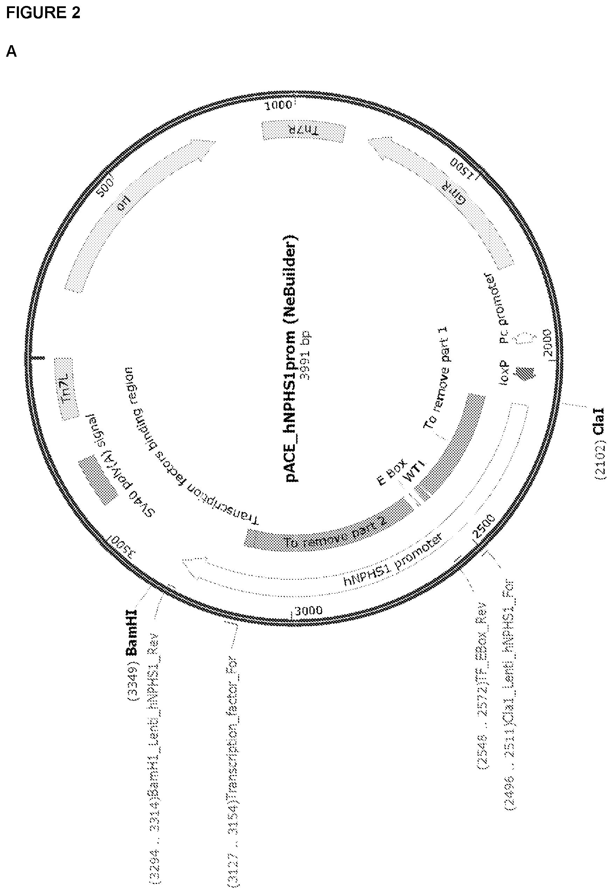

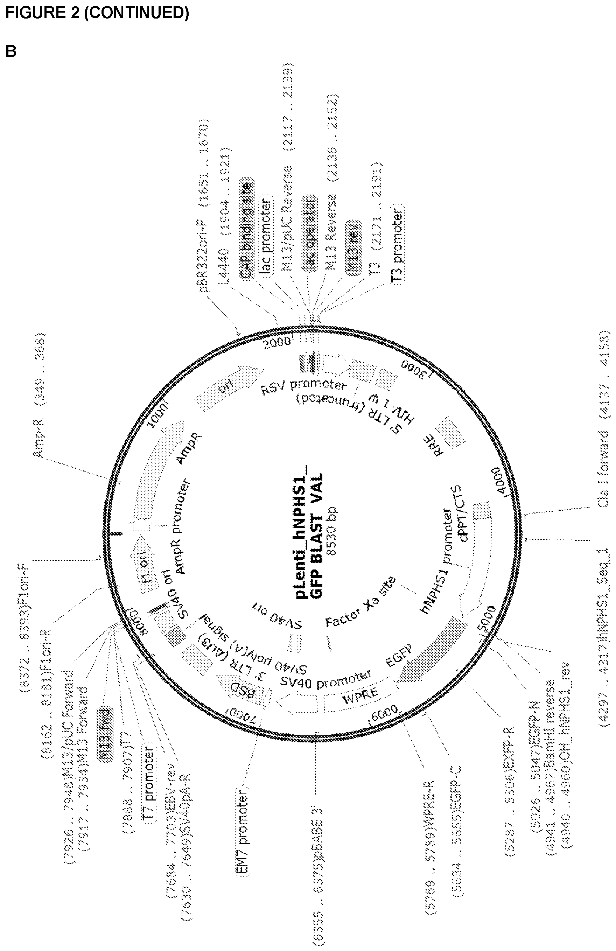

pACE_hNPHS1 promoter was used as a template to introduce BamHI and Clal restriction sites as shown in **Figure 2A****.** Fragments were then gel extracted and digested with Clal and BamHI for 1h at 37°C prior to ligation into pLenti GFP Blast vector. Ligations were further transformed into stable competent E.Coli cells, DNA was extracted and sequenced (Midi Promoter). The final lentiviral vector is shown in **Figure 2B****.**

### Mini nephrin promoter

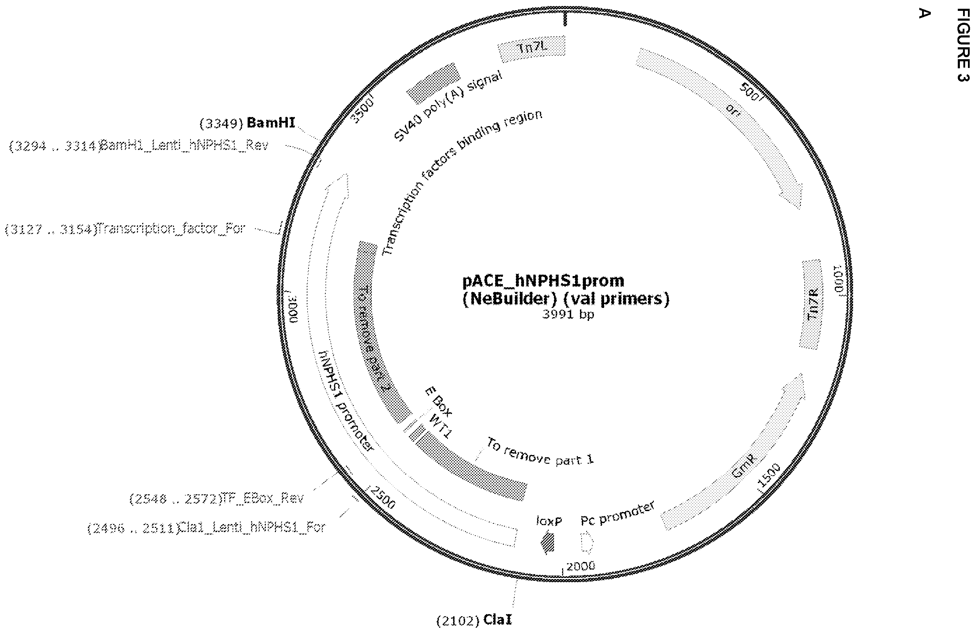

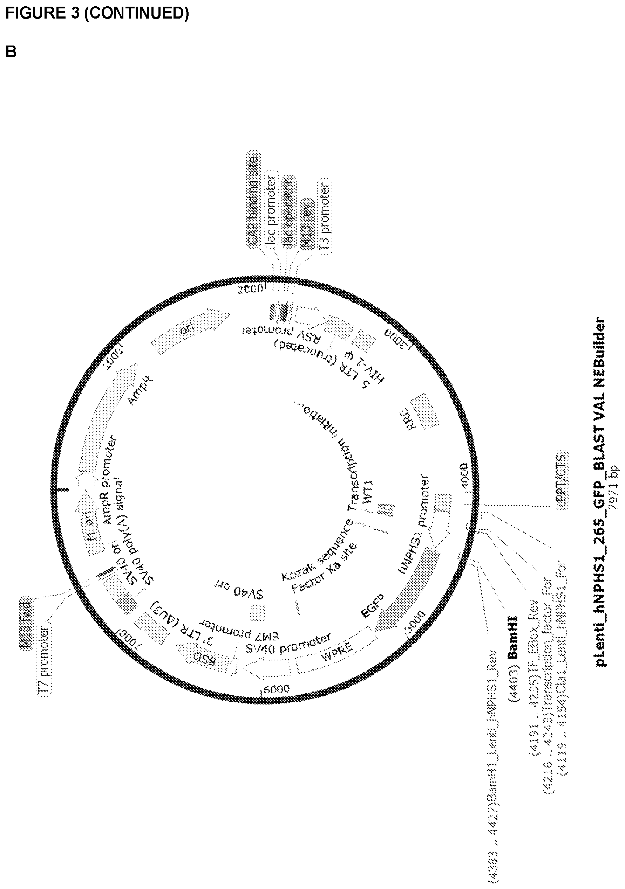

pACE_hNPHS1 promoter was used to PCR the overhangs (OHs) as shown in **Figure 3A****.** Two sections of the promoter containing OHs were gel extracted for the NEBuilder HiFi Assembly reaction into pLenti GFP Blast vector. The ligation reaction was then cleaned using the DNA clean up kit, prior to its transformation into stable competent *E.Coli* cells. DNA was extracted and sequenced. The final lentiviral vector is shown in **Figure 3B****.**

### Testing vector constructs

The minimal nephrin promotors were used to express GFP in *in vitro* cell models to check efficacy and podocyte-specificity.

pLenti GFP Blast Nephrin Promoter constructs (Full Length, Midi and Mini) were used to transfect HEK293T cells for 48h to make virus, which was further used to create human conditionally immortalized podocytes stably expressing either GFP-tagged FL NPHS1, midi or mini promoters.

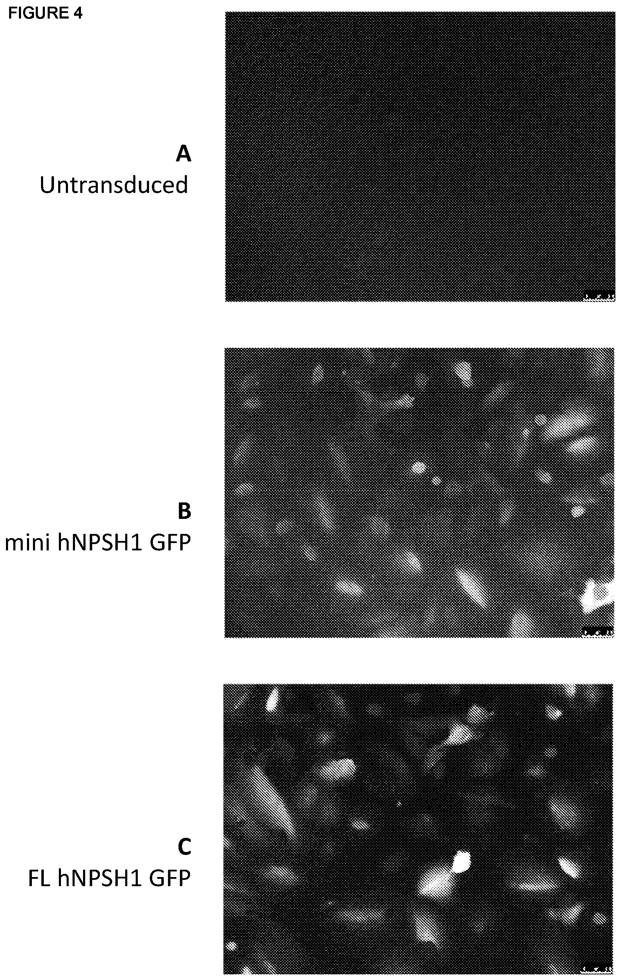

Conditionally immortalised human podocytes (ciPodocytes) were transfected with the lentiviral vectors to determine whether the minimal promoters were able to drive GFP expression. Both the midi and mini nephrin promoters were shown to drive GFP expression. **Figure 4** shows a representative fluorescence microscopy image showing GFP expression from the mini nephrin promoter. **Figure 5** shows a representative western blot showing GFP expression from the mini nephrin promoter. These results show that a mini nephrin promoter is able to drive transgene expression in podocytes.

The lentiviral vectors were also used to transduce human glomerular cells. ciPodocytes and glomerular endothelial cells were transduced with a lentivirus comprising GFP coupled to the mini nephrin reporter. **Figure 6A-C** shows FACS analysis displaying median GFP fluorescence (AFU) of all live singlets of conditionally immortalised human podocytes (LY) and glomerular endothelial cells (GEnC) using a Novocyte Analyser. Untransduced cells (Cell Control) were compared with those transduced with lentivirus constructs harbouring a GFP expression cassette controlled by the full length human nephrin promoter (hNPHS1.GFP) or the mini human nephrin promoter (265.GFP). All cells were differentiated for 10 days, trypsinised (100 uL) and diluted in PBS, 2%FBS, 1:1000 DRAQ7 (150uL). Data and error bars represent 3 technical repeats (100 uL, >2500 cells) ±SEM. These results show podocyte specificity for a minimal nephrin promoter when compared to glomerular endothelial cells.

### Example 2 - design, construction, and testing of minimal nephrin promoter coupled to COL4A3, COL4A4, and COL4A5

### Design and construction of AAV constructs

The following AAV transfer plasmids comprising COL4A3, COL4A4, and COL4A5 coupled to the mini nephrin promoter ("265") were designed and constructed:
- pAAV.265.Col4a3.3flag.sv40, an AAV plasmid comprising COL4A3 coupled to mini nephrin promoter (see **Figure 7A****).**
- pAAV.265.Col4a4.3flag.sv40, an AAV plasmid comprising COL4A4 coupled to mini nephrin promoter (see **Figure 7B****)**
- pAAV.265.Col4a5.3flag.sv40, an AAV plasmid comprising COL4A5 coupled to mini nephrin promoter (see **Figure 7C****)**

A Smal digestion was performed to confirm that the identity of the plasmids (see **Figures 7D-****E).** This shows successful cloning of COL4 a3, a4 and a5 into AAV, with the 265bp mini nephrin promoter and a SV40 polyA tail.

### Testing of AAV constructs

The following AAV viral vectors were prepared using standard methods:
- AAV.COL4A3.nephrin265.Sv40 with LK03 serotype
- AAV.COL4A5.nephrin265.Sv40 with LK03 serotype
- AAV.COL4A5.nephrin265.Sv40 with 2/9 serotype

**Figure 8A** shows immunoprecipitation experiments of the full-length FLAG-tagged Col4a3 (LK03) or Col4a5 (LK03) in human differentiated ciPodocytes pulled down with the anti-FLAG antibody. Anti-FLAG antibody precipitated both Col4a3 and Col4a5. Human-FLAG IgG was used as a control.

**Figure 8B** shows western blots of protein lysates showing the expression levels of Col4a3 (LK03 capsid serotype), Col4a5 (LK03) and Col4a5 (2/9 capsid serotype) in human or mouse differentiated ciPodocytes. Non-infected human and mouse cipodocytes were used as controls.

**Figure 8C** shows confocal images showing immunofluorescence staining of transduced Col4a5 in Human wild-type CiPodocytes/Col4a5 3xFlag AAV CiPodocytes with F-Actin. Col4a5 is present at the cytosolic level in the human differentiated podocytes infected with Col4a5 3xFlag AAV virus in comparison to the wild-type counterpart.

These results show that we have successfully transduced human podocytes with COL4a3 and COL4a5 full length coupled to a mini nephrin promoter and that a mini nephrin promoter drives expression of the full length COL4a3 or COL4a5 in the human podocytes.

### Example 3 - Expression of CFH, CFI and CFHL1 in HEK cells

### Materials and methods

60-80% confluent 293T Human Embryonic Kidney cells grown in DMEM supplemented with 10% FBS were triple transfected with pHelper (HGTI1), one of the two pAAV Rep-Cap (LK03 or AAV9) and one of the three ITR-expression plasmids containing 1) CFH under the 265bp minimal nephrin promoter of SEQ ID NO: 47 (pAAV-265-CFH), 2) CFI under the full-length minimal nephrin promoter of SEQ ID NO: 14 (pAAV-FL-CFI) or 3) CFHL1 under the full-length minimal nephrin promoter of SEQ ID NO: 14 (pAAV-FL-CFHL1). All constructs were tagged with MYC and FLAG.

Transfection was carried out on a 150mm culture dish in serum-free media in the presence of polyethylenimine (PEI). Media was changed to DMEM with FBS the following day after transfection. On Day 4 post-transfection, media and cells were collected and processed separately. Media was frozen and stored at -80°C. Cells were lysed with RIPA buffer supplemented with proteinase inhibitors and stored at -80°C.

Protein concentration in the cell lysates was measured using Pierce BCA protein assay and 10ug of total protein from each sample was loaded onto a 4-15% polyacrylamide Tris-Glycine gel. A total of 2.6ul of media from each sample was loaded onto the gel. Protein was transferred to a nitrocellulose membrane using the iBlot2 dry blotting system. The following primary antibodies were used for protein detection: anti-Factor H (Abcam, cat. ab124769), anti-Factor I (Abcam, ab278524), anti-MYC-tag (CST, cat.2276S), anti-FLAG-tag (CST, cat. 14793S) and anti-GAPDH (Millipore, cat. MAB374).

### Results

Factor H was expressed in both the cell lysates and the media from 293T HEK cells transfected with the CFH expression plasmid **(****Figure 9****, lanes 2, 3, 8, 9).** Expression was detected using a Factor H-specific antibody. Factor H was not detected in the cell lysates or media of untransfected cells or cells transfected with the CFI or CFHL1 expression plasmids **(****Figure 9****, lanes 1, 4-7, 10-13).** These results demonstrate that a 265bp minimal nephrin promoter allows expression of the transgene in target cells.

### Example 4 - Transduction of Factor H mutated podocytes with AAV2/9 265-CFH or transfection with plasmid encoding CFH

### Transduction of Factor H mutated podocytes with AAV2/9 265-CFH

Conditionally immortalised human podocytes with a mutation in endogenous Factor H (Muehlig et al, 2020) grown in RPMI supplemented with 10% FBS and 1% ITS were seeded in 6-well culture plates and grown at 33°C until 70-80% confluency. Cells were incubated with AAV2/9 containing a CFH transgene under the control of the 265bp minimal nephrin promoter (SEQ ID NO: 47). Cells incubated without the virus were used as a non-transduced (NT) control. On the same day following viral transduction, cells were transferred to a non-permissive temperature of 37°C to allow for cell differentiation and transgene expression. Media was changed twice on the subsequent days following transduction. On Day 10 post-transduction, cell media was collected and the concentration of human Factor H was measured by ELISA using an anti-Factor H antibody (Abcam, cat. ab252359).

Human podocytes with a mutation in Factor H were transduced with AAV2/9 virus containing a CFH transgene under the control of the 265 bp minimal nephrin promoter demonstrated higher concentrations of human Factor H in the culture media than untransduced cells **(****Figures 10A and 10B****).** This demonstrates that a Factor H transgene delivered by AAV and under the control of a 265bp minimal nephrin promoter can be expressed in human podocytes.

### Transfection of Factor H mutated podocytes with plasmid encoding CFH

Conditionally immortalised human podocytes with a mutation in endogenous Factor H (Muehlig et al, 2020) grown in RPMI supplemented with 10% FBS and 1% ITS were seeded in 6-well culture plates and grown at 33°C until 70-80% confluency. For plasmid transfection, cells were incubated with 1.5ug of expression plasmid in serum-free media in the presence of polyethylenimine. Cells where no plasmid was added were used as a non-transfected (NT) control. The media was changed the following day to media containing FBS. On Day 3 post-transfection, media was collected and analysed by ELISA (Abcam, cat. ab252359).

Podocytes transfected with a plasmid expressing the CFH transgene under the control of a 265bp minimal nephrin promoter demonstrated higher concentrations of human Factor H than the non-transfected control **(****Figure 10C****).**

### Example 5 - Expression of CFH in the kidney in WT mice

### Materials and methods

100 µl of AAV2/9 gene therapy product (pAAV.NPHS1(265).hCFH.WPRE.bGH) or saline was administered to wild-type C57BL6 mice by IV tail vein injection. AAV expressed tagged wild-type human CFH transgene was under control of the 265bp minimal nephrin promoter (SEQ ID NO: 47). AAV was harvested and purified by ultracentrifugation 3 days later and titrated (~1.5x10e13/ml) in PBS. All animals completed the study on Day 21 and culled. Kidneys were snap frozen in liquid nitrogen and used for RNA extraction by RNeasy Micro Kit (Qiagen Cat. No. / ID: 74004) as per the manufacturers' protocol. RNA was then converted into cDNA using High-Capacity RNA-to-cDNA^{™} Kit (4387406) prior to qPCR analysis.

Quantitative qPCR was performed on DNA samples from kidneys of pAAV_CFH injected mice. Standard curve qPCR with SYBR green reagents and passive ROX method was used to detect ITR presence in the kidney DNA samples. Viral genomes per pg of DNA were calculated using a standard curve of known quantities of an ITR amplicon. Finally, viral genomes per cell were calculated based on the assumption that diploid mouse cells have 6pg of DNA.

Immunofluorescence staining was performed on the frozen kidney sections 5 microns thick using an anti-nephrin antibody (PROGEN) and an anti-CFH antibody (ab124767).

Tissue was embedded in OCT compound (VWR, cat. number 361603E) and snap frozen in liquid nitrogen. 10uM sections were cut using a cryostat (Thermo, Cryostar NX270). Tissue sections were fixed with 4% Paraformaldehyde for 20 minutes at room temperature, permeabilized with 0.3% Triton-X for 15 minutes at room temperature and blocked with 5% BSA for 30 minutes at room temperature.

Primary antibodies (Rabbit monoclonal [EPR6226] to Factor H, Abcam cat. number ab124769 and Nephrin (NPHS1) (1243-1256) Guinea Pig Polyclonal Antibody, Origene cat. number BP5030) were diluted in 5% BSA. (1:100 for anti-factor H and 1:300 for anti-nephrin). Incubation was done at room temperature for 1 hour.

Secondary antibodies (Goat anti-Rabbit IgG (H+L) Secondary Antibody, Alexa Fluor Plus 488, Invitrogen, cat. number A32731 and Goat anti-Guinea Pig IgG (H+L) Alexa Fluor 568, Invitrogen, cat. number A-11075) were diluted 1:500 in PBS and incubation was done 30 minutes at room temperature.

Slides were mounted in Fluoromount-G, slide mounting medium (Southern Biotech, cat. number 0100-01) and imaged on a Leica DM750 Fluorescence Microscope at 20X magnification.

### Results

Injection of wild type mice with AAV containing human CFH under the 265bp minimal nephrin promoter leads to infection of the kidney by the AAV and greater expression of the virus and human CFH in the kidney compared to wild type mice injected with saline control **(****Figure 11A-****C).** Immunofluorescence staining demonstrates the co-localization of nephrin and CFH in the mouse glomerulus (**Figure 11D**).

### Example 7 - Expression of GFP using 265bp v 818bp v FL minimal nephrin promoters (plasmid transfection) in kidney cells

Plasmids were constructed comprising eGFP under the control of: (i) the full-length (FL) minimal nephrin promoter of SEQ ID NO: 14 (PS0281); (ii) the 818bp minimal nephrin promoter of SEQ ID NO: 59 (PS0301); or (iii) the 265bp minimal nephrin promoter of SEQ ID NO: 47 (P20282).

Podocytes, glomerular endothelial cells (GENCs), and proximal tube epithelial cells (PTECs) were seeded at a density of 1.5e5 cells per well of a six well plate and allowed to attach overnight. For HEK293T cells, 5e5 cells per well were used. The following day 3ug of plasmid DNA in 150ul of DMEM was mixed with 9ul PEI (also in 150ul DMEM), incubated for 15 minutes at room temperature, and analysed 48 hours later by flow cytometry for eGFP expression. Non transfected cells were used as a negative control for gating of negative and positive populations.

The results in **Figure 12** show that all three promoters were capable of driving eGFP expression in all cell types tested. These results demonstrate that minimizing the full length nephrin promoter to either 818bp or 265bp still allows expression of the transgene in target cells, despite removing large regions of the nephrin promoter sequence.

### Example 8 - Expression of GFP using 265bp v FL minimal nephrin promoters (AAV transduction) in kidney cells

### Materials and methods

**AAV Production:** 60-80% confluent 293T Human Embryonic Kidney cells grown in DMEM supplemented with 10% FBS were triple transfected with pHelper (HGTI1, PS0150), a Rep-Cap plasmid (LK03, PS0240) and one of the two ITR-expression plasmids containing the Full Length (FL) minimal nephrin promoter or the 265bp minimal nephrin promoter driving eGFP expression (PS0281 and PS0282, respectively). Transfection was carried out on a 150mm culture dish in the presence of polyethylenimine (PEI). Media was changed to DMEM with FBS the following day after transfection. On Day 4 post-transfection, media and cells were collected and processed separately. PEG was added to filtered supernatants to precipitate viral particles which were subsequently centrifuged at 1500g for 20 minutes. Cell pellets were resuspended in TD buffer and frozen at -80 degrees, followed by thawing at 37 degrees and vortexed. Freeze-thaw cycles were repeated 5 times. Pellets from the PEG treated supernatant were then added to the lysed cells and Benzonase and Sodium deoxycholate was further added and incubated at 37 degrees for 30 minutes. Samples were then centrifuged and the supernatant filtered and subsequently purified via iodixanol gradient ultracentrifugation.

**AAV QC:** AAV samples were titred via qPCR to determine the number of viral genomes per ml of viral supernatant. Primers designed to target the eGFP sequence were used. SDS-PAGE analysis was performed on samples to determine purity. Alkaline gel electrophoresis was used to demonstrate incorporation of full transgene cassettes into the viral particles.

**AAV Transduction:** Cells were seeded into wells of a 6-well plate at a density of 1.5e5 cells/well and allowed to adhere overnight. The following day viral particles were added a multiplicity of infection (MOI) of 5e5 particles per cell. Cells were placed at 37 degrees to initiate differentiation and cultured for 10 days. After 10 days cells were analysed for eGFP expression by flow cytometry.

### Results

The results in **Figure 13** show that both promoters were capable of driving eGFP expression in all cell types tested. The 265bp minimal nephrin promoter showed higher eGFP expression levels in each of the cells tested.

These data again demonstrate that a 265bp minimal nephrin promoter is capable of driving eGFP expression in target cells. Additionally, that this promoter may be incorporated into an AAV vector for use in gene therapy. The use of a 265bp minimal nephrin promoter, in place of the full length minimal nephrin promoter, may allow the incorporation of more genetic material into a transgene cassette for AAV purposes.

### Example 9 - Expression of human podocin using 265bp v FL minimal nephrin promoters (plasmid transfection) in HEK

Plasmids were constructed comprising HA-tagged podocin under the control of: (i) the full-length (FL) minimal nephrin promoter of SEQ ID NO: 14 (FL.NPSH1-Podocin-HA); or (ii) the 265bp minimal nephrin promoter of SEQ ID NO: 47 (265.hNPSH1-Podocin-HA).

293T Human Embryonic Kidney cells grown in DMEM supplemented with 10% FBS, 1% Penicillin/Streptomycin and 1% sodium pyruvate were seeded at a density of 5e5 in 6-well culture plates. Transfection was carried out the following day when the cells reached 70-80% confluency. Transfection reagent mix was prepared by complexing 3ug of expression plasmid with polyethylenimine (PEI) at ratio of 1:3 in serum-free media. Non-transfected control received a transfection mix containing PEI at the same ratio without any expression plasmid. Cells were incubated with the transfection mix at 37°C, 5% CO2 until the following day when the media was changed to complete media. Cells were harvested on Day 2 post transfection and analysed by FACs for expression of Podocin by staining for HA-tag with anti-HA antibody.

The results in **Figure 14A** show that both promoters were capable of driving human Podocin-HA expression in HEK cells. This demonstrates that minimizing the full length minimal nephrin promoter to 265bp still allows expression of the transgene in target cells, despite removing the majority of the promoter sequence.

### Example 10 - Expression of human podocin using 265bp v FL minimal nephrin promoters (AAV transduction) in human podocytes

### Materials and method

**AAV Production:** 60-80% confluent 293T Human Embryonic Kidney cells grown in DMEM supplemented with 10% FBS,1% Penicillin/Streptomycin and 1% sodium pyruvate were triple transfected with pHelper (HGTI1, PS0150), pAAV Rep-Cap (LK03, PS0240) and one of the two ITR-expression plasmids containing the Full Length (FL) minimal nephrin promoter or the 265bp minimal nephrin promoter driving Podocin-HA expression (FL.NPSH1-Podocin-HA and 265.hNPSH1-Podocin-HA, respectively). Transfection was carried out on a 150mm culture dish in the presence of polyethylenimine (PEI)at a ratio of 1:2. Media was changed to DMEM with FBS the following day after transfection. On Day 4 post-transfection, media and cells were collected and processed separately. PEG was added to filtered supernatants to precipitate viral particles which were subsequently centrifuged at 1500g for 20 minutes. Cell pellets were resuspended in TD buffer and frozen at -80 degrees, followed by thawing at 37 degrees and vortexed. Freeze-thaw cycles were repeated 5 times. Pellets from the PEG treated supernatant were then added to the lysed cells. Benzonase and Sodium deoxycholate was further added and incubated at 37 degrees for 30 minutes. Samples were then centrifuged and the supernatant filtered and subsequently purified via iodixanol gradient ultracentrifugation.

**AAV QC:** AAV samples were titred via qPCR to determine the number of viral genomes per ml of viral supernatant. Primers designed to target the Podocin sequence were used.

**AAV Transduction:** Cells were seeded into wells of a 12-well plate at a density of 0.5e5 cells/well and allowed to adhere overnight. The following day viral particles were added a multiplicity of infection (MOI) of 5e5 particles per cell. Cells were placed at 37 degrees to initiate differentiation and cultured for 9 days. After 9 days cells were harvested and stained with anti-HA antibody for HA expression by flow cytometry as a measure of Podocin expression.

### Results

As demonstrated with the plasmid transfection data in HEK cells **(****Figure 11A****),** the results shown in **Figure 14B** further confirm that both promoters are capable of driving expression of a transgene in human podocytes. The 265bp minimal nephrin promoter showed higher podocin expression levels in the human podocytes.

### Example 11 - In vivo expression of GFP using 265bp v FL promoters via AAV transduction

The 265- and FL- nephrin promoters were evaluated *in vivo* in WT mice (C57/BI6). AAV particles as described in **Example 8,** except that AAV9 (PS0241) was used instead of LK03, were administered via tail vein injection of ~1x10e13 AAV2/9 per mouse (2x mice per treatment group and 1x untreated mouse). Tissue harvest of kidney occurred 4 weeks post injection. qPCR analysis performed on the kidney and GFP copy number normalised to 18S (housekeeping gene). 18S rRNA is a reliable normalisation gene for real time PCR.

The results in **Figure 15** show that there was detectable GFP expression in the kidney using both the 265bp and full length minimal nephrin promoters. The expression in the kidney was higher using the 265bp minimal nephrin promoter.

## Claims

1. A promoter which is capable of driving transgene expression in kidney cells and comprises or consists of a nucleotide sequence having at least 90% identity to SEQ ID NO: 47.

2. The promoter according to claim 1, wherein the promoter comprises or consists of a nucleotide sequence having at least 95%, at least 96%, at least 97%, at least 98%, or at least 99% identity to SEQ ID NO: 47.

3. The promoter according to any preceding claim, wherein: the promoter comprises a proximal promoter region having at least 95%, at least 96%, at least 97%, at least 98%, or at least 99% identity to SEQ ID NO: 17, optionally wherein the proximal promoter region comprises each of (a) a retinoic acid receptor binding site, (b) a WT1 binding site, and (c) an enhancer box; and wherein the promoter has a length of 1.1 kb or less.

4. The promoter according to claim 3, wherein one or more of the following nucleotide sequences is present in the proximal promoter region: (a) GGGGTCA at position 7 to position 13 of the proximal promoter region; (b) CGGAGGCTGGGGAGGCA at position 14 to position 30 of the proximal promoter region; and (c) ATGTG at position 49 to position 53 of the proximal promoter region.

5. The promoter according to claim 3 or 4, wherein the promoter comprises a core promoter region having at least 95%, at least 96%, at least 97%, at least 98%, or at least 99% identity to SEQ ID NO: 18, optionally wherein the core region comprises each of (d) a transcription factor binding region; and (e) a transcription initiation site.

6. The promoter according to any of claims 3 to 5, wherein the promoter comprises a 5'UTR having at least 95%, at least 96%, at least 97%, at least 98%, or at least 99% identity to SEQ ID NO: 19.

7. The promoter according to any preceding claim, wherein the promoter comprises or consists of the nucleotide sequence of SEQ ID NO: 47.

8. A polynucleotide comprising the promoter according to any one of claims 1 to 7.

9. The polynucleotide according to claim 8, wherein the promoter is operably linked to a protein coding sequence, preferably wherein the promoter is operably linked to a protein coding sequence encoding NPHS2 or a fragment and/or variant thereof; a COL4A3, COL4A4 or COL4A5 polypeptide, or a fragment or derivative thereof; or CFI, CFH or FHL-1, or a fragment and/or variant thereof, more preferably wherein the promoter is operably linked to:
(a) a protein coding sequence encoding NPHS2, or a fragment and/or variant thereof, preferably wherein the protein coding sequence encodes an amino acid sequence which is at least 70%, at least 80%, at least 90%, at least 95%, at least 98% or at least 99% identical to SEQ ID NO: 55 or a fragment thereof;
(b) a protein coding sequence encoding a COL4A3, COL4A4 or COL4A5 polypeptide, or a fragment or derivative thereof, preferably wherein the protein coding sequence encodes an amino acid sequence which is at least 70%, at least 80%, at least 90%, at least 95%, at least 98% or at least 99% identical to one of SEQ ID NOs: 21-23 or a fragment thereof; and/or
(c) a protein coding sequence encoding CFI, CFH or FHL-1, or a fragment and/or variant thereof, preferably wherein the protein coding sequence encodes an amino acid sequence which is at least 70%, at least 80%, at least 90%, at least 95%, at least 98% or at least 99% identical to one of SEQ ID NOs: 49, 51 or 53, or a fragment thereof,
optionally wherein the protein coding sequence is operably linked to one or more further regulatory elements, such as a post-transcriptional regulatory element and/or a polyadenylation sequence.

10. A vector comprising the polynucleotide according to claim 8 or 9.

11. The vector according to claim 10, wherein the vector is:
(a) capable of transducing podocytes, optionally wherein the vector is capable of specifically transducing podocytes; and/or
(b) a viral vector, such as an adeno-associated virus (AAV) vector, a lentiviral vector, a retroviral vector, an adenoviral vector, an adeno-associated viral vector, a herpes simplex viral vector, an alphaviral vector, a flaviviral vector, a rhabdoviral vector, a measles viral vector, a Newcastle disease viral vector, a poxviral vector, and a picornaviral vector, preferably wherein the vector is an AAV vector, optionally wherein the viral vector is in the form of a viral vector particle, preferably wherein the viral vector is in the form of an AAV vector particle, preferably wherein the vector is the form of an AAV vector particle encapsidated by AAV3B, LK03, or AAV9 capsid proteins.

12. A cell comprising the polynucleotide according to claim 8 or 9, or the vector according to claim 10 or 11.

13. A pharmaceutical composition comprising the polynucleotide according to claim 8 or 9, the vector according to claim 10 or 11, or the cell according to claim 12.

14. A polynucleotide according to claim 8 or 9, vector according to claim 10 or 11, or cell according to claim 12 for use in medicine.

15. *In vitro* use of the promoter according to any one of claims 1 to 7 to drive expression of a coding sequence, preferably wherein the expression is kidney-specific.

## Patentansprüche

1. Promotor, der in der Lage ist, eine Transgenexpression in Nierenzellen anzutreiben, und eine Nukleotidsequenz umfasst oder aus einer Nukleotidsequenz besteht, die eine Identität von mindestens 90 % mit SEQ ID NO: 47 aufweist.

2. Promotor nach Anspruch 1, wobei der Promotor eine Nukleotidsequenz umfasst oder aus einer Nukleotidsequenz besteht, die eine Identität von mindestens 95 %, mindestens 96 %, mindestens 97 %, mindestens 98 % oder mindestens 99 % mit SEQ ID NO: 47 aufweist.

3. Promotor nach einem vorhergehenden Anspruch, wobei: der Promotor eine proximale Promotorregion umfasst, die eine Identität von mindestens 95 %, mindestens 96 %, mindestens 97 %, mindestens 98 % oder mindestens 99 % mit SEQ ID NO: 17 aufweist, optional wobei die proximale Promotorregion jedes von (a) einer Retinsäure-Rezeptor-Bindungsstelle, (b) einer WT1-Bindungsstelle und (c) einer Enhancer-Box umfasst; und wobei der Promotor eine Länge von 1,1 kb oder weniger aufweist.

4. Promotor nach Anspruch 3, wobei eine oder mehrere der folgenden Nukleotidsequenzen in der proximalen Promotorregion vorhanden sind: (a) GGGGTCA an Position 7 bis Position 13 der proximalen Promotorregion; (b) CGGAGGCTGGGGAGGCA an Position 14 bis Position 30 der proximalen Promotorregion; und (c) ATGTG an Position 49 bis Position 53 der proximalen Promotorregion.

5. Promotor nach Anspruch 3 oder 4, wobei der Promotor eine Kernpromotorregion umfasst, die eine Identität von mindestens 95 %, mindestens 96 %, mindestens 97 %, mindestens 98 % oder mindestens 99 % mit SEQ ID NO: 18 aufweist, optional wobei die Kernregion jede von (d) einer Transkriptionsfaktor-Bindungsregion; und (e) einer Transkriptionsinitiationsstelle umfasst.

6. Promotor nach einem der Ansprüche 3 bis 5, wobei der Promotor eine 5'-UTR umfasst, die eine Identität von mindestens 95 %, mindestens 96 %, mindestens 97 %, mindestens 98 % oder mindestens 99 % mit SEQ ID NO: 19 aufweist.

7. Promotor nach einem vorhergehenden Anspruch, wobei der Promotor die Nukleotidsequenz von SEQ ID NO: 47 umfasst oder aus dieser besteht.

8. Polynukleotid, umfassend den Promotor nach einem der Ansprüche 1 bis 7.

9. Polynukleotid nach Anspruch 8, wobei der Promotor mit einer Protein-kodierenden Sequenz wirkverbunden ist, vorzugsweise wobei der Promotor mit einer Protein-kodierenden Sequenz wirkverbunden ist, die NPHS2 oder ein Fragment und/oder eine Variante davon kodiert; ein COL4A3-, COL4A4- oder COL4A5-Polypeptid oder ein Fragment oder Derivat davon; oder CFI, CFH oder FHL-1 oder ein Fragment und/oder eine Variante davon, stärker bevorzugt, wobei der Promotor mit Folgendem wirkverbunden ist:
(a) einer Protein-kodierenden Sequenz, die NPHS2 kodiert oder ein Fragment und/oder eine Variante davon, wobei die Protein-kodierende Sequenz vorzugsweise eine Aminosäuresequenz kodiert, die zu mindestens 70 %, mindestens 80 %, mindestens 90 %, mindestens 95 %, mindestens 98 % oder mindestens 99 % identisch mit SEQ ID NO: 55 ist, oder ein Fragment davon;
(b) einer Protein-kodierenden Sequenz, die ein COL4A3-, COL4A4- oder COL4A5-Polypeptid kodiert oder ein Fragment oder Derivat davon, wobei die Protein-kodierende Sequenz vorzugsweise eine Aminosäuresequenz kodiert, die zu mindestens 70 %, mindestens 80 %, mindestens 90 %, mindestens 95 %, mindestens 98 % oder mindestens 99 % identisch mit einem von SEQ ID NOs: 21-23 ist, oder ein Fragment davon; und/oder
(c) einer Protein-kodierenden Sequenz, die CFI, CFH oder FHL-1 kodiert oder ein Fragment und/oder eine Variante davon, wobei die Protein-kodierende Sequenz vorzugsweise eine Aminosäuresequenz kodiert, die zu mindestens 70 %, mindestens 80 %, mindestens 90 %, mindestens 95 %, mindestens 98 % oder mindestens 99 % identisch mit einer der SEQ ID NOs: 49, 51 oder 53 ist, oder ein Fragment davon,
optional wobei die Protein-kodierende Sequenz mit einem oder mehreren weiteren regulatorischen Elementen wirkverbunden ist, wie einem posttranskriptionellen regulatorischen Element und/oder einer Polyadenylierungssequenz.

10. Vektor, umfassend das Polynukleotid nach Anspruch 8 oder 9.

11. Vektor nach Anspruch 10, wobei der Vektor Folgendes ist:
(a) in der Lage, Podozyten zu transduzieren, wobei der Vektor optional in der Lage ist, Podozyten spezifisch zu transduzieren; und/oder
(b) ein viraler Vektor, wie etwa ein Adeno-assoziierter-Virus(AAV)-Vektor, ein lentiviraler Vektor, ein retroviraler Vektor, ein adenoviraler Vektor, ein Adeno-assoziierter viraler Vektor, ein Herpes-Simplex-Virus-Vektor, ein alphaviraler Vektor, ein flaviviraler Vektor, ein rhabdoviraler Vektor, ein Masern-Virus-Vektor, ein Newcastle-Disease-Virus-Vektor, ein Poxvirus-Vektor und ein Picornavirus-Vektor, wobei der Vektor vorzugsweise ein AAV-Vektor ist, wobei optional der virale Vektor in der Form eines viralen Vektorpartikels ist, wobei vorzugsweise der virale Vektor in der Form eines AAV-Vektorpartikels ist, wobei vorzugsweise der Vektor die Form eines AAV-Vektorpartikels ist, das durch AAV3B-, LK03- oder AAV9-Kapsidproteine enkapsidiert ist.

12. Zelle, umfassend das Polynukleotid nach Anspruch 8 oder 9 oder den Vektor nach Anspruch 10 oder 11.

13. Pharmazeutische Zusammensetzung, umfassend das Polynukleotid nach Anspruch 8 oder 9, den Vektor nach Anspruch 10 oder 11 oder die Zelle nach Anspruch 12.

14. Polynukleotid nach Anspruch 8 oder 9, Vektor nach Anspruch 10 oder 11 oder Zelle nach Anspruch 12 zur Verwendung in der Medizin.

15. *In-vitro*-Verwendung des Promotors nach einem der Ansprüche 1 bis 7, um die Expression einer kodierenden Sequenz anzutreiben, wobei die Expression vorzugsweise nierenspezifisch ist.

## Revendications

1. Promoteur capable d'induire l'expression d'un transgène dans des cellules rénales et comprenant ou étant constitué d'une séquence nucléotidique comportant au moins 90 % d'identité avec SEQ ID N° : 47.

2. Promoteur selon la revendication 1, dans lequel le promoteur comprend ou est constitué d'une séquence nucléotidique comportant au moins 95 %, au moins 96 %, au moins 97 %, au moins 98 % ou au moins 99 % d'identité avec SEQ ID N° : 47.

3. Promoteur selon une quelconque revendication précédente, dans lequel : le promoteur comprend une région promotrice proximale comportant au moins 95 %, au moins 96 %, au moins 97 %, au moins 98 % ou au moins 99 % d'identité avec SEQ ID N° : 17, éventuellement ladite région promotrice proximale comprenant chacun parmi (a) un site de liaison au récepteur de l'acide rétinoïque, (b) un site de liaison WT1, et (c) une boîte amplificatrice (enhancer box) ; et ledit promoteur comportant une longueur de 1,1 kb ou moins.

4. Promoteur selon la revendication 3, dans lequel une ou plusieurs des séquences nucléotidiques suivantes sont présentes dans la région promotrice proximale : (a) GGGGTCA à la position 7 jusqu'à la position 13 de la région promotrice proximale ; (b) CGGAGGCTGGGGAGGCA à la position 14 jusqu'à la position 30 de la région promotrice proximale ; et (c) ATGTG à la position 49 jusqu'à la position 53 de la région promotrice proximale.

5. Promoteur selon l'une des revendications 3 ou 4, ledit promoteur comprenant une région promotrice centrale comportant au moins 95 %, au moins 96 %, au moins 97 %, au moins 98 % ou au moins 99 % d'identité avec SEQ ID N° : 18, éventuellement ladite région centrale comprenant chacun parmi (d) une région de liaison à un facteur de transcription ; et (e) un site d'initiation de la transcription.

6. Promoteur selon l'une quelconque des revendications 3 à 5, ledit promoteur comprenant une 5'UTR comportant au moins 95 %, au moins 96 %, au moins 97 %, au moins 98 % ou au moins 99 % d'identité avec SEQ ID N° : 19.

7. Promoteur selon une quelconque revendication précédente, ledit promoteur comprenant ou étant constitué de la séquence nucléotidique de SEQ ID N° : 47.

8. Polynucléotide comprenant le promoteur selon l'une quelconque des revendications 1 à 7.

9. Polynucléotide selon la revendication 8, dans lequel le promoteur est lié de manière opérationnelle à une séquence codante pour une protéine, de préférence dans lequel le promoteur est lié de manière opérationnelle à une séquence codante pour une protéine codant NPHS2 ou un fragment et/ou une variante de celui-ci ; un polypeptide COL4A3, COL4A4 ou COL4A5, ou un fragment ou un dérivé de celui-ci ; ou CFI, CFH ou FHL-1, ou un fragment et/ou une variante de ceux-ci, idéalement dans lequel le promoteur est lié de manière opérationnelle à :
(a) une séquence codante pour une protéine codant NPHS2, ou un fragment et/ou une variante de celui-ci, de préférence ladite séquence codante pour une protéine codant une séquence d'acides aminés qui est identique à au moins 70 %, au moins 80 %, au moins 90 %, au moins 95 %, au moins 98 % ou au moins 99 % à SEQ ID N° : 55 ou un fragment de celle-ci ;
(b) une séquence codante pour une protéine codant un polypeptide COL4A3, COL4A4 ou COL4A5, ou un fragment ou un dérivé de celui-ci, de préférence, ladite séquence codante pour une protéine codant une séquence d'acides aminés qui est identique à au moins 70 %, au moins 80 %, au moins 90 %, au moins 95 %, au moins 98 % ou au moins 99 % à l'une de SEQ ID N° : 21 à 23 ou un fragment de celles-ci ; et/ou
(c) une séquence codante pour une protéine codant CFI, CFH ou FHL-1, ou un fragment et/ou une variante de ceux-ci, de préférence ladite séquence codante pour une protéine codant une séquence d'acides aminés qui est identique à au moins 70 %, au moins 80 %, au moins 90 %, au moins 95 %, au moins 98 % ou au moins 99 % à l'une de SEQ ID N° : 49, 51 ou 53, ou un fragment de celles-ci,
éventuellement, ladite séquence codante pour une protéine étant liée de manière opérationnelle à un ou plusieurs éléments régulateurs supplémentaires, tels qu'un élément régulateur post-transcriptionnel et/ou une séquence de polyadénylation.

10. Vecteur comprenant le polynucléotide selon l'une des revendications 8 ou 9.

11. Vecteur selon la revendication 10, ledit vecteur étant :
(a) capable de transduire des podocytes, éventuellement ledit vecteur étant capable de transduire spécifiquement des podocytes ; et/ou
(b) un vecteur viral, tel qu'un vecteur de virus adéno-associé (AAV), un vecteur lentiviral, un vecteur rétroviral, un vecteur adénoviral, un vecteur viral adéno-associé, un vecteur viral de l'herpès simplex, un vecteur alphaviral, un vecteur flaviviral, un vecteur rhabdoviral, un vecteur viral de la rougeole, un vecteur viral de la maladie de Newcastle, un vecteur poxviral et un vecteur picornaviral, de préférence ledit vecteur étant un vecteur AAV, éventuellement ledit vecteur viral étant sous la forme d'une particule de vecteur viral, de préférence ledit vecteur viral étant sous la forme d'une particule de vecteur AAV, de préférence ledit vecteur étant sous la forme d'une particule de vecteur AAV encapsidée par des protéines de capside AAV3B, LK03 ou AAV9.

12. Cellule comprenant le polynucléotide selon l'une des revendications 8 ou 9, ou le vecteur selon l'une des revendications 10 ou 11.

13. Composition pharmaceutique comprenant le polynucléotide selon l'une des revendications 8 ou 9, le vecteur selon l'une des revendications 10 ou 11, ou la cellule selon la revendication 12.

14. Polynucléotide selon l'une des revendications 8 ou 9, vecteur selon l'une des revendications 10 ou 11, ou cellule selon la revendication 12, destiné(e) à être utilisé(e) en médecine.

15. Utilisation *in vitro* du promoteur selon l'une quelconque des revendications 1 à 7 pour induire l'expression d'une séquence codante, de préférence ladite expression étant spécifique du rein.
